(19) Europäisches Patentamt
European Patent Office
Office européen des brevets

(11) EP 1 270 592 B1

(12) EUROPÄISCHE PATENTSCHRIFT

(45) Veröffentlichungstag und Bekanntmachung des Hinweises auf die Patenterteilung:
**29.09.2004 Patentblatt 2004/40**

(51) Int Cl.[7]: **C07K 16/18**, G01N 33/561, G01N 33/539, G01N 33/577, G01N 33/541, G01N 33/68, G01N 33/50

(21) Anmeldenummer: **01114192.6**

(22) Anmeldetag: **12.06.2001**

(54) **Monoklonaler Antikörper, mbAb 1E8, welcher für die zwei ersten N-terminalen Aminosäuren von Amyloid-beta-Peptiden spezifisch ist und dessen Verwendung zum Nachweis von Amyloid-beta Peptiden und/oder sAPPa**

Monoclonal antibody, mAb 1E8, which is specific for the first 2 amino-terminal amino acids of beta-amyloid peptides and its use in the detection of beta-amyloid peptides or sAPPs

Anticorps monoclonal, mAb1E8, spécifique pour les 2 premiers acides aminés N-terminaux des peptides beta-amyloides et son utilisation dans la détection de peptides beta-amyloides ou sAPPs

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(43) Veröffentlichungstag der Anmeldung:
**02.01.2003 Patentblatt 2003/01**

(73) Patentinhaber: **Wiltfang, Jens**
**91056 Erlangen (DE)**

(72) Erfinder:
- **Wiltfang, Jens, Dr.**
  **37120 Bovenden/Eddigehausen (DE)**
- **Dyrks, Thomas, c/o Schering Aktiengesellschaft**
  **13342 Berlin (DE)**
- **Mönning, Ursula, Dr., c/o Schering Aktiengesell.**
  **13342 Berlin (DE)**

(74) Vertreter: **Patentanwälte Rehberg + Hüppe**
**Nikolausberger Weg 62**
**37073 Göttingen (DE)**

(56) Entgegenhaltungen:
- **CHEVALLIER N ET AL: "CATHEPSIN D DISPLAYS IN VITRO BETA-SECRETASE-LIKE SPECIFICITY" BRAIN RESEARCH, AMSTERDAM, NL, Bd. 750, Nr. 1/2, 1997, Seiten 11-19, XP000921314 ISSN: 0006-8993**
- **TAKAOMI COMINGS SAIDO ET AL: "Spatial resolution of the primary betaamyloidogenic process induced in postischemic hippocampus" THE JOUNAL OF BIOLOGICAL CHEMISTRY, Bd. 269, Nr. 21, 27. Mai 1994 (1994-05-27), Seiten 15253-15257, XP002182078**
- **ARAI T ET AL: "IMMUNOHISTOCHEMICAL LOCALIZATION OF AMYLOID BETA-PROTEIN WITH AMINO-TERMINAL ASPARTATE IN THE CEREBRAL CORTEX OF PATIENTS WITH ALZHEIMER'S DIESEASE" BRAIN RESEARCH, AMSTERDAM, NL, Bd. 823, Nr. 1/2, 1999, Seiten 202-206, XP001021822 ISSN: 0006-8993**
- **WILTFANG J ET AL: "IMPROVED ELECTROPHORETIC SEPARATION AND IMMUNOBLOTTING OF BETA-AMYLOID (ABETA) PEPTIDES 1-40, 1-42, AND 1-43" ELECTROPHORESIS, WEINHEIM, DE, Bd. 18, März 1997 (1997-03), Seiten 527-532, XP001021757 ISSN: 0173-0835**
- **H.-W. KLAFKI ET AL: "Electrophoretic separation of beta-A4 Peptides (1-40) and (1-42)" ANALYTICAL BIOCHEMISTRY, Bd. 237, 1996, Seiten 24-29, XP002182079**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 270 592 B1

Printed by Jouve, 75001 PARIS (FR)

## Beschreibung

Die Erfindung bezieht sich auf einen monoklonalen Antikörper und auf die Verwendung des Antikörpers zum Nachweis von Aβ-Peptiden und/oder sAPPα. Dabei geht es insbesondere um die neurochemische Diagnostik neuropsychatrischer Erkrankungen unter Betrachtung von Aβ-Peptidkonzentrationen und hierbei wieder insbesondere um Demenzdiagnostik an Körperflüssigkeits- oder Gewebeproben.

Aus DE-Z: Psycho, 24 (1998), 726-731 ist es bekannt, dass bei Patienten mit Alzheimer Erkrankung erniedrigte Konzentrationen von Aβ1-42 im Liquor nachweisbar sind. Bei diesen Patienten gibt es auch eine Neigung zu einer Erhöhung bei der Konzentration von N-terminal modifizierten Aβ-Peptiden Aβx-42. Demgegenüber soll es keine Konzentrationsänderungen aufgrund einer Alzheimererkrankung bei dem Aβ-Peptid Aβ1-40 geben. Die Konzentrationen der Aβ-Peptide Aβ1-42 und Aβ1-40 im Liquor von Alzheimerpatienten sollen keine absolute Korrelation mit klinischen oder testpsychologischen Parametern des Schweregrads der Demenz zeigen, obwohl sie intraindividuell sehr konstant sein sollen.

Es sind Hinweise bekannt, dass auch sAPPα bei Alzheimer-Demenz im Liquor erniedrigt ist.

Um nähere Informationen über die Korrelation von Demenzerkrankungen und eventuell anderen neuropsychatrischen Erkrankungen mit der Konzentration aller oder bestimmter Aβ-Peptide in Körperflüssigkeits- oder -gewebeproben zu gewinnen, muss ein Mittel bereitstehen, mit dem die Konzentrationen der Aβ-Peptide sehr genau und reproduzierbar zu bestimmen sind, damit vorhandene Korrelationen nicht durch unvermeidliche Fehler bei den Konzentrationsbestimmungen verwischen.

Aus CHEVALLIER N ET AL: 'CATHEPSIN D DISPLAYS IN VITRO BETASECRETASE-LIKE SPECIFICITY' BRAIN research, AMSTERDAM, NL, Bd. 750, Nr. 1/2, 1997, Seiten 11-19, XP000921314 ISSN: 0006-8993 ist ein Antikörper FCA18 bekannt, der nur die erste terminale Aminosäure des Aβ-Proteins erkennt, obwohl er gegen die ersten acht N-terminalen Aminosäuren des Aβ-Proteins gezogen wurde.

Aus TAKAOMI COMINGS SAIDO ET AL: 'Spatial resolution of the primary betaamyloidogenic process induced in postischemic hippocampus' THE JOURNAL OF BIOLOGICAL CHEMISTRY, Bd. 269, Nur. 21, 27. Mai 1994 (1994-05-27), Seiten 15253-15257, XP002182078 ist ein Antikörper 9204 bekannt, der beispielsweise das Aβ-Peptid Aβ1-40 erkennt, nicht aber das Aβ-Peptid Aβ 2-40.

In ARAI T ET AL: 'IMMUNOHISTOCHEMICAL LOCALIZATION OF AMYLOID BETA-PROTEIN WITH AMINO-TERMINAL ASPARATE IN THE CEREBRAL CORTEX OF PATENTES WITH ALZHEIMER'S DIESEASE' BRAIN RESEARCH, AMSTERDAM, NL, Bd. 823, Nr. 1/2, 1999, Seiten 202-206, XP001021822 ISSN: 0006-8993 ist ein Antikörper Aβ 1 beschrieben, welcher mit dem Peptid Aβ 1-42 reagiert, jedoch nicht mit dem N-terminal um zwei Aminosäuren gekürzten Pyroglutamat-Derivat A-β (N3[pE]). Es ist nicht offenbart, ob der Antikörper Aβ1 ein um eine Aminosäure gekürztes Aβ-Peptid bindet.

Der Erfindung liegt daher die primäre Aufgabe zugrunde, ein Mittel zum genauen und reproduzierbaren Bestimmen von Konzentrationen von Aβ-Peptiden in einer Körperflüssigkeits- oder -gewebeprobe bereitzustellen. Weiterhin geht es darum, die Anwendung dieses Mittels zu optimieren und aus den damit messbaren Korrelationen zwischen Aβ-Peptidkonzentrationen und neuropsychatrischen Erkrankungen Vorhersagen abzuleiten, die bei der zukünftigen neurochemischen Diagnostik neuropsychatrischer Erkrankungen nutzbar sind.

Das Mittel, mit dem die Aufgabe der Erfindung gelöst wird, ist der monoklonale Antikörper, der als mAb 1E8 (Zellkultur UM 1998 clone 1E8) bezeichnet wird und der von Hybridomen produziert wird, die bei der DSMZ-Deutsche Sammlung von Mikroorganismen und Zellkulturen GmbH, Braunschweig, am 19.12.2000 hinterlegt wurden und denen die DSMZ-Aufnahmenummer DSM ACC2485 zugeordnet wurde.

Der Antikörper mAb 1E8 kann radioaktiv markiert sein. Er ist aber auch in Verbindung mit einem sekundären Antikörper zu seiner Markierung verwendbar.

Es hat sich herausgestellt, dass der Antikörper mAb 1E8 mit hoher Selektivität und Spezifizität an Aβ-Peptide Aβ1-x und Aβ2-x sowie an lösliches β-Amyloidvorläuferprotein nach α-Sekretaseschnitt (sAPPα) anbindet. Damit ist die Voraussetzung geschaffen, dass die Konzentration dieser Peptide mit dem Antikörper mAb 1E8 bestimmbar ist.

Die Verwendung des Antikörpers mAb 1E8 kann in einem Western-Imunoblott erfolgen. Dabei kann die effektive Selektivität des Antikörpers mAb 1E8 erhöht werden, indem vor dem Einsatz des Antikörpers unspezifische Bindungsstellen mit einem Blockiermittel blockiert werden. Ein unter dem Handelsnamen "Roti-Block" erhältliches synthetisches Reagenz hat sich in diesem Zusammenhang gegenüber einer üblichen Verwendung von Milchpulver als Blockiermittel als sehr vorteilhaft herausgestellt, da es bei weitgehend erhaltener Selektivität die effektive Avidität - und damit Nachweisempfindlichkeit - des Antikörpers mAb 1E8 erhöht. Dies gilt nicht generell, da beispielsweise ein anderer kommerziell erhältlicher mAb (6E10) mit diesem Verfahren nicht kompatibel ist.

Da der Antikörper mAb 1E8 sowohl Aβ-Peptide Aβ1-x als auch Aβ-Peptide Aß2-x als auch sAPPα erkennt, müssen diese Peptide zum selektiven Nachweis voneinander getrennt werden. Dies ist durch eine Natrium-Laurylsulphat-Polyacrylamid-Gelelektrophorese (SDS-PAGE) möglich. Bei einer herkömmlichen SDS-PAGE werden allerdings nur die Aβ-Peptide Aβ1-x und/oder Aβ2-x insgesamt von dem sAPPα abgetrennt, weil die effektiven Molekülgrößen der Aβ-Peptide Aβ1-x und/oder Aβ2-x nicht hinreichend unterschiedlich sind. Durch Harnstoffzugabe und in zusätzlicher Abhängigkeit von Detergenzienkonzentration, Gelporengröße, pH-Wert und Temperatur kann jedoch eine aminosäureprimärsequenzspezifische Konformationsänderung der Aβ-Peptide induziert werden, die die Lauflängen der carboxyterminal unterschiedlich langen Aβ-Peptide Aβ1-x und Aβ2-x in der SDS-PAGE unterscheidbar macht. Diese Methode wird hier auch als Aβ-SDS-PAGE bezeichnet. Von anderen in der jeweiligen Körperflüssigkeits- bzw. -gewebeprobe enthaltenen Substanzen können die Aβ-Peptide zuvor durch isoelektrische Fokussierung in einer zu der Richtung der SDS-PAGE senkrechten Richtung separiert werden.

So kann unter Verwendung des Antikörpers mAb 1E8 in einer Probe, die aus der Gruppe ausgewählt ist, welche Liquor, Hirnhomogenisat, Plasma und Mischungen davon umfasst, eine Konzentration des Aβ-Peptids Aβ1-42 bestimmt werden, von der bereits bekannt ist, dass absolute Erhöhungen im Hirnhomogenat und Erniedrigung im Liquor derselben beispielsweise in Korrelation mit Alzheimererkrankungen auftreten.

Unter Verwendung des Antikörpers mAb 1E8 ist es aber auch möglich, eine Probe, die aus der Gruppe ausgewählt ist, welche Liquor, Hirnhomogenisat und Mischungen davon umfasst, auf das Vorliegen einer nachweisbaren Konzentration des Aβ-Peptids Aβ2-42 zu überprüfen. Eine solche Nachweisgrenze liegt bei 100 pg/ml oder höher. Wenn sie überschritten wird, kann hieraus auf das Vorliegen einer Demenzerkrankung aus der Gruppe der Proteinfaltungskrankheiten bei dem Patienten, von dem die Probe stammt, geschlossen werden. Zur Guppe der Proteinfaltungskrankheiten gehören neben der Alzheimerkrankheit die Lewy-Körperchen-Demenz und die Creutzfeldt-Jakob-Krankheit. Bei vielen Patienten mit diesen Krankheiten konnten hohe Konzentrationen des Aβ-Peptids Aβ2-42 in Liquorproben festgestellt werden.

Die Auswertung von Plasmaproben ist hier im Gegensatz zu den vorangehend beschriebenen Verwendungen des Antikörpers mAb 1E8 schwierig, weil die Konzentration des Aβ-Peptids Aβ2-42 bereits im Liquor bei der Nachweisgrenze liegt, und die natürliche Konzentration der Aβ-Peptide im Plasma deutlich niedriger ist als im Liquor. Um gleich hohe Konzentrationen in den Proben zu erhalten, muss daher im Plasma immer ein Aufkonzentrationsschritt, beispielsweise durch Immunopräzipitation mit dem Antikörper mAb 1E8, vor der eigentlichen Konzentrationsbestimmung durchgeführt werden.

Als besonders signifikant für das Vorliegen einer Demenzerkrankung aus der Gruppe der Proteinfaltungserkrankungen hat sich ein Anstieg im Verhältnis zwischen der Konzentration des Aβ-Peptids Aβ2-42 zu der Konzentration des Aβ-Peptids Aβ1-42 in der jeweiligen Probe herausgestellt. Bei diesen Demenzerkrankungen kommt es offensichtlich zu einem Vorgang, der die Erzeugung des Aβ-Peptids Aβ2-42 zu Lasten der Erzeugung des Aβ-Peptids Aβ1-42 fördert.

Die Verwendung des Antikörpers mAb 1E8 erschließt noch weitere neurochemische Diagnosemöglichkeiten. So kann in einer Probe, die aus der Gruppe ausgewählt ist, welche Liquor, Hirnhomogenisat, Plasma und Mischungen davon umfasst, mindestens ein Konzentrationsverhältnis bestimmt werden, das aus der Gruppe ausgewählt ist, welche ein Verhältnis zwischen einer Konzentration des Aβ-Peptids Aβ1-42 zu einer Konzentration des Aβ-Peptids Aβ1-40, ein Verhältnis zwischen einer Konzentration des Aβ-Peptids Aβ1-42 zu einer Konzentration des Aβ-Peptids Aβ1-38 und ein Verhältnis zwischen einer Konzentration des Aβ-Peptids Aβ1-38 zu einer Konzentration des Aβ-Peptids Aβ1-40 umfasst. Den Bestimmungen dieser Konzentrationsverhältnisse liegt die neue Erkenntnis zugrunde, dass bei verschiedenen neuropsychatrischen Erkrankungen signifikante Verschiebungen dieser relativen Konzentration an Aβ-Peptiden gegenüber einem Vergleichspatientenkollektiv auftreten.

So kann beim Vergleichen des Konzentrationsquotienten Aβ1-38/Aβ1-40 mit einem vorgegebenen Grenzwert darauf geschlossen werden, dass beim Unterschreiten des Grenzwerts eine Alzheimererkrankung vorliegt. Dieser Grenzwert liegt im Liquor typischerweise zwischen 0,285 und 0,300. Diese Zahlenangaben beziehen sich wie alle weiteren Zahlenangaben, soweit im Einzelfall nichts anderes angegeben ist, auf Liquorproben, die nach ihrer Gewinnung jeweils einmalig zur Konservierung eingefroren wurden.

Umgekehrt kann beim Vergleichen dieses Konzentrationsquotienten Aβ1-38/Aβ1-40 mit einem anderen Grenzwert darauf geschlossen werden, dass beim Überschreiten eine chronisch entzündliche Erkrankung des zentralen Nervensystems vorliegt. Dieser Grenzwert liegt im Liquor typischerweise zwischen 0,250 und 0,260. Er liegt damit zwar unterhalb des Grenzwerts, bei dessen Unterschreiten auf eine Alzheimer Krankheit geschlossen wird. Die Überschneidung ist aber nur gering. Dabei ist auch zu sehen, dass die hier getroffenen Diagnosen im Rahmen einer differenziellen Diagnostik zu sehen sind. So kann beim Überschreiten des Grenzwerts für die Alzheimererkrankung relativ sicher ausgeschlossen werden, dass eine solche Krankheit vorliegt. Umgekehrt kann beim Unterschreiten des Grenzwerts für eine chronische entzündliche Erkrankung des zentralen Nervensystems darauf geschlossen werden, dass keine solche Erkrankung vorliegt.

Auch der Konzentrationsquotient Aβ1-42/ Aβ1-40 weist bei dem Unterschreiten eines vorgegebenen Grenzwerts daraufhin, dass eine Alzheimererkrankung vorliegt. Hier liegt der Grenzwert im Liquor typischerweise zwischen 0,130 und 0,145.

Bei dem Konzentrationsquotienten 1-42/Aβ1-38 hat sich herausgestellt, dass dieser bei Erfüllung einer Ungleichung:

$$A*A\beta 1\text{-}42/A\beta 1\text{-}38+B > A\beta 1\text{-}38/A\beta 1\text{-}40$$

Zusammen mit dem Konzentrationsquotienten Aβ1-38/Aβ1-40 auf das Vorliegen einer Alzheimererkrankung hinweist, wobei A und B Konstanten sind, für die im Liquor

$$0{,}2 < A < 0{,}8$$

und

$$0{,}5^* A < B < 2^* A$$

und

$$B < 0{,}9$$

gilt.

Die bis hierher und nachfolgend beschriebenen neurochemischen Diagnosemöglichkeiten sind zwar unter Verwendung des Antikörpers mAb 1E8 entwickelt worden. Sie sind aber genauso mit anderen Mitteln zum Bestimmen der Konzentrationen der einzelnen Aβ-Peptide Aβ1-x und Aβ2-x umsetzbar. Hierbei kann es zu Verschiebungen der angegebenen Grenzwerte durch unterschiedliche Spezifizitäten der Nachweismittel für die einzelnen Aβ-Peptide relativ zu den Spezifizitäten des Antikörpers mAb 1E8 kommen.

Neben den bislang angesprochenen Konzentrationsquotienten zwischen den Konzentrationen einzelner Aβ-Peptide hat sich herausgestellt, dass relative Anteile einzelner Aβ-Peptide an den insgesamt vorliegenden Aβ-Peptiden ebenfalls diagnostisch in Bezug auf neuropsychatrische Erkrankungen auswertbar sind. So kann in einer Probe, die aus der Gruppe ausgewählt ist, welche Liquor, Hirnhomogenisat, Plasma und Mischungen davon umfasst, mindestens ein relativer Anteil Aβ1-n% einer Konzentration eines Aβ-Peptids Aβ1-n an einer Konzentration von Aβ-Peptiden Aβ1-x bestimmt werden, wobei der relative Anteil Aβ1-n% aus der Gruppe ausgewählt ist, welche einen relativen Anteil Aβ1-42% einer Konzentration des Aβ-Peptids Aβ1-42, einen relativen Anteil Aβ1-40% einer Konzentration des Aβ-Peptids Aβ1-40 und einen relativen Anteil Aβ1-38% einer Konzentration des Aβ-Peptids Aβ1-38 umfasst, und wobei die Konzentration von Aβ-Peptiden Aβ1-x wenigstens die Konzentration der Aβ-Peptide Aβ1-38, Aβ1-40 und Aβ1-42 aus der Gruppe der Aβ-Peptide Aβ1-37, Aβ1-38, Aβ1-39, Aβ1-40 und Aβ1-42 umfasst.

Mit dem Antikörper mAb 1E8 ist es bei geeigneter Verfahrensführung möglich, die Konzentrationen sämtlicher genannter Aβ-Peptide neben derjenigen des sAPPα zu bestimmen. Die stärksten Konzentrationen werden bei den Aβ-Peptiden Aβ1-38, Aβ1-40 und Aβ1-42 festgestellt. Bei diesen Aβ-Peptiden finden sich auch signifikante Änderungen der relativen Anteile beim Auftreten neuropsychatrischer Erkrankungen. Wegen der Dominanz der Aβ-Peptide Aβ1-38, Aβ1-40 und Aβ1-42 reicht es dabei aus, die relativen Anteile in Bezug auf dieses Kollektiv der drei primär vorkommenden Aβ-Peptide zu bestimmen. Es ist aber auch eine Berücksichtigung aller fünf oben genannten Aβ-Peptide für die Bezugsgröße der relativen Anteile möglich und sinnvoll. Die Konzentrationen auf nur die drei hauptsächlich vorkommenden Aβ-Peptide ermöglicht es auch, die hier beschriebene Diagnostik mit Hilfe anderer Verfahren zur Konzentrationsbestimmung durchzuführen. So können die Konzentrationen der drei Aβ-Peptide Aβ1-38, Aβ1-40 und Aβ1-42 beispielsweise mit speziellen Essays für diese drei Aβ-Peptide ermittelt werden.

Bei der Analyse des relativen Anteils Aβ1-38% hat sich herausgestellt, dass dieser beim Überschreiten eines vorgegebenen Grenzwerts auf das Vorliegen einer chronischen entzündlichen Erkrankung des zentralen Nervensystems hinweist, wobei dieser vorgegebene Grenzwert typischerweise und bei Verwendung des Antikörpers mAb 1E8 zur Konzentrationsbestimmung der Aβ-Peptide im Liquor zwischen 15,0 und 15,7% liegt.

Bei der Analyse des relativen Anteils Aβ1-42% hat sich ergeben, dass ein Unterschreiten eines vorgegebenen Grenzwerts auf das Vorliegen einer Alzheimererkrankung hinweist.

Dieser vorgegebene Grenzwert liegt zwischen 8 und 9%. Unter Verwendung des Antikörpers mAb 1E8 zur Konzentrationsbestimmung der Aβ-Peptide im Liquor kann er auf den Bereich 8,3 bis 8,8% eingegrenzt werden.

Gleichzeitig weist das Überschreiten eines vorgegebenen Grenzwerts durch den relativen Anteil Aβ1-42% auf das Vorliegen einer chronischen entzündlichen Erkrankung des zentralen Nervensystems hin. Dieser Grenzwert liegt im Liquor zwischen 9,1 und 9,6%.

Der relative Anteil Aβ1-40% weist dann auf das Vorliegen einer Alzheimererkrankung hin, wenn ein vorgegebener Grenzwert überschritten wird, der im Liquor typischerweise zwischen 59 und 61% liegt.

Es besteht eine positive Korrelation zwischen Schwere der Alzheimer-Demenz, abgebildet über den Mini-Mental-Status Test (MMSE, 0-30 Punkte; 27-20: leichte Demenz; 19-11 Punkte: mittelschwere Demenz; 10-0 Punkte: schwere Demenz), und Aβ1-40%. Demenzpatienten mit Aβ1-40% ≥ 60 haben im Mittel einen deutlich höheren Schweregrad der Demenz (MMSE 15-16) im Vergleich zu Demenzpatienten mit Aβ1-40%<60 (MMSE 19-20). Dagegen besteht eine negative Korrelation zwischen Schwere der Alzheimer-Demenz und Aβ1-38%. Demenzpatienten mit Aβ1-38% < 17 haben im Mittel einen deutlich höheren Schweregrad der Demenz (MMSE 14-15) im Vergleich zu Demenzpatienten mit Aβ1-38% ≥ 17 (MMSE 19-20).

Aufgrund der Beziehung (Aβ1-38%⇓ & Aβ1-40%⇑ = Schweregrad der Demenz ⇑) ist wie zu erwarten der Quotient Aβ1-38/Aβ1-40 negativ mit dem Schweregrad der Demenz korreliert: Demenzpatienten mit Aβ1-38/Aβ1-40 < 0,28 haben im Mittel einen deutlich höheren Schweregrad der Demenz (MMSE 14-15) im Vergleich zu Demenzpatienten mit Aβ1-38/Aβ1-40 ≥ 0,28 (MMSE 19-20).

In der Anwendung des Antikörpers mAb 1E8 hat sich herausgestellt, dass für diesen Antikörper eine Fraktion der Aβ-Peptide Aβ1-x und Aβ2-x zugänglich ist, die von der Probenvorbehandlung abhängig ist. Die nachweisbaren Aβ-Konzentrationen könnten dadurch maximiert werden, dass die Aβ-Peptide in den Proben durch eine Behandlung mit einem Detergenz aufgeschlossen wurden. Bewährt hat sich hierzu eine SDS-Hitzedenaturierung.

In diesem Zusammenhang konnte weiter festgestellt worden, dass eine Kältekonservierung von Proben, die vor dem Aufschluss mit einem Detergenz erfolgt, die anschließend auch nach einem Aufschluss der Probe mit einem Detergenz bestimmbaren Konzentrationen von Aβ-Peptiden reduziert. Hieraus lässt sich die Forderung ableiten, die Proben vor der Kältekonservierung und jeglicher anderer Kältebehandlung bereits der Probenbehandlung mit dem Detergenz zu unterwerfen.

Darüber hinaus hat sich ergeben, dass Teilmengen des Aβ-Peptids Aβ1-42, die nach einer Kältebehandlung auch durch Aufschluss der Probe mit einem Detergenz nicht mehr dem Nachweis mit dem Antikörper mAb 1 E8 zugänglich sind, bei Patieten mit und ohne Proteinfaltungserkrankungen unterschiedlich groß sind. Es gibt offensichtlich unterschiedliche Fraktionen des Aβ-Peptids Aβ1-42 in einer Probe, wobei diese Fraktionen unterschiedlich auf Kältebehandlungen und den Aufschluss mit einem Detergenz reagieren und gleichzeitig in ihren Konzentrationen mit dem Vorliegen von Proteinfaltungskrankheiten variieren. Das unterschiedliche Verhalten dieser Fraktionen gegenüber Kälte weist auf eine Kältepräzipitation hin, so dass auch andere Präzipitationstechniken zum Unterscheiden der beiden Fraktionen möglich sind.

So kann eine Probe in mindestens zwei Teilproben unterteilt werden, von denen eine erste Teilprobe der Probenbehandlung mit dem Detergenz vor oder statt einer Präzipitationsbehandlung unterworfen wird, während die zweite Teilprobe der Präzipitationsbehandlung vor oder statt der Probenbehandlung mit dem Detergenz unterworfen wird. Anschließend werden die in beiden Probenteilen bestimmten Konzentrationen des Aβ-Peptids Aβ1-42 miteinander verglichen. Die erwähnte Präzipitationsbehandlung kann neben einer Kältebehandlung auch ein Imunoaffnitätsverfahren umfassen. Interessant ist es insbesondere, eine Differenz ΔAβ1-42 zwischen den in den beiden Probenteilen bestimmten Konzentrationen des Aβ-Peptids Aβ1-42 zu ermitteln. Dieser Wert ist ein hochsignifikanter Hinweis auf das Vorliegen einer Proteinfaltungskrankheit.

In der praktischen Anwendung Antikörpers mAb 1E8 können die Aβ-Peptide, an die der Antikörper mAb 1E8 gebunden ist, mit einem gegen den Antikörper mAb 1E8 gerichteten sekundären Antikörper markiert werden. Der gegen den Antikörper mAb 1E8 gerichtete sekundäre Antikörper kann bereits mit einem mengenmäßig registrierbaren Marker versehen sein oder nach seiner Immunreaktion mit dem Antikörper mAb 1E8 mit einem mengenmäßig registrierbaren Marker versehen werden.

Bei der mengenmäßigen Registrierung des Markers ist es bevorzugt, diese Registrierung photometrisch mit einer CCD-Kamera vorzunehmen, weil dieses Vorgehen eine sehr hohe Linearität zwischen dem Signal der CCD-Kamera und den registrierten Mengen des markierten Antikörpers mAb 1E8 gewährleistet.

Der neue Antikörper ist neben den bisher beschriebenen Diagnosemöglichkeiten auch zum reinen Aufkonzentrieren von Aβ-Peptiden Aβ1-x und/oder Aβ1-x und/oder Aβ2-x und/oder sAPPα geeignet.

Eine weitere Verwendungsmöglichkeit ergibt sich bei der Unterscheidung von Aβ-Peptiden Aβ1-x und Aβ2-x von Aβ-Peptiden Aβn-x mit n >2, da der Antikörper mAb 1E8 eine ausgeprägte N-terminale Spezifizität aufweist und an Aβ-Peptide Aßn-x mit n>2 deutlich geringer anbindet (< 5%), wenn er unter den spezifischen Bedingungen des Aβ-SDS-PAGE/Immunoblot eingesetzt wird.

Die Erfindung wird im Folgenden durch eine Charakterisierung und eine Beschreibung eines Verfahrens zur Herstellung des Antikörpers mAb 1E8 sowie in Form einer Beschreibung von Anwendungen des Antikörpers mAb 1E8 näher erläutert und beschrieben.

In den beigefügten Figuren zeigt:

**Fig. 1:** ein Flußdiagramm der Patientenkollektive, bei denen Aβ-Peptide mittels Aβ-SDS-PAGE/Immunoblot im Liquor oder Plasma gemessen wurden. Die einfach umrahmten Patientenkollektive sind Teilmengen ihrer doppelt umrahmten Obergruppen. Einige Patienten befinden sich gleichzeitig in mehreren Kollektiven (vergl. Tabellen 5a-d) Die NDC-3 Untergruppen IP-Plasma-3 (n=5), IP-CSF-3 (n=5) und SDS-CSF-3 (n=5) sind nicht aufgeführt (vergl. 2.9.1).

**Fig. 2:** einen Aβ-IPG-2D-PAGE/Immunoblot-2 (Panel A, C) und Aβ-SDS-PAGE/Immunoblot-2 (Panel B) von synthetischen Aß-Peptiden, humanem Liquor und Liquor mit Zusatz synthetischer Aβ-Peptide.

**Fig. 3:** eine Bestimmung von Aβ-Peptiden im Liquor bei NDC-3 mittels Aβ-SDS-PAGE/Immunoblot-2 und ein Vergleich zwischen Trenngel mit (Panel A) und ohne Harnstoff (Panel B). Dabei gilt für die Spalten 1 bis 8: 10 µl Liquor pro Patient. Der Liquor wurde nativ eingefroren und anschließend SDS-/hitzedenaturiert. Für die Spalten a bis e gilt: Mix synthetischer Aβ-Peptide (Verdünnungsreihe).

**Fig. 4:** eine Auftragung von Aβ1-42 im Liquor bei den Patientenkollektiven NDC-1 und AD-1, bestimmt mittels Aβ-SDS-PAGE/Immunoblot-1 und densitometrischer Filmauswertung.

**Fig. 5:** eine Auftragung des Quotienten Aβ1-42/Aβ1-40 im Liquor bei NDC-1 und AD-1, bestimmt mittels Aβ-SDS-PAGE/Immunoblot-1 und densitometrischer Filmauswertung.

**Fig. 6:** eine Auftragung des Quotienten Aβ1-42/Aβ1-38 im Liquor bei NDC-1 und AD-1, bestimmt mittels Aβ-SDS-PAGE/Immunoblot-1 und densitometrischer Filmauswertung.

**Fig. 7:** eine Auftragung von Aβ1-42 im Liquor bei NDC-1 und AD-1, bestimmt mittels Immunopräzipitation (IP ohne Detergenz, mAb 6E10) und Aβ-SDS-PAGE/Immunoblot-1 mit densitometrischer Filmauswertung.

**Fig. 8:** eine Auftragung von Aβ-Peptidkonzentrationen, bestimmt mittels Aβ-SDS-PAGE/Immunoblot-2 im Liquor bei OND-3, CID-3 und AD-3.

**Fig 9:** eine Auftragung von Aβ-Peptidkonzentrationen, bestimmt mittels Aβ-SDS-PAGE/Immunoblot-2 im Liquor bei Patienten der Gruppen OND-3 und AD-3 mit ein oder zwei ApoE ε4 Allelen (AD-3ε4plus, OND-3ε4plus), im Vergleich zu OND-3 Patienten ohne ApoE ε4 Allel (ONDε4minus).

**Fig. 10:** eine Korrelation von Aβ-38 und Aβ1-40 im Liquor bei NDC-3 und eine Korrelationsmatrix der Aβ-Peptide.

**Fig. 11:** eine Auftragung von Aβ1-38% über Aβ1-40% im Liquor bei OND-3, CID-3 und AD-3. Die Regressionsgrade bezieht sich auf das Kollektiv AD-3. In Pfeilrichtung steigt der Schweregrad der Demenz. Die Grenzwertlinien (Aβ1-38%=15.5, Aβ1-42%= 9.6) beziehen sich auf das Kollektiv CID-3. Einzelne Patienten werden über ihre Code-Nummern identifiziert.

**Fig. 12:** eine Auftragung von Aβ1-40% über Aβ1-42% im Liquor bei OND-3, CID-3 und AD-3. Die Regressionsgrade bezieht sich auf das Kollektiv AD-3. In Pfeilrichtung steigt der Schweregrad der Demenz. Die Grenzwertlinien (Aβ40%=63.0, Aβ42%= 8.5) beziehen sich auf das Kollektiv AD-3. Einzelne Patienten werden über ihre Code-Nummern identifiziert.

**Fig. 13:** eine Auftragung von Aβ1-40% über Aβ1-38% im Liquor bei OND-3, CID-3 und AD-3. Die Regressionsgrade bezieht sich auf das Kollektiv AD-3. In Pfeilrichtung steigt der Schweregrad der Demenz. Die Grenzwertlinien (Aβ38%=15.5, Aβ40%= 60.0) beziehen sich auf das Kollektiv CID-3. Die unterbrochenen Grenzwertlinien (Aβ38%=16.0, Aβ40%= 63.0) kennzeichnen AD-3 Patienten mit schwerer Demenz. Einzelne Patienten werden über ihre Code-Nummern identifiziert.

**Fig. 14:** einen Box-Plot der MMSE Testergebnisse im Liquor bei AD-3 in Abhängigkeit von den prozentualen Aβ-Peptidanteilen an der Aβ-Peptidgesamtkonzentration.

**Fig. 15:** MMSE Testergebnis in Abhängigkeit des prozentualen Aβ-Peptidanteils im Liquor an der Aβ-Peptidgesamtkonzentration bei AD-3.

**Fig. 16:** eine Auftragung von Aβ1-38/Aβ1-40 über Aβ1-42/Aβ1-38 im Liquor bei OND-3, CID-3 und AD-3. Die Regressionsgrade bezieht sich auf das Kollektiv AD-3. Die gestrichelte Grenzwertlinie ist eine Parallele zur Regressionsgeraden. In Pfeilrichtung steigt der Schweregrad der Demenz. Die Grenzwertlinien (Aβ1-38/Aβ1-40 =0.26, Aβ1-42/Aβ1-38 = 0.57) beziehen sich auf das Kollektiv CID-3. Einzelne Patienten werden über ihre Code-Nummern identifiziert.

**Fig. 17:** eine Auftragung von Aβ1-38/Aβ1-40 über Aβ1-42/Aβ1-40 im Liquor bei OND-3, CID-3 und AD-3. Die Regressionsgrade bezieht sich auf das Kollektiv AD-3. In Pfeilrichtung steigt der Schweregrad der Demenz. Die Grenzwertlinien (Aβ1-38/Aβ1-40 =0.26, Aβ1-42/Aβ1-40 = 0.16) beziehen sich auf das Kollektiv CID-3. Einzelne Patienten werden über ihre Code-Nummern identifiziert.

**Fig. 18:** einen Box-Plot der MMSE Testergebnisse im Liquor bei AD-3 in Abhängigkeit des Quotienten Aβ1-38/Aβ1-40.

**Fig. 19:** eine Auftragung von Aβ1-42nativ im Liquor bei NDC-3KP und AD-3KP über ΔAβ1-42%, d.h. in Abhängigkeit von der kältepräzipitationsbedingten Erniedrigung von Aβ1-42. Die Grenzwertlinien (Aβ1-42 = 2100, ΔAβ1-42% = -17) beziehen sich auf das Kollektiv AD-3KP. Die Nullachse, d.h. keine kältepräzipitationsbedingte Erniedrigung von Aβ1-42, ist durch eine Linie gekennzeichnet. Der ApoE-Genotyp wird für die NDC-3KP Patienten angegeben. 9/11 AD-3KP Patienten hatten mindestens ein ApoE ε4 Allel. Für 2/11 AD-3KP Patienten lag der ApoE ε4 Genotypus nicht vor.

**Fig. 20:** eine Auftragung von Aβ1-42SDS im Liquor bei NDC-3KP und AD-3KP in Abhängigkeit von ΔAβ1-42%, d.h. in Abhängigkeit von der kältepräzipitationsbedingten Erniedrigung von Aβ1-42. Die Grenzwertlinien (Aβ1-42 = 2100, ΔAβ1-42% = -17) beziehen sich auf das Kollektiv AD-3KP. Die Nullachse, d.h. keine kältepräzipitationsbedingte Erniedrigung von Aβ1-42, ist durch eine Linie gekennzeichnet. Der ApoE-Genotyp wird für die NDC-3KP Patienten angegeben. 9/11 AD-3KP Patienten hatten mindestens ein ApoE ε4 Allel. Für 2/11 AD-4 Patienten lag der ApoE ε4 Genotypus nicht vor.

**Fig. 21a:** einen Aβ-SDS-PAGE/Immunoblot-2: Immunopräzipitate (RIPA-IP, mAb 1E8) RIPA-löslicher Aβ-Peptide aus Homogenaten von temporalem Kortex bei AD im Vergleich mit frontotemporaler Demenz (FTD). Auftragsvolumen 4 µl. In den Spalten a bis c: Mix synthetischer Aβ-Peptide (Verdünnungsreihe). In den Spalten 1 bis 4 und 5: temporaler Kortex bei AD. (* Das Immunopräzipitat bei AD wurde bei einigen Patienten zwanzigfach verdünnt.) In den Spalten 8 bis 10: temporaler Kortex bei FTD.

**Fig. 21b:** einen Aβ-SDS-PAGE/Immunoblot-2: Immunopräzipitate (RIPA-IP, mAb 1E8) RIPA-löslicher Aβ-Peptide aus Homogenaten von temporalem Kortex bei AD im Vergleich mit frontotemporaler Demenz (FTD), Lewy-Körperchen Demenz (LBD) und Kontrollpatient ohne Demenz (NDC). Auftragsvolumen 4 µl. In den Spalten

a bis d: Mix synthetischer A□-Peptide (Verdünnungsreihe)
5 und 6: temporaler Kortex bei AD
11 und 12: temporaler Kortex bei FTD
13 bis 15: temporaler Kortex bei LBD; (13) LBD CERAD A, (14) LBD CERAD C, (15) LBD CERAD C
16: temporaler Kortex bei NDC

**Fig. 22:** einen Aβ-SDS-PAGE/Immunoblot-2: Immunopräzipitate (mAb 1E8) RIPA-löslicher Aβ-Peptide bei AD aus Hirnhomogenaten unterschiedlicher Hirnregionen. Intraindividueller Vergleich von Cerebellum und temporalem Kortex. Auftragsvolumen 4 µl. In den Spalten:
a bis c: Mix synthetischer Aß-Peptide (Verdünnungsreihe)

1 bis 7: Cerebellum
1 * bis 7*: temporaler Kortex, * Immunopräzipitate zwangzigfach verdünnt

**Fig. 23a:** einen Aβ-SDS-PAGE/Immunoblot-2, in dem vergleichend zu einem Mix synthetischer Aβ-Peptide (1) dargestellt sind: (2) SDS-/Hitzedenaturierte Zellkulturüberstände von humanAPP751Sw transgenen H4 Neuroglioma Zellen (Auftragsvolumen 4 µl), (3) 10 µl Liquor eines NDC Patienten und (4) 10 µl Liquor eines AD Patienten.

**Fig. 23b:** einen Aβ-SDS-PAGE/Immunoblot-2 in dem vergleichend zu einem Mix synthetischer Aβ-Peptide (1) die Immunopräzipitate (mAb 1E8) RIPA-löslicher Aβ-Peptide folgender Hirnhomogenate dargestellt sind:

(2) temporaler Kortex bei AD
(3) temporaler Kortex bei frontotemporaler Demenz
(4) temporaler Kortex, Kontrollpatient ohne Demenz

* Das Immunopräzipitat bei AD wurde zwanzigfach verdünnt.

**Fig. 24:** drei Aβ-IPG-2D-PAGE/Immunoblots-2:
oben: eine zweidimensionale Auftrennung synthetischer Aβ-Peptide. Durch die N-terminale Verkürzung um Aspartat wird der Isoelektrische Punkt (Ip) um eine pH-Einheit basischer. Mitte: Immunopräzipitation (mAb 1E8) und zweidimensionale Auftrennung von Liquor eines Patienten mit AD, der in der Aβ-SDS-PAGE die Bande mit dem Rf-Wert für Aβ2-42 zeigte. unten: Immunopräzipitation (mAb 1E8) RIPA-löslicher Aβ-Peptide und zweidimensionale Auftrennung aus dem temporalen Kortex bei AD. Die Bande mit dem Rf-Wert für A□2-42 in der Aβ-SDS-PAGE wird auch zweidimensional als Aβ2-42 identifiziert.

**Fig. 25a:** einen Aβ-SDS-AGE/Immunoblot-2 von Aβ-Peptiden im Liquor vom Meerschweinchen. Der Liquor wurde vor dem Einfrieren SDS-/Hitzedenaturiert. In den Spalten:

1 bis 3: 10µl Liquor von Meerschweinchen 1,2 und 3
a bis d: Verdünnungsreihe synthetischer Aβ-Peptide.

**Fig. 25b:** einen Aβ-SDS-AGE/Immunoblot-2 von Aβ-Peptiden im Liquor vom Kaninchen. Der Liquor wurde vor dem Einfrieren SDS-/Hitzedenaturiert. In den Spalten:

1 bis 3: 10µl Liquor
a: synthetische Aβ-Peptide.

**Fig. 26:** einen Aβ-SDS-AGE/Immunoblot-2 von hippokampalen Gewebeschnitten mit Kurzzeitkultur (0-8h) vom adulten Meerschweinchen. Jeweils zwei Gewebeschnitte (Dicke 500 µm) wurden gepoolt, in Anwesenheit von RIPA Detergentien homogenisiert und immunopräzipitiert (mAb 1E8). Die zugehörigen Kulturüberstände (2 x 500µl) wurden gleichfalls gepoolt und in Anwesenheit von RIPA immunopräzipitiert (mAb 1E8). In den Spalten:
0 bis 8, intrazellulär: Zeitverlauf der intrazellulären Konzentration von Aβ-Peptiden im hippokampalen Gewebeschnitt unmittelbar nach Gewinnung (0) bis zu acht Stunden (8; Doppelmessung) in Kurzzeitkultur. 1 bis 8, Überstände: Zeitverlauf der in die Kulturüberstände freigesetzten Aβ-Peptide. synth. Aβ: synthetisches Aβ1-40 und Aβ1-42

**Fig. 27:** zwei Aβ-SDS-PAGE/Immunoblots-2, die eine Behandlung einer human751APPSw trangenen H4 Neuroglioma Zelllinie mit unterschiedlichen Proteaseinhibitoren (23a) und einen dosisabhängigen Effekt von Calpaininhibitor-1 (23b) zeigen. Die freigesetzten Aβ-Peptide wurden in jeweils 4 µl SDS-/Hitzedenaturierten Zellkulturüberstanden quantifiziert (vergl. Fig. 23a,b). Vergleichend wurde die sAPPα Konzentration in den Zellkulturüberständen bestimmt.
Im Panel a ist: (1) DMSO-Kontrolle; (2) 50 µM Calpaininhibitor-1; (3) 100 µM Calpaininhibitor-3; (4) 5 µM MG132; (5) 25 µM Calpeptin; (a-d) Mix synthetischer A□-Peptide.
Im Panel b ist: (1,1*) DMSO-Kontrolle; (2,2*) 12.5 µM Calpaininhibitor-1; (3,3*) 25 µM Calpaininhibitor-1; (4,4*) 50 µM Calpaininhibitor-1; (a-e) Mix synthetischer Aβ-Peptide; * Doppelbestimmung.

**Fig. 28a:** einen Aβ-SDS-PAGE/Immunoblot-2, der eine Behandlung einer humanAPP751Sw trangenen H4 Neuroglioma Zelllinie mit unterschiedlichen Konzentrationen von Calpaininhibitor-1 zeigt (vergl. Fig. 23b). Die freigesetzten Aβ-Peptide wurden in jeweils 4 µl SDS/hitzedenaturierten Zellkulturüberstanden quantifiziert . Vergleichend zur DMSO-Kontrolle wurde dosisabhängig der Effekt von Calpaininhibitor-1 auf die Aβ-Peptidkonzentration im Überstand untersucht.

**Fig. 28b:** einen Aβ-SDS-PAGE/Immunoblot-2, der eine Behandlung einer humanAPP751Sw trangenen H4 Neuroglioma Zelllinie mit unterschiedlichen Konzentrationen von Calpaininhibitor-1 zeigt (vergl. Fig. 23b). Die freigesetzten Aβ-Peptide wurden in jeweils 4 µl SDS/hitzedenaturierten Zellkulturüberstanden quantifiziert. Vergleichend zur DMSO-Kontrolle wurde dosisabhängig der Effekt von Calpaininhibitor-1 auf den prozentualen Anteil einer Aβ-Peptidspecies an der Aβ-Peptidgesamtkonzentration untersucht.

Weiterhin sind 21 Tabellen beigefügt.

**0 Herstellung des monoklonalen anti Aβ-Antiköpers 1E8**

Die Herstellung des monoklonalen Antikörpers mAb 1E8 erfolgte in Auftrag der Schering AG bei der Auftragsfirma "nano Tools Antiköpertechnik" in Denzlingen nach deren Standardverfahren. Die Erstellung der Immunisierungs- und Screening-Strategie erfolgte in Absprache mit der Schering AG.

Kurzbeschreibung: Zur Immunisierung Balb/c Mäusen wurde das gesammte Aβ Protein (1-42) eingesetzt (10µg/Immunisierung). Es erfolgte eine Primärimmunisierung sowie 3 sich anschließende Booster-Immunisierungen. Die Immunisierungen erfolgten im Abstand von jeweils 2 Wochen. Danach wurde das Tier getötet und die Milz zur Zellfusion mit einer Maus-Myelomzellinie eingesetzt. Die fusionierten Zellen wurden auf 96-Loch-Gewebekulturplatten übertragen und in Gegenwart von Feeder-Makrophagen kultiviert.

Zur Identifizierung der N-terminal spezifischen Antikörper wurden das Peptid 1-16 kovalent an entsprechend aktivierte ELISA-Platten gekoppelt und für das Screening mit den Hybridomzellüberständen eingesetzt. Die Aβ 1-16-positiven Klone wurden danach rekloniert und wiederholt getestet. Nach Expansion der Klone erfolgte die Cryo-Konservierung. Die Anreicherung des Antiköpers wurde mit Hilfe der Ionenaustauscherschromatographie unter nicht-denaturierenden Bedingungen durchgeführt..

Zur Eingrenzung der Spezifität des Antiköpers wurde ein Epitop-Mapping aus Cellullosegebundenen linearen Peptiden vorgenommen (Spotsynthese). Dazu wurde die Primärsequenz von Aβ als Serie überlappender Peptide in Form von "Spots" auf einer Cellulosemembran synthetisiert und die Membran analog zum Western-Blot-Verfahren mit den monoklonalen Antikörpern inkubiert. Die Detektion spezifisch-gebundener Antikörper erfolgte mit einem Sekundärantikörper.

Die Analyse ergab, das der Antikörper 1E8, der Subklasse IgG1 kappa zugehörig, ein N-terminales lineares Epitop detektiert, das aus den ersten 8 N-terminalen Aminosäuren der Aβ-Sequenz ausbildet wird. Dieser Antikörper erwies sich in nachfolgenden Experimenten als geeignet für den Nachweis von nativem Aβ im Western Bot, Immunpräzipiation, Immunohistochemie und ELISA.

## 1 Übersicht

### 1.1 Beschreibung der Methodik

Es wird eine Natrium-Laurylsulfat-Polyacrylamid-Gelelektrophorese (SDS-PAGE) für die Auftrennung des β-Amyloidvorläuferproteins (APP) und seiner Metabolite, insbesondere den β-Amyloid Peptiden (Aβ-Peptide), beschrieben. Die spezielle SDS-PAGE, im folgenden Aβ-SDS-PAGE genannt, nutzt ein multiphasisches Puffersystem (Bicin/Bistris/Tris/ Sulfat), und der Trennmechanismus beruht auf einer harnstoffinduzierten Konformationsänderung von Aβ-Peptiden bei Eintritt in das Trenngelkompartiment. Die Konformationsänderung ist hochgradig spezifisch für die Aminosäureprimärsequenz der jeweiligen Aβ-Peptide und führt zu einer reproduzierbaren Änderung des effektiven Molekularradius. Damit lassen sich zahlreiche Aβ-Peptide auftrennen, die sich N- und C-terminal zum Teil nur um eine Aminosäure unterscheiden und aufgrund des geringen Massenunterschiedes mittels konventioneller SDS-PAGE nicht aufgetrennt werden können. Die Konformationsänderung wird unter den Bedingungen des multiphasischen Puffersystems durch die Zugabe von Harnstoff oberhalb einer Konzentration von 6M ausgelöst. Die Aβ-peptidspezische Konformationsänderung wird bei definiertem pH-Wert und Ionenstärke neben der Molarität des eingesetzten Harnstoffs durch die verwendete Porengröße des Polyacrylamidgels, die Konzentration des Detergenz (SDS) und die Temperatur während der Trennung bestimmt. Die optimale Trenngelmatrix für die Auftrennung eines breiten Spektrums N- und C-terminal (Nt, Ct) modifizierter Aβ-Peptide wurde mit 12%T/5%C/8MHarnstoff/0.25%SDS gefunden.

Die Aβ-SDS-PAGE wurde mit der isoelektrischen Fokussierung (IEF) innerhalb einer ersten analytischen Dimension unter Verwendung von Trägerampholyten oder immobilisierter pH-Gradienten (IPG) zur zweidimensionalen Elektrophorese kombiniert (Aβ-2D-PAGE und Aβ-IPG-2D-PAGE). Anhand von Aβ-SDS-PAGE und Aβ-IPG-2D-PAGE wurde das elektrophoretische Laufverhalten der synthetischen Aβ-Peptide $A\beta_{1-33}$, $A\beta_{1-34}$, $A\beta_{1-35}$, $A\beta_{1-37}$, $A\beta_{1-38}$, $A\beta_{1-39}$, $A\beta_{1-40}$, $A\beta_{1-42}$, $A\beta_{2-40}$, $A\beta_{3-40}$, $A\beta_{3p-40}$, $A\beta_{2-42}$, $A\beta_{3-42}$ und $A\beta_{3p-42}$ charakterisiert.

Der Nachweis erfolgte mittels Western-Immunoblot (PVDF-Membran) und "enhanced chemiluminescence (ECL)" (Aβ-SDS-PAGE/Immunoblot, Aβ-2D-PAGE/Immunoblot, Aβ-IPG-2D-PAGE/Immunoblot). Dafür wurde der monoklonale Antikörper mAb 1E8 eingesetzt, für den eine ungewöhnlich hohe N-terminale Spezifität nachgewiesen wurde. Unter den Bedingungen des eingesetzten Western-Immunoblot erkennt der mAb 1E8 bei Fehlen des N-terminalen Aspartat die entsprechenden Aβ-Peptide 2-x (z.B. $A\beta_{2-40}$, $A\beta_{2-42}$) mit ähnlich guter Nachweisempfindlichkeit wie die Aβ-Peptide 1-x (z.B. $A\beta_{1-40}$, $A\beta_{1-42}$). Die synthetischen Aβ-Peptide $A\beta_{3-40}$ oder $A\beta_{3-42}$, bei denen zusätzlich N-terminal die Aminosäure Alanin fehlt, und ihre Pyroglutamat-Derivate werden dagegen in physiologisch relevanten Konzentrationen nicht mehr nachgewiesen. Die Nachweisempfindlichkeit im Western-Immunoblot konnte selektiv für den mAb 1E8 verbessert werden, indem anstelle des sonst überwiegend verwendeten Milchpulvers (Immunoblot-1), ein synthetisches Reagenz für die Blockierung unspezifischer Bindungsstellen einsetzt wurde (Immunoblot-2). Ein kommerziell erhältlicher N-terminal spezifischer monoklonaler Antikörper (6E10), der sonst häufig für den Nachweis von Aβ-Peptiden mittels Western-Immunoblot eingesetzt wird, ist nicht kompatibel mit Immunoblot-2. Durch die Kombination von Aβ-SDS-PAGE/Immunoblot-2 mit dem Nachweis der APP-Metabolite über eine hochempfindliche CCD-Kamera konnte ein quantitativer Western-Immunoblot mit einer Nachweisempfindlichkeit von 1 pg für $A\beta_{1-40}$ und 2 pg für $A\beta_{1-42}$ aufgebaut werden. Für den Aβ-SDS-PAGE/ Immunoblot-2 mit CCD-Detektion können Intra- und Interassayvariationskoeffizienten von weniger als 10 % für 20 pg Aβ-Peptid realisiert werden. Für den Aβ-SDS-PAGE/Immunoblot-2 mit Filmdetektion beträgt die Nachweisempfindlichkeit 0.3 pg für $A\beta_{1-40}$ und 0.6 pg für $A\beta_{1-42}$. Andere Western-Immunoblot Verfahren mit gleich guter Nachweisempfindlichkeit und Variationskoeffizienten für den Nachweis für Aβ-Peptiden sind bislang nicht bekannt. Die oben genannte Nachweisempfindlichkeit ist Voraussetzung für eine neurochemische Demenzdiagnostik im Liquor, wenn die Aβ-Peptide direkt nach SDS-/Hitzedenaturierung mittels Western-Immunoblot und CCD-Kamera quantifiziert werden sollen, d.h. ohne vorherige selektive Anreicherung durch Immunopräzipitation. Weiter konnte nachgewiesen werden, dass die Trennschärfe des Aβ-SDS-PAGE/Immunoblot für die neurochemische Demenzdiagnostik gerade durch diese Probenvorbehandlung noch einmal wesentlich gesteigert werden kann, wenn die SDS-/Hitzedenaturierung vor dem Einfrieren der Liquorproben erfolgt.

**1.2 Aβ-SDS-PAGE/Immunoblot und neurochemische** Demenzdiagnostik

Der Aβ-SDS-PAGE/Immunoblot-2 (s.o.) realisiert erstmals die direkte Quantifizierung von sAPPα und Aβ-Peptiden mittels CCD-Kamera in nur 10 µl humanen oder tierischen (Meerschweinchen, Kaninchen) Liquorproben. Mit dieser Methode konnte erstmals nachgewiesen werden, dass neben $A\beta_{1-40}$ und $A\beta_{1-42}$ drei weitere carboxyterminal (C-terminal) verkürzte Aβ-Peptide ($A\beta_{1-37}$, $A\beta_{1-38}$, $A\beta_{1-39}$) hochgradig konserviert in humanem und tierischem Liquor vorkommen. Dabei ist nicht wie bisher angenommen $A\beta_{1-42}$ nach $A\beta_{1-40}$ das zweithäufigste Aβ-Peptid im humanen Liquor, sondern $A\beta_{1-38}$. Weiter konnte gezeigt werden, dass die drei zusätzlichen C-terminal verkürzten Aβ-Peptide auch im humanen Plasma nachweisbar sind, hier aber mit wesentlich geringerer Konzentration und anderer relativer Verteilung. Insbesondere das Verhältnis $A\beta_{1-42}/A\beta_{1-38}$ scheint ZNS-spezifisch unterschiedlich zu sein.

Bei einigen Patienten mit AD wird im Liquor zusätzlich das N-terminal verkürzte Aβ-Peptid 2-42 nachweisbar, dass in den Hirnhomogenaten von Patienten mit AD regelhaft extensiv erhöht ist.

Es konnte erstmals nachgewiesen werden, dass im Gegensatz zu den absoluten Konzentrationen der Aβ-Peptide im Liquor, die prozentualen Anteile Aβ1-n% der Aß-Peptide Aβ1-n, mit n = 37, 38, 39, 40 oder 42, an ihrer Gesamtmenge Aβ1-x mit hoher Sensitivität und Spezifität Patienten mit AD und chronisch-entzündlichen ZNS-Erkrankungen (CID) identifizieren. Im Gegensatz zu den Absolutkonzentrationen korrelieren die relativen Aβ-Peptidanteile auch signifikant mit dem Schweregrad der Demenz. Weiter konnten für Patienten mit AD spezifische Zusammenhänge zwischen bestimmten prozentualen Aβ-Peptidanteilen gezeigt werden. Dies gilt auch für bestimmte Aβ-Peptidquotienten und die entsprechenden Korrelationen lassen sich für eine verbesserte neurochemische Demenzdiagnostik nutzen. Insbesondere der Zusammenhang zwischen $A\beta_{1-38}$% und $A\beta_{1-42}$% bzw. die Korrelation zwischen den Aβ-Peptidquotienten $A\beta_{1-38}/A\beta_{1-40}$ und $A\beta_{1-42}/A\beta_{1-38}$ zeigt bei AD eine vergleichsweise hohe Korrelation und ist diagnostisch vielversprechend. Der überraschend deutliche Unterschied zwischen den absoluten und anteiligen Konzentrationen der Aβ-Peptidspecies bezüglich ihrer Eignung für die neurochemische Demenzdiagnostik ist wahrscheinlich dadurch bedingt, dass krankheitsspezifische Änderungen der γ-Sekretaseaktivität auftreten und diese besser über eine Veränderung der relativen Aβ-Peptidanteile abgebildet werden.

Zur Probenvorbereitung für den Aβ-SDS-PAGE/Immunoblot werden Aβ-Peptide und weitere APP-Metabolite SDS-/Hitzedenaturiert. Alternativ kann zur selektiven Konzentrierung von Aβ-Peptiden zuvor eine Immunopräzipitation (IP) durchgeführt werden. Die Ergebnisse zeigen, dass in Abhängigkeit der Probenvorbereitung unterschiedliche Anteile der in biologischen Flüssigkeiten vorkommenden Aβ-Peptide gemessen werden können. Dabei ist ein Detergenzien(SDS)-dissozierbarer Anteil von einer Aβ-Peptidfraktion unterscheidbar, die Antikörpern innerhalb von Immunopräzipitation oder ELISA-Verfahren direkt zugänglich ist, d.h. ohne gleichzeitige Behandlung mit Detergentien. Diese Differenzierung erklärt sich wahrscheinlich durch hochaffine Bindung der Aβ-Peptide an andere Proteine oder Aβ-Autoaggregate. Der SDS-dissozierbare Anteil ist dabei deutlich höher als die Antikörperdissozierbare Fraktion. Dieses Phänomen ist spezifisch für $A\beta_{1-42}$ besonders stark ausgeprägt.

Für $A\beta_{1-42}$ ist - im Gegensatz zu $A\beta_{1-40}$- eine kältepräzipitationsbedingte (KP) Erniedrigung durch das Einfrieren der Liquorproben nachweisbar. Die KP-bedingte Erniedrigung wird wahrscheinlich hauptsächlich von der Aggregat-gebundenen Fraktion von $A\beta_{1-42}$ getragen und führt bei einem beträchtlichen Teil der Patienten ohne Alzheimer-Demenz (NDC) zu einer AD-typischen Erniedrigung der Liquorspiegel von $A\beta_{1-42}$. Dieser Effekt ist besonders deutlich bei Patienten mit mindestens einem ApoE ε4 Allel und erklärt wahrscheinlich, warum auch bei Patienten ohne AD, aber mit ε4 Allel, im zuvor eingefrorenen Liquor vergleichsweise tiefe Konzentrationen von $A\beta_{1-42}$ gemessen werden.

Durch "protektive" SDS-/Hitzedenaturierung vor dem Einfrieren der Probe kann die KP-bedingte Erniedrigung von $A\beta_{1-42}$ bei Patienten ohne AD effektiv verhindert werden. Patienten mit AD zeigen dagegen auch bei Vorbehandlung der Liquorprobe mit SDS/Hitzedenaturierung vor dem Einfrieren niedrige Konzentrationen von $A\beta_{1-42}$ im Liquor. Damit wird die diagnostische Trennschärfe des Aβ-SDS-PAGE/Immunoblot für die neurochemische Demenzdiagnostik durch SDS-/Hitzedenaturierung der Liquorproben vor dem Einfriervorgang ganz wesentlich verbessert. Der Aβ-SDS-PAGE/ Immunoblot in Verbindung mit der oben genannten Probenvorbereitung ist auch für die Früh- und präklinische Diagnostik der AD vielversprechend, da zu erwarten ist, dass grenzwertig tiefe $A\beta_{1-42}$ Liquorspiegel besonders dann eine beginnnde AD anzeigen, wenn sie nicht über eine KP-abhängige Erniedrigung erklärt werden können. Alternativ ist durch prospektive Studien bei Patienten mit leichten kognitiven Störungen zu überprüfen, ob nicht schon eine ausgeprägte KP-abhängige Erniedrigung von $A\beta_{1-42}$ *per se* prädiktiven Wert für die spätere Entwicklung einer AD hat.

Die KP-abhängige Erniedrigung von $A\beta_{1-42}$ im Liquor bei AD kann über folgende Hypothesen erklärt werden:

1. der Liquor von Patienten mit AD enthält bei unveränderter Aβ-Peptidgesamtkonzentration selektiv weniger $A\beta_{1-42}$
2. die KP-bedingte Erniedrigung von $A\beta_{1-42}$ kann trotz SDS-/Hitzedenaturierung nicht verhindert werden
3. $A\beta_{1-42}$ ist im Liquor bei AD nicht erniedrigt, sondern durch SDS-stabile Bindung an Trägerproteine oder Aβ-Peptidaggregate nur vermindert meßbar

Im letzteren Fall (3) wäre diese Fraktion von $A\beta_{1-42}$ auch dem enzymatischen Katabolismus entzogen, damit pathophysiologisch relevant und potentiell ein molekulares "target" für Wirkstoffindungsprojekte. Dabei muß nicht unbedingt die Zusammensetzung oder die molekulare Primärstruktur $A\beta_{1-42}$-bindender Proteine innerhalb des Komplexes verändert sein, sondern bei gleicher Primärstruktur könnte die Affinität der Bindung in Abhängigkeit der Konformation von $A\beta_{1-42}$ unterschiedlich hoch sein.

Der Befund, dass bei Patienten mit und ohne AD ein spezifischer Unterschied in der detergentien-dissoziierbaren Fraktion von $A\beta_{1-42}$ im Liquor nachweisbar wird, kann auch für andere Verfahren der neurochemischen Demenzdiagnostik genutzt werden (ELISA, Fluoreszenzkorrelationsspektroskopie).

Besonders vielversprechend ist der Einsatz des ELISA-Triplett $A\beta_{1-38}$, $A\beta_{1-40}$ und $A\beta_{1-42}$ mit Berechnung der Aβ-Peptidquotienten (38/40, 42/38, 42/40) und Bestimmung der KPabhängigen Erniedrigung der Aβ-Peptide durch differentielle Probenvorbehandlung.

### 1.3 Aβ-SDS-PAGE/Immunoblot und neuropathologische Diagnostik

Der Aβ-SDS-PAGE/Immunoblot kann für die neuropathologische *post mortem* Diagnostik demenentieller Erkrankungen eingesetzt werden. Bei Analyse der Detergentien(RIPA)-löslichen Fraktion von Aβ-Peptiden in Hirnhomogenaten von Patienten mit AD, anderen dementiellen Erkrankungen und Kontrollen können krankheits- und hirnregional-spezifische Expressionsmuster der Aβ-Peptide 1-37, 1-38, 1-40, 1-42 und 2-42 gezeigt werden. Auffällig ist insbesondere die massive Erhöhung von $A\beta_{2-42}$ in der RIPA-löslichen Fraktion der Hirnhomogenate bei AD und Patienten mit Lewy-Körperchen-Demenz (LBD). Bei LBD werden diese hohen Konzentrationen von $A\beta_{2-42}$ beobachtet, wenn die Patienten gleichzeitig eine ausgeprägte β-Amyloidpathologie zeigen (LBD, CERAD C). Auch $A\beta_{1-42}$ ist bei AD und LBD (CERAD C) regelmäßig und deutlich erhöht. Dabei zeigte die Konzentration der übrigen Aβ-Peptide eine hohe interindividuelle Varianz. Dies könnte ein Hinweis auf phänotypische Subtypen der sporadischen AD sein, oder verlaufsabhängig den Scheregrad der Demenz anzeigen.

Der hier verwendete RIPA-Detergentienmix ist nicht in der Lage, reife neuritische β-Amyloidplaques zu solubilisieren. Die extensiv erhöhten Konzentrationen von $A\beta_{2-42}$ können daher nicht über $A\beta_{2-42}$ aus dieser β-Amyloidplaquefraktion erklärt werden. Entsprechend ist auch unwahrscheinlich, dass $A\beta_{2-42}$ überwiegend durch unspezifische β-Amyloidplaqueassoziierte posttranslationale Veränderungen entsteht. Die hohen intrazerebralen Konzentrationen von $A\beta_{2-42}$ sind pathophysiologisch relevant, da das Fehlen von Aspartat die Aggregationsneigung von $A\beta_{1-42}$ erhöht und diese N-terminale Modifikation anscheinend der Bildung reifer β-Amyloidplaques voraus geht.

### 1.4 Quantifizierung von APP-Metaboliten mittels Aβ-SDS-PAGE/ Immunoblot in Zellkultur- und Tiermodellen

Die carboyterminal verkürzten Aß-Peptide 1-37, 1-38 und 1-39 konnten auch regelhaft im cisternalen Liquor von Meerschweinchen und Kaninchen nachgewiesen werden. Weiter gelang der Nachweis in Homogenaten und Überständen (Kurzzeitkultur) von hippokampalen Gewebeschnitten des adulten Meerschweinchens.

Weiter konnte eine neuartige neuronale (telenzephale) Primärkultur des Hühnchens etabliert und gezeigt werden, dass auch hier das Aβ-Peptidquintett mit vergleichbarer relativer Verteilung wie im humanen Liquor in die Überstände freigesetzt wird.

Das Aβ-Peptidquntett - und zusätzlich $A\beta_{2-42}$ - kann auch in den Überständen einer Neuroglioma Tumorzellinie (H4) nachgewiesen werden, die humanes APP751 mit schwedischer Doppelmutation ($_{human}APP751_{Sw}$) überexprimiert. Nach Behandlung der Zellen mit Proteaseinhibitoren, die potentielle Inhibitoren von β-/γ-Sekretasen sind, konnte neben der bekannten dosisabhängigen Reduktion von $A\beta_{1-40}$ und $A\beta_{1-42}$ auch eine Reduktion der C-terminal verkürzten Aβ-Peptide 1-37, 1-38, und 1-39 nachgewiesen werden. Zusätzlich wurde die Entstehung von $A\beta_{2-42}$ gehemmt. Interessant war in diesem Zusammenhang, dass die Entstehung der Ct-verkürzen Aβ-Peptide - besonders deutlich für $A\beta_{1-37}$ - im Vergleich zu $A\beta_{1-40}$ und $A\beta_{1-42}$ mit anderer Kinetik und schon früher gehemmt wurden. Dieser Effekt wurde besonders deutlich bei Betrachtung der Anteile der einzelnen Aβ-Peptidspecies an ihrer Gesamtkonzentration. Hier zeigt sich eine interessante Analogie zu den oben diskutieren krankheitsspezifischen Veränderungen der Aβ-Peptide im Liquor, die auch wesentlich sensitiver über die prozentualen Peptidanteile im Liquor erfasst werden konnten. Entsprechend kann vermutet werden, dass eine Heterogenität der γ-Sekretaseaktivität über Veränderungen in der relativen Zusammensetzung des Aβ-Peptidquintetts abgebildet werden kann. Dies ist relevant für Wirkstoffindungsprojekte zur Identifizierung isoformspezifische γ-Sekretaseinhibitoren.

Bei Behandlung der transgenen H4 Neuroglioma Zellkultur mit Calpaininbitor-1 konnte bei niedriger Konzentration des Proteaseinhibitors die vorbeschriebene initiale (paradoxe) Konzentrationssteigerung von $A\beta_{1-42}$ belegt werden. Interessant ist hier, dass der Anstieg von $A\beta_{1-42}$ nicht mit einer Konzentrationssteigerung von $A\beta_{2-42}$ korreliert war. Dieser Befund spricht gegen eine sekundäre Entstehung von $A\beta_{2-42}$ aus $A\beta_{1-42}$ und für die Hypothese, dass $A\beta_{2-42}$ durch einen kombinierten β-/γ-Sekretaseschnitt entsteht. In Zusammenhang mit der deutlich und regelmäßig erhöhten Konzentration von $A\beta_{2-42}$ in Hirnhomogenaten bei AD und dem Nachweis von $A\beta_{2-42}$ in Liquorproben von Patienten mit AD stellt sich die Frage, ob bei AD eine bestimmte Isoform der β-Sekretase (BACE) überexprimiert wird oder das physiologisch entstehende $A\beta_{2-42}$ bei AD vermindert katabolisiert wird.

## 2. Hintergrund

Die molekularen Grundlagen der AD, ihr Bezug zu neueren medikamentösen Ansätzen der Demenzerkrankung und Verfahren der neurochemischen Demenzdiagnostik sind in zwei Übersichtsarbeiten zusammengefaßt (Wiltfang et al., 1998 und 2000).

Bisher wurde ein weiteres SDS-PAGE/Immunoblot Verfahren für die Analyse von $A\beta_{1-40}$ und $A\beta_{1-42}$/1-43 in humanem lumbalen Liquor beschrieben (Ida et al., 1996). Die Differenzierung der Aβ-Peptide kann hier jedoch nicht durch die elektrophoretische Trennung vorgenommen werden, sondern erfolgt auf der Blot-Membran durch C-terminal selektive monoklonale Antikörper. Gleichzeitig muß $A\beta_{1-42}$ vor der Trennung durch Einengung der Probe konzentriert werden. Die Einengung erfolgt ohne vorherige SDS-Denaturierung, was bedingt durch die hohe Aggregationsneigung von $A\beta_{1-42}$ methodisch problematisch erscheint. Für die Bestimmung von $A\beta_{1-40}$ und 1-42 müssen bei dieser Methodik getrennte Elektrophoresen durchgeführt werden.

Das für den Aβ-SDS-PAGE/Immunoblot eingesetzte multiphasische Puffersystem (Wiltfang et al., 1991), kombiniert die Vorteile spezieller Puffersysteme für Proteine (Laemmli, 1970) und Peptide (Schagger and von Jagow, 1987). Entsprechend können mit diesem SDS-PAGE Verfahren sowohl Proteine als auch Peptide in einem homogenen Polyacrylamidtrenngelsystem mit hoher Auflösung aufgetrennt werden. Weiter wurde unter Verwendung der Harnstoffversion (Wiltfang et al., 1991) des letztgenannten SDS-PAGE Verfahrens die elektrophoretische Trennung von $A\beta_{1-40}$ und $A\beta_{1-42}$ beschrieben (Klafki et al., 1996). Die Anwendung dieses Verfahrens auf Zellkulturmodelle des familiären AD, die mit Inhibitoren APP-spaltender Enzyme (γ-Sekretase) vorbehandelt wurden, zeigte mittels Nachweis der *in vivo* radioaktiv markierten Aβ-Peptide 1-40 und 1-42, dass unterschiedliche γ-Sekretasen an der enzymatischen Entstehung von $A\beta_{1-42}$ beteiligt sind (Klafki et al., 1996). Mit einer Modifikation dieses Systems konnte auch die Trennung zwischen $A\beta_{1-42}$ und $A\beta_{1-43}$ realisiert werden (Wiltfang et al., 1997). Die maximale immunologische Nachweisempfindlichkeit des letztgenannten Verfahrens lag bei 50 pg $A\beta_{1-42}$, was für die hier gezeigten Applikationen bei weitem nicht ausreicht. Gleichzeitig wurde in dem hier vorgestellten Verfahren die Trenngelmatrix optimiert, um zusätzliche N-terminal und C-terminal modifizierte Aβ-Peptide aufzutrennen.

Die SDS-PAGE läßt sich als zweite analytische Dimension mit der isoelektrischen Fokussierung (IEF) in der ersten Dimension als 2D-PAGE kombinieren (O'Farrell, 1975; O'Farrell et al., 1977). Damit gelingt eine zweidimensionale Auftrennung von Polypeptiden und Proteinen nach isoelektrischem Punkt und effektivem Molekularradius. Die isoelektrische Fokussierung, bei Verwendung hochgradig gespreizter immobilisierter pH-Gradienten (Gorg et al., 1995; Gorg et al., 1997; Righetti and Bossi, 1997), kann minimale Ladungsunterschiede aufdecken. Der zweidimensionale Aβ-SDS-PAGE/Immunoblot kann daher auch für die hochauflösende Analyse von posttranslationalen Modifikationen der APP-Metabolite eingesetzt werden. Gleichzeitig kann eine Nachweisempfindlichkeit im oberen Femtogrammbereich realisiert werden. Posttranslationale Modifikationen können in spezifischer Weise das Aggregationsverhalten von Aβ-Peptiden beeinflussen und sind damit pathophysiologisch und diagnostisch relevant (Thome et al., 1996; Thome J., 1996; Kuo et al., 1997; Russo et al., 1997; Tamaoka et al., 1997). Für die neurochemische Demenzdiagnostik ist relevant, dass selektiv der Anteil von N-terminal modifiziertem $A\beta_{X-42/43}$ zu $A\beta_{1-42/43}$, nicht jedoch der Anteil N-terminal modifiziertes $A\beta_{X-40}$ zu $A\beta_{1-40}$ ansteigt (Tamaoka et al., 1997). Zusätzlich zur Bestimmung der Aβ-Peptide erlaubt der Aβ-SDS-PAGE/Immunoblot auch die Quantifizierung von sAPPα, dass bei AD im Liquor erniedrigt gemessen wird (Sennvik et al., 2000). Für die Bestimmung von sAPPα wird das harnstoffhaltige Trenngelkompartiment mit einem oberen (kathodischen) Trenngel ohne Harnstoff und größerer Porengröße kombiniert.

Der quantitative Aβ-SDS-PAGE/Immunoblot erlaubt die gleichzeitige und ultrasensitive Bestimmung eines Spektrums von APP-Metaboliten, die hohe Relevanz für die neurochemische Frühdiagnostik und Pathogenese der AD besitzen. Das Verfahren kann auch innerhalb der tierexperimentellen und klinischen Evaluation neuer Pharmaka eingesetzt werden, die in den Metabolismus oder Katabolismus von Aβ-Peptiden eingreifen.

## 3 Materialien und Methoden

### 3.1 Aβ-SDS-PAGE

#### 3.1.1 Material und Reagentien

Bio-RAD (Richmond, CA, USA): Mini Protean II Elektrophorese System, Acrylamid (Best.-Nr. 161-0101), N,N'-Methylen-bis-acrylamid (Best.-Nr. 161-0201); Merck (Darmstadt, Deutschland): Ammoniumperoxodisulfat (AMPS, Best.-Nr. 1201.1000), Bromphenolblau (Best.-Nr. 8122), 0.5 M $H_2SO_4$ (Best.-Nr. 1.09072.1000), Natriumhydroxid Plätzchen p.A. (NaOH, Best.-Nr. 6498), Aktivkohle p.A. (Best.-Nr. 1.02186.0250), Saccharose (Best.-Nr. 1.07654.1000)

Paesel+Lorei (Hanau, Deutschland): Tris ultra rein (Best.-Nr. 100840)

Biomol (Hamburg, Deutschland): Natrium Laurylsulfat, ultra rein, 2x cryst. (SDS, Best.-Nr. 51430), Bis-(2-hydroxyethyl)-imino-tris(hydroxymethyl) methan (Bis-Tris, Best.-Nr. 50003), N,N'-Bis-(2-hydroxyethyl)glycine p.A. (Bicine, Best.-Nr. 01848); GibcoBRL/Life Technologies (Karlsruhe, Deutschland): Harnstoff (Best.-Nr. 15716-012);

Serva (Heidelberg, Deutschland): N,N,N',N'-Tetramethylethylen-di-Amin (TEMED, Best.-Nr. 35925); Sigma (Steinheim, Deutschland): 2-Mercaptoethanol (Best.-Nr. M-7154);

Bachem (Bubendorf, Schweiz): $A\beta_{1-38}$, $A\beta_{1-40}$, $A\beta_{1-42}$

Forschungsinstitut für Molekulare Pharmakologie (Berlin, Deutschland): $A\beta_{1-33}$, $A\beta_{1-34}$, $A\beta_{1-35}$, $A\beta_{1-37}$, $A\beta_{1-39}$, $A\beta_{2-40}$, $A\beta_{2-42}$, $A\beta_{3-40}$, $A\beta_{3-42}$, $A\beta_{3p-40}$, $A\beta_{3-42}$

Amersham Pharmacia Biotech AB (Buckinghamshire, England) und Serva (Heidelberg, Deutschland): Trypsin Inhibitor Rinderlunge ($M_r$ 6500), Mellitin ($M_r$ 2847) und Met-Lys-Bradykinin ($M_r$ 1320) wurden über Serva bezogen und dem "low-molecular-weight (LMW) marker kit" von Amersham Pharmacia zugefügt. Das LMW Kit setzt sich zusammen aus: Phosphorylase b ($M_r$ 94000), Rinderserum Albumin ($M_r$ 67000), Ovalbumin ($M_r$ 43000), Carbonic Anhydrase ($M_r$ 30000), Trypsin Inhibitor, Sojabohne ($M_r$ 20100) und α-Lactalbumin ($M_r$ 14400).

#### 3.1.2 Gelzusammensetzung und Elektrophorese

Die SDS-PAGE wurde durchgeführt mittels des Bio-Rad Mini Protean II Elektrophorese System. Die Größe der verwendeten Gelkompartimente war wie folgt: Trenngellänge etwa 54 mm; Sammelgellänge etwa 5 mm (entsprechend einem Volumen von 250 µl); Kammgelhöhe etwa 12-15 mm; Geldicke jeweils 0.50 mm, Gelbreite jeweils 85 mm. Trenn- und Sammgele für die zweite analytische Dimension innerhalb der Aβ-2D-PAGE haben eine Geldicke von 1.0 mm.

Für den Probenauftrag wird ein 15-zähniger Probenauftragskamm verwendet (Zahnbreite ca. 3 mm, Zahnabstand 2 mm). Die resultierende Probenauftragstasche im Kammgel mißt etwa 3x10 mm. Die max. Probenauftragsmenge sollte 10 µl nicht überschreiten, um Verschleppungen zwischen den Probenschächten sicher zu verhindern. Die Proben werden nach Einfüllen des Kathodenpuffers unterschichtet. Elektrophorese: a) 12 mA / 0.5 mm Geldicke bei konstanter Stromstärke über 2h, b) 1,0 mm Gele der zweiten analytischen Dimension: 60 Volt / 1,0 mm Geldicke für 10 min, 120 Volt / 1,0 mm Geldicke über 1h 45min.

Für die Trenngelkompartimente wurde die Harnstoffversion des Bicin/Tris SDS-PAGE Verfahrens von Wiltfang et al. (Wiltfang et al., 1991) verwendet und für die vorgestellten Applikationen in wesentlichen Aspekten modifiziert. Tabelle 1 faßt die konzentrierten Puffer für die Gelkompartimente, Kathodenpuffer, Anodenpuffer und die Acrylamidstammlösung zusammen. Die Gelzusammensetzung für die Aβ-SDS-PAGE zur optimierten Trennung von APP-Metaboliten und Aβ-Peptiden in humanen oder tierischen biologischen Proben findet sich in Tabelle 2.

#### 3.1.3 Probenvorbereitung für Aβ-SDS-PAGE

*3.1.3.1 Aufnahme von Liquorproben*

300µl Aliquots des SDS-SB-3 ohne 2-Mercaptoethanol (Tabelle 3) werden mittels Speed-Vac in 1.5 ml Eppendorf Probengefäßen ("safe lock") auf Trockensubstanz eingeengt und bis zum Gebrauch bei Raumtemperatur gelagert. Liquorproben werden aliquotiert und mittels des in den Eppendorfgefäßen als Trockensubstanz vorgelegten SDS-SB-3 unterschiedlich aufgearbeitet:

(a) Nativ eingefrorener Liquor
330 µl zentrifugierter Liquor wird nativ bei -80°C in 1.5 ml Eppendorfgefäßen eingefroren und gelagert. Nach Auftauen und Vortexschritt wird mit 300 µl Liquor und 2.5% v/v 2-Mercaptoethanol der vorgelegte SDS-SB-3 aufgenommen und nach Vortexschritt für 5 min bei 95°C erhitzt. Anschließend erfolgt die Aβ-SDSPAGE.

(b) Vorbehandlung mittels SDS-/Hitzedenaturierung
Der als Trockensubstanz vorgelegte SDS-SB-3 wird mit 300 µl zentrifugiertem Liquor aufgenommen und nach Vortexschritt für 5 min. bei 95°C erhitzt (keine Zugabe von 2-Mercaptoethanol !). Anschließend wird der SDS-/Hitzedenaturierte Liquor bei -80°C gelagert. Vor Aβ-SDS-PAGE wird die Probe nach Zugabe von 2.5% v/v 2-Mercaptoethanol erneut für 5 min. bei 95°C erhitzt.

(c) Einengung von Liquorproben für die Bestimmung von Aβ-Peptiden
Nach SDS-/Hitzedenaturierung, aber vor Zugabe von 2-Mercaptoethanol, kann die Liquorprobe, oder auch andere biologische Proben, mittels SpeedVac auf Trockensubstanz eingeengt werden und mit 100 µl $H_2O_{dd}$ und 2.5% v/v 2-Mercaptoethanol aufgenommen werden (3-fache Konzentrierung). Vor Aβ-SDS-PAGE erfolgt erneut Erhitzen auf 95°C für 5 min.

Durch die SDS-/Hitzedenaturierung vor Einengung der Probe sollen Proteolyse, Präzipitation und Autoaggregation der Aβ-Peptide während der Konzentrierung vermieden werden.

Die reduzierte SDS-Konzentration in SDS-SB-3 ist erforderlich, weil höhere SDS-Konzentrationen nach dreifacher Einengung der Proben und bei einem Auftragsvolumen von ca. 10 µl zu einem beeinträchtigen Laufverhalten der Aβ-Peptide am anodischen Ende des harnstoffhaltigen Trenngels führen. Gleichzeitig ist die SDS-Konzentration von 0.5% w/v in SDS-SB-3 aber noch ausreichend hoch für eine vollständige SDS-/Hitzedenaturierung der Probe.

*3.1.3.2 Aufnahme anderer biologischer Proben*

Liegen die Proben flüssig vor (z.B. Zellkulturüberstände, Zellhomogenate) und ist die Aβ-Peptidkonzentration ausreichend hoch kann eine Volumeneinheit Probe mit einer Volumeneinheit des zweifach konzentrierten SDS-SB-2 aufgenommen werden (Tabelle 3).

*3.1.3.3 Aufnahme der Proben nach Immunopräzipitation (IP)*

Die mittels magnetischen Dynabeads (s.u.) immobilisierten APP-Metabolite werden nach dem abschließenden Waschschritt bei 37°C für 10 min im Ultraschallbad unter Verwendung des SDS-SB-1 oder SDS-PB-3 (jeweils ohne 2-Mercaptoethanol !) aus der Antigenbindung eluiert. Nach Zugabe von 2-Mercaptoethanol auf 2.5% w/v, erfolgt erhitzen auf 95°C für 5 min. Bei Verwendung von SDS-PB-3 können die Proben anschließend durch Einengung auf Trockensubstanz und Aufnahme mit $H_2O_{dd}$ mittels SpeedVac nochmals dreifach konzentriert werden.

**3.2 Konventionelle Aβ-2D-PAGE (Trägerampholyt IEF)**

Die Trägerampholyt-IEF in Rundgelen der ersten analytischen Dimension und die vertikale Aβ-SDS-PAGE der zweiten analytischen Dimension werden mit dem Mini-Protean II 2-D Cell System von Bio-Rad durchgeführt.

**3.2.1 Materialien und Reagenzien für die Trägerampholyt IEF**

Bio-RAD (Richmond, CA, USA): Mini-Protean II 2-D Cell System, Glasröhrchen (∅ 1mm), Agarose (162-0017);
Merck (Darmstadt, Deutschland): CHAPS (Best.-Nr. 1.11662.0010), Natriumhydroxid Plätzchen p.A. (NaOH, Best.-Nr. 6498), Bromphenolblau (Best.-Nr. 8122), Phosphorsäure 85% ($H_3PO_4$, Best.-Nr. 1.00573.1000); Serva (Heidelberg, Deutschland): Servalyt® pH 5-6 (Best.-Nr. 42924) pH 4-7 (Best.-Nr. 42948) pH 3-10 (Best.-Nr. 42951);
Fluka (Buchs, Schweiz): Igepal CA 630 (NP 40, Best.-Nr. 56741)
GibcoBRL/Life Technologies (Karlsruhe, Deutschland): Harnstoff (Best.-Nr. 15716-012);
Sigma (Steinheim, Deutschland): 2-Mercaptoethanol (Best.-Nr. M-7154)
Biomol (Hamburg, Deutschland): Natrium Laurylsulfat, ultra rein, 2x cryst. (SDS, Best.-Nr. 51430), Bis-(2-hydroxyethyl)-imino-tris(hydroxymethyl) methan (Bis-Tris, Best.-Nr. 50003), N,N'-Bis-(2-hydroxyethyl) glycin p.A. (Bicine, Best.-Nr. 01848)

### 3.2.2 Probenaufnahme für die Trägerampholyt IEF

Die Probenaufnahme erfolgt in IEF-SB (Tabelle 4a). Trockensubstanz und mittels MSP magnetisch immobilisierte Dynabeads (s.u.) werden direkt mit dem IEF-SB unmittelbar vor IEF aufgenommen und 10 min. im Ultraschallbad bei 37° C inkubiert. Für die Aufnahme von Liquorproben wird eine Volumeneinheit Liquor mit einer Volumeneinheit IEF-SB aufgenommen und 10 min. im Ultraschallbad bei 37° C inkubiert.

### 3.2.3 Erste analytische Dimension: Trägerampholyt IEF in Rundgelen

Glasröhrchen (∅ 1mm) werden zur Gelpolymerisation mit der Monomerlösung aus Tabelle 4b gefüllt. Die IEF Rundgele werden auf eine Länge von 60 mm polymerisiert. 20 µl Probe nach direkter Aufnahme in IEF-SB (10 µl Liquor plus 10 µl IEF-SB) oder 10 µl Eluat aus der Immunopräzipitation in IEF-SB (Tabelle 4a) werden aufgetragen und mit dem kathodischen Elektrolyten überschichtet. Diese Seite der Glasröhrchen wird mit der oberen kathodischen Elektrolytkammer verbunden. Die Zusammensetzung von Anolyt und Katholyt für die Trägerampholyt IEF findet sich in Tabelle 4c.

Anschließend wird wie folgt bei Raumtemperatur fokussiert: 100V x 1h, 200Vx11h, 500Vx2h, 1000Vx1h (Σ 4300 Vxh).

Nach IEF werden die Rundgele mittels Wasserdruck aus den Glasröhrchen ausgestoßen und für 5 min. im IEF-Equilibrierungspuffer (Tabelle 4d) bei Raumtemperatur inkubiert. Danach werden die IEF Gele auf das Sammelgel (s.o.) der Aβ-SDS-PAGE aufgelegt und mit der IEF-Agaroselösung (Tabelle 4d) in ihrer Position fixiert. Eine Probentasche für den vergleichenden Auftrag von synthetischen Aβ-Peptiden oder $M_r$ Markerproteinen wird mittels eines Teflonzahns in der heißen Agarose geformt.

### 3.2.4 Zweite analytische Dimension: Aβ-SDS-PAGE

Die Aβ-SDS-PAGE erfolgte, wie in Tabelle 1 und 2 ausgeführt (Trenngel: 12%T/5%C/8M Harnstoff). Ein Kammgel wird nicht polymerisiert. Die Geldicke der verwendeten Trenngele beträgt 1mm. Die Elektrophorese erfolgt bei Raumtemperatur: 10 min. / 60 V, 90 min. / 120 V.

## 3.3 Aβ-IPG-2D-PAGE

### 3.3.1 Material und Reagentien

<u>GibcoBRL/Life Technologies (Karlsruhe, Deutschland):</u> Harnstoff (Best.-Nr. 15716-012)
<u>Merck (Darmstadt, Deutschland):</u> CHAPS (Best.-Nr. 1.11662.0010), Bromphenolblau (Best.-Nr. 8122), Glyzerin (100%) (Best.-Nr. 1.04092.1000)
<u>Biomol (Hamburg, Deutschland):</u> Natrium Laurylsulfat, ultra rein, 2x cryst. (SDS, Best.-Nr. 51430); <u>Serva (Heidelberg, Deutschland):</u> Serdolit MB-1 (Best.-Nr. 40701), Dithiotreitol (DTT, Best.-Nr. 20710)
<u>Amersham Pharmacia Biotech AB (AB (Buckinghamshire, England):</u> Pharmalyte pH 3-10 (Best.-Nr. 17-0456-01), Pharmalyte pH 4-6,5 (Best.-Nr. 17-0452-01), Immobiline DryStrip 70 x 3 x 0.5 mm, pH 4-7 L (Best.-Nr. 17-6001-10);
<u>Sigma (Steinheim, Deutschland):</u> Iodoacetamide (Best.-Nr. I-6125)
<u>BioRAD (Richmond, CA, USA):</u> Agarose (162-0017)

### 3.3.2 Probenaufnahme

Die Probenaufnahme erfolgt in IPG-SB (Tabelle 5a). Trockensubstanz und mittels MSP magnetisch immobilisierte Dynabeads (s.u.) werden direkt mit dem IPG-SB unmittelbar vor IEF aufgenommen und 10 min. im Ultraschallbad bei 37° C inkubiert. Aufnahme flüssiger biologischer Proben: Nach Entfernung des Mischbettionenaustauschers (Serdolit MB-1) wird der IPB-SB in Eppendorf Probengefäßen aliquotiert (z.B. 100 µl) und mittels SpeedVac bei Raumtemperatur auf Trockensubstanz eingeengt. Der als Trockensubstanz vorgelegte IPG-SB wird im Volumenverhältnis 1:1 mit Probe aufgenommen (z.B. 100 µl) und nach Vortexschritt (1 min.) für 10 min. im Ultraschallbad bei 37° C inkubiert.

### 3.3.3 IPG-IEF

Die IEF mittels kommerzieller IPG "DryStrips" erfolgte entsprechend dem Protokoll des Herstellers (Amersham Pharmacia Biotech / Kurzanleitung 71-5009-57, Ausgabe AA, 99-04). IPG "DryStrips" (4-7, linearer pH-Gradient, Länge 7cm) wurden über Nacht bei Raumtemperatur unter Verwendung der Rehydrierungslösung aus Tabelle 5a auf eine Geldicke von 0.5 mm rehydriert. Die Probenauftragsvorrichtung ("sample cups") wird auf die basische Seite der IPG Streifen, etwa bei pH 6.5, aufgelegt (kathodischer Auftrag) und 30 µl Probe aufgetragen. Die IPG-IEF erfolgt für 30 min / 300V, 30 min / 800V, 30 min. / 1400V und 5 h / 2000V (Σ 12500 Vxh).

### 3.3.4 Zweite analytische Dimension: Aβ-SDS-PAGE

Nach IPG-IEF werden die "DryStrips" für 2x10 min. equilibriert (Tabelle 5b). Die erste Equilibrierungslösung enthält DTT (50 mg/5ml), die zweite Lösung Jodacetamid (240 mg / 5 ml) zur Neurtralisierung von überschüssigem DTT, das sonst bei Silberfärbung der Gele zu Färbeartefakten führt. Bei Western-Immunoblot entfällt der zweite Equilibrierungsschritt. Die eqilibrierten IPG "DryStrips" werden auf dem Sammelgel der Aβ-SDS-PAGE mittels Agaroselösung (Tabelle 5c) fixiert. Eine Probentasche für den vergleichenden Auftrag von synthetischen Aβ-Peptiden oder $M_r$ Markerproteinen wird mittels eines Teflonzahns in der heißen Agarose angelegt. Die Elektrophorese erfolgt entsprechend 3.1.2

## 3.4 Immunopräzipitation

### 3.4.1 Material und Reagentien, Antikörper

Biochrom KG (Berlin, Deutschland): HEPES (Best.-Nr. L1603), PBS Dulbecco, ohne $Ca^{++}$, ohne $Mg^{++}$ (Best.-Nr. L182-50); Merck (Darmstadt, Deutschland): Natriumhydroxid Plätzchen p.A. (NaOH, Best.-Nr. 6498); Natriumchlorid (NaCL, Best.-Nr. 1.01540.0500);
Fluka (Buchs, Schweiz): Igepal CA 630 (NP 40, Best.-Nr. 56741), Natrium Desoxycholate (Na-DOC, Best.-Nr. 30968); Biomol (Hamburg, Deutschland): Natrium Laurylsulfat ultra rein, 2x cryst. (SDS, Best.-Nr. 51430);
Boehringer (Mannheim, Deutschland): Proteinase inhibitor cocktail tablets, complete™ Mini (Best.-Nr. 1836153);
Deutsche Dynal GmbH (Hamburg, Deutschland): Dynabeads® M280 Schaf anti-Mouse IgG (Best.-Nr. 112.02);
Biometra (Göttingen, Deutschland): Magnetic Separation Stand (MPS); Sigma (Steinheim, Deutschland): Bovines Albumin (BSA, Best.-Nr. A-4378), 2-Mercaptoethanol (Best.-Nr. M-7154), Natrium Azid (Na-Azid, Best.-Nr. A-2002);
Paesel+Lorei (Hanau, Deutschland): Tris ultra rein (Best.-Nr. 100840); Schering AG (Berlin, Deutschland): mAb1E8 (Maus IgG1); Senetek PLC Drug Delivery Technologies, Inc. St. Louis, Mo, USA): mAb 6E10, gereinigter Maus IgG1 (Best.-Nr. 320-02).

### 3.4.2 Vorbereitung der Dynabeads M-280 (Schaf Anti-Maus IgG)

250µl Suspension (6.7 x $10^8$ beads/ml) ohne Schaumbildung gut schütteln und 3 x 5 min. mit 1 ml PBS/BSA (0.15 M NaCl in 0.01 M Na-Phosphat, 0.1 % w/v BSA) waschen. Beads im Magnetic Separation Stand (MSP) der Firma Biometra (Göttingen, Deutschland) immobilisieren und Überstand abnehmen.

### 3.4.3 Vorbehandlung der biologischen Proben

0.75 Volumeneinheiten Probe werden mit 0.25 Volumeneinheiten Protein-Inhibitor-Cocktail Stammlösung (PI-Stammlsg.) aufgenommen. PI-Stammlsg.: Löse 1 Tabl. Complete™ Mini in 1.5 ml $H_2O_{dd}$.

### 3.4.4 mAb-Aktivierung der magnetischen Mikropartikel ("beads") mittels der direkten IP-Methode

Etwa 1.675 x $10^8$ "beads" (250 µl vorbereitete Bead-Suspension, s.o.) werden im MSP in 1.5 ml Eppendorf Cups wandständig magnetisch immobilisiert und mit 7.5 µg mAb 6E10 (Senetek Drug Delivery Technologies, Inc., St. Louis, Mo, USA) oder 10 µg mAb 1E8 (Schering AG, Berlin, Deutschland) in 250 µl PBS/BSA bei 4 °C für 20 h inkubiert. Anschließend für 4 x 30 min. mit 1 ml PBS/BSA gewaschen und am Ende in 250 µl PBSBSA/0.01% Na-Azid aufgenommen und bei 4 °C bis zum Einsatz für die Immunopräzipitation gelagert. Die so aktivierten Beads können ohne nennenswerten Kapazitätsverlust bis zu drei Monate gelagert werden. Direkt vor Einsatz in die Immunopräzipitation biologischer Proben werden die aktivierten Beads 3x3 min. mit 250 µl PBS/BSA ohne Zusatz von Na-Azid gewaschen.

### 3.4.5 Immunopräzipation aus humanem Liquor

**a) ohne Detergentien**

25 µl aktivierter DynaBeads (ca. 1.675 x$10^7$ beads) werden mit 268 µl Liquor/PI-Stammlsg. (200 µl Liquor + 68 µl PI-Stammlsg.) vermischt und mit 732 µl 50 mM HEPES-Puffer, pH 7.4, in Eppendorf-Cups auf 1 ml gebracht. Die Inkubation erfolgt für 20 h bei 4 °C auf einem Schüttelmixer (ständige Agitation der Probe). Anschließend werden die Beads im MSP-Stand immobilisiert und der Überstand entfernt. Danach werden die Beads 4 x 5 min. bei Raumtenperatur mit 1 ml PBS/0.1%BSA gewaschen. Abschließend werden die Beads 1 x 3 min. bei Raumtenperatur in 1 ml 10 mM Tris/HCl (pH 7.5) gewaschen. Für die Aβ-SDS-PAGE erfolgt die Probenaufmahme der magnetisch immobilisierten Beads mit 25 µl SDS-PB-1 für 5 min. bei 95 °C. Für die Aβ-2D-PAGE wird mit 25 µl IEF-SB oder IPG-SB aufgenommen und für 10 min. bei 37 °C im Ultraschallbad inkubiert. Für die Aβ-SDS-PAGE werden 4 µl Probe aufgetragen, entsprechend der Aβ-Peptidmenge, die in 32 µl Liquorvolumen vorhanden ist. Für die Aβ-2D-PAGE werden 10 µl aufgetragen, entsprechend der Aβ-Peptidmenge die in 80 µl Liquorvolumen vorhanden ist.

**b) mit Detergentien ($RIPA_{0.5x}$-IP)**

200 µl Liquor werden mit 200 µl 5fach-konzentriertem $RIPA_{0.5x}$ Puffer (Tabelle 6) vermischt und mit 600 µl $H_2O_{dd}$ in Eppendorf Cups auf 1ml gebracht. Die Durchführung der Immunpräzipitation entspricht der unter A beschriebenen Methode. Der $RIPA_{0.5x}$-Puffer enthält Proteaseinhibitoren (Tabelle 6).

### 3.4.6 Immunopräzipation aus humanem Gehirngewebe ($RIPA_{1x}$-IP)

Gehirngewebe (ca. 50 mg) wird mit 1ml $RIPA_{1x}$ Puffer (Tabelle 6) in 1.5 ml Eppendorf Reaktionsgefäßen mittels Ultraschallfinger homogenisiert und anschließend für 5 min. (4°C) bei 20000 g zentrifugiert. Der Überstand wird abgenommen und der Proteingehalt des Homogenatüberstands wird auf 3 mg/ml mit $RIPA_{1x}$ Puffer eingestellt und 1 ml Hirnhomogenat zusammen mit 50 µl aktivierten DynaBeads (ca. 3.35 x$10^7$ beads) wie unter (a) beschrieben immunpräzipitiert. Der $RIPA_{1.0x}$-Puffer enthält Proteaseinhibitoren (Tabelle 6).

### 3.4.7 Immunopräzipation aus Zellkulturüberständen ($RIPA_{0.5x}$-IP)

400 µl Zellkulturüberstand werden mit 100 µl 5fach-konzentriertem $RIPA_{0.5x}$ Puffer (alternativ: 800 µl Zellkulturüberstand mit 200 µl 5fach-konzentriertem $RIPA_{0.5x}$ Puffer) und 25 µl aktivierten DynaBeads vermischt und wie unter (a) beschrieben immunpräzipitiert. Für die Aβ-SDS-PAGE werden 6 µl Probe aufgetragen, entsprechend der Aβ-Peptidmenge, die in 96 µl Zellkulturüberstandvolumen vorhanden ist.

### 3.5 Fixierung und Silberfärbung

### 3.5.1 Reagentien

Merck (Darmstad, Deutschland): Natriumthiosulfat-Pentahydrat pA ($Na_2S_2O_3$, Best.-Nr. 1.06516.0500), Natriumcarbonat pA ($Na_2CO_3$, Best.-Nr. 1.06392.1000), Glycin Puffersubstanz (Best.-Nr. 1.04169.0250), Formaldehyd min. 37% pA (Best.-Nr. 1.04003.1000), Glutardialdehyd 25%ig (Best.-Nr. 8.20603.0100), Natriumazetat wasserfrei (Best.-Nr. 1.06268.1000);
Paesel+Lorei (Hanau, Deutschland): Silbernitrat p.A. (Best.-Nr. 27-100-601);
Zentralapotheke der Universität Göttingen: Ethanol 99.9%, vergaellt

### 3.5.2 Durchführung

Nach Aβ-SDS-PAGE oder Aβ-2D-PAGE werden die Peptide und Proteine für 45 min bei Raumtemp. mit Glutardialdehyd in Borat/Phosphatpufer nach Wiltfang et al. (Wiltfang et al., 1997) fixiert. Die Silberfärbung wurde leicht modifiziert nach Heukeshoven et al. (Heukeshoven and Dernick, 1988) durchgeführt (Tabelle 7)

### 3.6 Western-Immunoblot

#### 3.6.1 Material und Reagentien, Antikörper

Paesel+Lorei (Hanau, Deutschland): Tris ultra rein (Best.-Nr. 100840); Sigma Steinheim, Deutschland): Borsäure (Borsäure, Best.-Nr. B-7901), Natrium Azid (Na-Azid, Best.-Nr. A-2002);J.T.Baker (Deventer, Holland): Methanol (Best.-Nr. 9263); BioRad Laboratories (Hercules, CA, USA): Filter Paper extra dick (Best.-Nr. 1703960), Non-Fat Dry Milk (Best.-Nr. 170-6404); Millipore Corporation (Bedford, MA, USA): Immobilon-P Transfer Membran (Best. -Nr. IPVH00010); Hoefer Pharmacia Biotech Inc. (San Francisco, CA, USA): SemiPhor semi-dry transfer unit (Best. -Nr. 80-6211-86); Biochrom KG (Berlin, Deutschland): PBS Dulbecco, ohne $Ca^{++}$, ohne $Mg^{++}$ (Best.-Nr. L182-50); Schering AG (Berlin, Deutschland): mAb 1E8 (Maus IgG1); Senetek PLC Drug Delivery Technologies, Inc. (St. Louis, Mo, USA): gereinigter mAb 6E10, Maus IgG1 (Best.-Nr. 320-02);
Roth (Karlsruhe, Deutschland): Roti-Block (Best.-Nr. A151.1).

#### 3.6.2 Durchführung des Western-Immunoblot

Im Anschluß an die Aβ-SDS-PAGE oder Aβ-2D-PAGE erfolgt der Transfer mittels Semidry-Westernblot und einem multiphasischen Puffersystem auf PVDF Nachweismembranen. Die Blotpuffer sind in Tabelle 8 zusammengestellt.
Der Aufbau des Blotsandwich von der Anode zur Kathode ist wie folgt: Eine Filterpapierlage mit Puffer A, eine Filterpapierlage und die PVDF-Membran mit Puffer B, Gel und abschließend zwei Filterpapierlagen mit Puffer C. Gele werden unmittelbar im Anschluß an die Elektrophorese für ca. 10 sec. in Puffer C inkubiert. Als Filterpapier wird extrastarkes Filterpapier der Firma BioRad verwendet. Nach Untersuchung von PVDF-Membranen unterschiedlicher Hersteller ergab die Immobilon-P Membran der Firma Millipore, die geringste Hintergrundfärbung und effektivste Immobilisation der Aβ-Peptide, insbesondere von $A\beta_{1-42}$, innerhalb des Immunoblotprotokolls. Die Immobilon-P Membranen werden entsprechend den Herstellerangaben vor Gebrauch mit Methanol benetzt, anschließend für 1 min. in $H_2O_{dd}$ inkubiert und dann in Puffer B überführt. Der Transfer erfolgt für Aβ-SDS-PAGE (∅ 0.5 mm) für 30 min. oder Aβ-2D-PAGE Gele (∅ 1.0 mm) für 45 min. bei Raumtemperatur mit 1 $mA/cm^2$.

Nach Ende des Western-Blot wird die Immobilon-P Membran für ca. 30 sec. in $H_2O_{dd}$ gewaschen und 3 min. in der Mikrowelle in PBS (ohne Tween-20 !) gekocht. Der Kochschritt ist essentiell, um die maximale Nachweisempfindlichkeit zu realisieren.

##### *3.6.2.1 Immunoblot-1 (Milchpulver-Blockschritt)*

Die für den Immunoblot-1 verwendeten Puffer, Lösungen und Antikörper sind in Tabelle 9a & b zusammengefaßt.

- Blockschritt: 1 h bei Raumtemperatur in 4 ml PBS-T-M / $cm^2$ Membran
- Inkubation mit primärem mAb: 15 h bei 4° C und abschließend 30 min. bei Raumtemp. in einer 1:4000 Verdünnung des mAb 1E8 (Schering AG, Berlin, Deutschland) oder in einer 1:1000 Verdünnung des mAb 6E10 (aufgereinigt: 1 mg/ml; Senetek Drug Delivery Technologies, Inc., St. Louis, Mo, USA) in 0.074 ml PBS-T-M /$cm^2$ (Einschweißen in Kunststoffolie, hochfrequenter Agitation mit Rotationsmixer)
- Waschschritt 1: 3x10 min. mit PBS-T (4 ml/$cm^2$) bei Raumtemp.
- Inkubation mit sekundärem mAb: 1 h bei Raumtemp. mit einer 1:3000 Verdünnung des sekundären mAb (biotinylierter anti-Maus IgG, Pferd, H+L; Vector Laboratories, Burlingame, CA, USA) in 0.074 ml PBS-T-M / $cm^2$ Membran (Einschweißen in Kunststoffolie, hochfrequente Agitation mit Rotationsmixer)
- Waschschritt 2: wie Waschschritt 1
- Streptavidin-Avidin Verstärkung: 1 h bei Raumtemperatur mit 1:3000 Verdünnung von "Streptavidin biotinylated horseradish peroxidase complex RPN 1051" (Amersham, Buckinghamshire, England) in PBS-T mit 0.26ml/$cm^2$ Membran (Einschweißen in Kunststoffolie, hochfrequente Agitation mit Rotationsmixer)
- Waschschritt 3: wie Waschschritt 1
- ECL-Entwicklung: 5 min. bei Raumtemp. mit 0.1 ml/$cm^2$ ECLPlus™ Lösung (RPN 2132; Amersham, Buckinghamshire, England) gemäß Angaben des Herstellers. Anschließend Entfernung von überschüssigem Reagenz (5 sec. zwischen 2 Folien Filterpapier) und Einschlagen der feuchten Membran in Frischhaltefolie.

*3.6.2.2 Immunoblot-2 (Roti-Block)*

Die für den Immunoblot-2 verwendeten Puffer, Lösungen und Antikörper sind in Tabelle 9a & b zusammengefaßt.

- Blockschritt: 1 h bei Raumtemperatur in 25ml 1:10 Roti-Block/$H_2O_{dd}$
- Inkubation mit primärem mAb: 15 h bei 4° C und abschließend 30 min. bei Raumtemp. in einer 1:4000 Verdünnung des mAb 1E8 (Schering AG, Berlin, Deutschland). Der mAb 6E10 ist bedingt durch ein hohes Hintergrundsignal nicht kompatibel mit Roti-Block.
- Waschschritt 1: 3x 10 min. mit PBS-T (4 ml/$cm^2$) bei Raumtemp.
- Inkubation mit sekundärem Ab: 1 h bei Raumtemp. mit einer 1:3000 Verdünnung des sekundären mAb (biotinylierter anti-Maus IgG, Pferd, H+L; Vector Laboratories, Burlingame, CA, USA) in 0.074 ml PBS-T/$cm^2$ Membran (Einschweißen in Kunststoffolie, hochfrequente Agitation mit Rotationsmixer)
- Waschschritt 2: wie Waschschritt 1
- Streptavidin-Avidin Verstärkung: 1 h bei Raumtemperatur mit 1:3000 Verdünnung von "Streptavidin biotinylated horseradish peroxidase complex RPN 1051" (Amersham, Buckinghamshire, England) in PBS-T mit 0.26ml/$cm^2$ Membran (Einschweißen in Kunststoffolie, hochfrequente Agitation mit Rotationsmixer)
- Waschschritt 3: wie Waschschritt 1
- ECL-Entwicklung: 5 min. bei Raumtemp. mit 0.1 ml/$cm^2$ ECLPlus™ Lösung (RPN 2132; Amersham, Buckinghamshire, England) gemäß Angaben des Herstellers. Anschließend Entfernung von überschüssigem Reagenz (5 sec. zwischen 2 Folien Filterpapier) und Einschlagen der feuchten Membran in Frischhaltefolie.

**3.6.3 Quantifizierung des ECL-Signals mittels densitometrischer Filmauswertung**

*3.6.3.1 Material und Reagentien*

Amersham Pharmacia Biotech AB (Buckinghamshire, England): $ECL^{Plus}$ Western Blotting Detection System (Best.-Nr. RPN 2132), Hyperfilm™ ECL™ (Best.-Nr. RPN 2103H);
Schleicher und Schuell (Dassel, Deutschland): Gel-Blotting Papier (Best.-Nr. 426690); Tropix (Bedford, MA, USA): Development Folders, 14 cm x 19 cm (Best.-Nr. XF030); Biometra (Göttingen, Deutschland): BioDoc software
Epson Deutschland GmbH (Düsseldorf, Deutschland): Laserscanner Epson GT 9000

*3.6.3.2 ECL-Entwicklung*

Nach dem letzten Waschschritt in PBS-T wird die PVDF-Membran auf eine Teflonunterlage gelegt und überschüssiger Waschpuffer durch Auflegen einer Lage KIMWIPES® Lite 200 Laborwischtücher entfernt. Es folgt eine Inkubation für 5 min. bei Raumtemperatur mit 0. 1 ml/$cm^2$ ECLPlus™ Lösung. Um überschüssige ECLPlus™ Lösung zu entfernen, wird die Membran zwischen zwei Lagen Gel-Blotting Papier gelegt und zur Signaldetektion in einen "Development Folder" überführt, der eine optimale Detektion gewährleistet und das Austrocknen der Membran verhindert.

*3.6.3.3 Quantfizierung*

Aufgetragen wurden jeweils 8 µl Liquorprobe. Jedes Gel führte eine Verdünnungsreihe eines Gemisches synthetischer Aβ-Peptide 1-40 und 1-42 ($A\beta_{1-42}$: 5,10,15,25 pg; $A\beta_{1-40}$: 20, 50, 75, 100 pg). Die Messungen wurden als Dreifachbestimmung durchgeführt. Die Berechnung der Aβ-Peptid Konzentrationen erfolgte für jedes Gel anhand seiner Eichreihe. Anschließend wurde der Mittelwert (n=3) und Variationskoeffizient (VK) berechnet. Der Intraassay-Variationskoeffizient wurde anhand der drei Einfachbestimmungen berechnet, die auf jeweils getrennten Gelen im gleichen Experiment unter Verwendung identischer Stammlösungen bestimmt wurden. Der Interaasasy-Variationskoeffizient wurde anhand der $A\beta_{1-42}$ Mittelwerte bestimmt, die in unabhängigen Experimenten (d.h. Untersuchungstagen) ermittelt wurden.

Mittels Dreifachbestimmung ermittelte Ausreißer wurden bei Berechnung beider Variationskoeffizienten nicht eliminiert, d.h. alle technisch auswertbaren Aβ-Peptidbanden gingen in die Berechnung ein.

Zusätzlich wurden für $A\beta_{1-40}$, $A\beta_{1-42}$ und $A\beta_{1-38}$ die Rohwerte (Flächeneinheiten) der drei Banden pro Spur erfasst und als Quotienten aufeinander bezogen ($A\beta_{1-42}/A\beta_{1-40}$, $A\beta_{1-42}/A\beta_{1-38}$).

Die ECL-Detektion nach Western-Immunoblot (primärer mAb: 1E8) erfolgte mittels Belichtung von Hyperfilm™ über 5 min. Die densitometrische Auswertung erfolgte mittels Laserscanners (Epson GT 9000) und Auswertesoftware (Fa. Biometra, BioDoc software).

**3.6.4 Quantifizierung des ECL-Signals mittels CCD-Kamera**

*3.6.4.1 Materialien und Geräte*

Bio-RAD Laboratories (Hercules, CA, USA): Fluor-S MAX MultiImager System (Best.-Nr. 170-7720); Quantity One Software (Best.-Nr. 170-8601)

*3. 6.4.2 ECL-Entwicklung*

Die ECL-Entwicklung wurde wie unter 3.6.3.2 beschrieben durchgeführt.

*3. 6.4.3 Durchführung*

Aufgetragen wurden jeweils 10 µl Liquorprobe. Jedes Gel führte eine Verdünnungsreihe eines Gemisches der synthetischen Aβ-Peptide 1-37, 1-38, 1-39, 1-40 und 1-42 ($A\beta_{1-37}$: 5, 10, 20, 40, 80 pg; $A\beta_{1-38}$: 15, 30, 60, 90, 120 pg; $A\beta_{1-39}$: 5, 10, 20, 30, 60 pg; $A\beta_{1-40}$: 25, 50, 100, 200, 300 pg; $A\beta_{1-42}$: 5, 10, 20, 40, 80 pg). Die ECL-Detektion mittels CCD-Kamera erfolgte bei einer Auflösung von 80x80µm mittels serieller Expositionszeiten für 5, 20, 60 und 120 Sekunden. Für die Quantifizierung der Gele relativ zu ihrer jeweiligen Eichreihe wurde die Auswertesoftware "Quantity One" (Fa. Bio-RAD Laboratories, Hercules, CA, USA) eingesetzt.

Die Messungen wurden als Vierfachbestimmung durchgeführt. Die Berechnung der Aβ-Peptid Konzentrationen erfolgte für jedes Gel anhand seiner Eichreihe. Anschließend wurde der Mittelwert (n=4) und Variationskoeffizient (VK) berechnet. Der Intraassay-Variationskoeffizient wurde anhand der vier Einfachbestimmungen berechnet, die auf jeweils getrennten Gelen im gleichen Experiment unter Verwendung identischer Stammlösungen bestimmt wurden. Der Interaasasy-Variationskoeffizient wurde anhand der Mittelwerte bestimmt, die in unabhängigen Experimenten (d. h. Untersuchungstagen) ermittelt wurden. Mittels Vierfachbestimmung ermittelte Ausreißer wurden bei Berechnung beider Variationskoeffizienten nicht eliminiert, d.h. alle technisch auswertbaren Aβ-Peptidbanden gingen in die Berechnung ein.

**3.7 Telenzephale Primärkultur des Hühnchens**

Eier der Hühnerrasse White Leghorn werden bei 37°C im Brutschrank über 10 Tage bebrütet. Am 10. Tag wird der Hühnerembryo unter sterilen Bedingungen entnommen und das Gehirn freipräpariert. Die vorderen Hirnbläschen werden abgetrennt, von den anliegenden Meningen befreit und in HEPES-gepuffertem DMEM aufgefangen. Das so gewonnene Gewebe wird über 15 Minuten einem Trypsinverdau unterzogen und nach dreimaligem Waschen mit DMEM mehrfach durch eine Kanüle aufgezogen. Nach Rünfminütiger Zentrifugation des Homogenats bei 550g wird der Überstand dekantiert, das Pellet in Kultivationsmedium (DMEM +5% Fetales Kälberserum + 5% Hühnerserum) aufgenommen und erneut durch eine Kanüle aufgezogen. Im Anschluß an eine Zellzahlbestimmung mittels Neubauer-Zählkammer wird die Zelldichte der Suspension auf 1.5 Mio. Zellen/ml eingestellt und diese in die Kultivationsgefäße ausplattiert, so dass sich eine Zelldichte von 375.000 Zellen/$cm^2$ ergibt. Zur Verbesserung der Zellanheftung wurden die Kultivationsgefäße zuvor über 24 Stunden mit einer Poly-L-Lösung (0,1mg/ml Poly-L-Lysin in 0,1M Borat/NaOH-Puffer, sterilfiltriert, pH 8,4) beschichtet. Am 2. Kultivationstag erfolgt ein 50%iger Mediumwechsel, am 5. Kultivationstag ein 100%iger Medienwechsel unter gleichzeitiger Zugabe der zu untersuchenden Testsubstanz. Die Inkubationszeiten können bis zu 48 Stunden betragen.

**3.8 Gewinnung von Liquor**

Drei bis 10 ml lumbaler Liquor wurde mittels lumbaler Liquorpunktion gewonnen und in Polypropylen Probengefäßen aufgefangen. Nach Zentrifugation (1000g, 10 min, 4° C) wurden die Proben innerhalb von 24 Stunden in Aliquots von 150 µl bis zur Bestimmung bei -80°C in Polypropylengefäßen (Eppendorf, 1.5 ml) gelagert. Die Proben dürfen nicht mehrfach eingefroren und aufgetaut werden.

### 3.9 Patienten

Insgesamt wurde der lumbale Liquor bei 130 Patienten untersucht. Bei fünf dieser Patienten wurden Aβ-Peptide zusätzlich im Blutplasma gemessen. Die Patienten verteilten sich auf die beiden diagnostischen Obergrruppen Neuropsychiatriscche Erkrankungen ausschließlich Alzheimer-Demenz ("neuropsychiatric disesase controls", NDC) und Patienten mit klinisch wahrscheinlicher (sporadischer) Alzheimer-Demenz (AD). Methodisch bedingt wurden mehrere NDC- und AD-Gruppen mit jeweils unterschiedlichen Patienten untersucht, wobei zusammengehörige Patientenkollektive durch eine fortlaufende arabische Nummerierung gekennzeichnet sind (z.B. NDC-1, AD-1). Die Kollektive NDC-1 und NDC-2 enthalten auch Patienten mit dementiellen Erkrankungen anderer Genese als AD. Das Kollektiv NDC-3 enthält nur Patienten mit nicht-dementiellen neuropsychiatrischen Erkrankungen. Dieses Kollektiv wurde differenziert in Patienten mit chronisch entzündlichen Erkrankungen des ZNS ("chronic inflammatory CNS disease", CID-3) und den verbleibenden Patienten mit anderen neuropsychiatrischen Erkrankungen ("other neruopsychiatric diseases", OND-3). Innerhalb der OND-3 und der AD-3 Gruppe wurde weiter in Abhängigkeit des ApoE ε4-Genotypus differenziert. Fig. 1 gibt einen Überblick über die Patientengruppen und ihre hierarchische Zuordnung. Tabelle 10a-d nennt die Patienten, die sich gleichzeitig in mehreren Patientengruppen befinden.

Die klinische Diagnostik erfolgte gemäß ICD-10. Die Diagnose einer Alzheimer Demenz wurde gemäß den international überwiegend verwendeten Kriterien der "Work Group of the National Institute of Neurological and Communicative Disorders and Stroke (NINCDS)" und der Richtlinien des "Alzheimer's Disease and Related Disorders Association (ARDA)" vorgenommen (McKhann et al., 1984). Die Proben wurden ausschließlich innerhalb der klinischen Routinediagnostik gewonnen. Für die hier vorgestellten Messungen wurde kein zusätzliches Liquorvolumen gewonnen. Entsprechend konnte die retrospektive Untersuchung nur erfolgen, wenn nach abgeschlossener Routinediagnostik noch aliquotierter Liquor zur Verfügung stand.

#### 3.9.1 NDC-1 und AD-1

Aβ-Pepitde wurden im lumbalen Liquor bei 65 Patienten mittels Aβ-SDS-PAGE/Immunoblot-1 und densitometrischer Filmauswertung quantifiziert. Die Probenaufnahme der zuvor nach Liquorpunktion und Zentrifugation nativ eingefrorenen Proben folgte gemäß 3.2.2a. Die Diagnosen und Meßwerte der Patienten sind in Tabelle 12 dargestellt und in Tabelle 13 zusammengefaßt. Patienten die gleichzeitig in anderen Kollektiven vertreten sind finden sich in Tabelle 10a und 10c.

- NDC-1: n = 30, Alter = 59.2 ± 12.6 (MW ± SD), Geschlecht: 19/11 (weiblich/männlich).
- AD-1: n = 35, Alter = 69.7 ± 8.8 (MW ± SD), Geschlecht: 18/17 (weiblich/männlich). Zehn der AD-1 und 20 der NDC-1 Patienten wurden vergleichend mittels Immunopräzipitation (mAb 6E10, IP ohne Detergentien ) und Aβ-SDS-PAGE/ Immunoblot-1 untersucht (vergl. Tabelle 12 und 13). Alle Patienten des Kollektivs AD-1 wurden vergleichend mit einem kommerziellem ELISAA$\beta_{1-42}$ untersucht (vergl. Tabelle 13).

#### 3.9.2 NDC-$2^{KP}$

Bei zehn Patienten wurde die Konzentration von A$\beta_{1-40}$ und A$\beta_{1-42}$ im lumbalen Liquor in Abhängigkeit der Probenvorbehandlung mittels Aβ-SDS-PAGE/ Immunoblot-1 und densitometrischer Filmauswertung untersucht. Dabei wurde das Ausmaß der kältepräzipitationsbedingten Erniedrigung (KP) von Aβ-Peptiden untersucht. Die Proben der Patienten wurden nach Liquorpunktion und Zentrifugation aliquotiert. Ein Aliquot wurde vor dem Einfrieren mit SDS-/ Hitzedenaturierung gemäß 3.2.2a vorbehandelt, im folgenden A$\beta_{1-40}$SDS oder A$\beta_{1-42}$SDS genannt. Das andere Aliquot wurde ohne Vorbehandlung bei -80° C eingefroren, im folgenden A$\beta_{1-40}$nativ oder A$\beta_{1-42}$nativ genannt. Die Diagnosen und Meßwerte der Patienten sind in Tabelle 16a und 16b zusammengefaßt. Patienten die gleichzeitig in anderen Kollektiven vertreten sind finden sich in Tabelle 10a.

- NDC-$2^{KP}$: n = 10, Alter = 45.8 ± 13.4 (MW ± SD), Geschlecht: 6/4 (weiblich/männlich)

#### 3.9.3 NDC-3 und AD-3 mit Untergruppen

Bei 49 Patienten des Kollektivs NDC-3 und 12 Patienten des Kollektivs AD-3 wurde die Konzentration der Aβ-Peptide im lumbalen Liquor mittels Aβ-SDS-PAGE/Immunoblot-2 und CCD-Kamera untersucht. Die Probenaufnahme der nach Liquorpunktion und Zentrifugation nativ eingefrorenen Proben folgte gemäß 3.2.2a. Die Diagnosen und Meßwerte der Patienten sind in Tabelle 19 dargestellt und in Tabelle 20 zusammengefaßt. Patienten die gleichzeitig in anderen Kollektiven vertreten sind finden sich in Tabelle 10b, c und d.

- NDC-3: n = 47, Alter = 45.2 ± 15.8 (MW ± SD), Geschlecht: 19/28 (weiblich/männlich).
- AD-3: n = 12, Alter = 73.0 ± 7.9 (MW ± SD), Geschlecht: 9/3 (weiblich/männlich).

Innerhalb des Kollektivs NDC-3 wurden in Abhängigkeit von Art der Probe und Probenvorbehandlung weitere Untergruppen gebildet: Gepaarte Liquor- und EDTA Plasmaproben wurden bei fünf der NDC-3 Patienen gewonnen. Diese Proben wurden mittels Immunopräzipitation (RIPA-IP, 1E8) untersucht und werden nachfolgend IP-CSF-3 und IP-Plasma-3 genannt. Vergleichend wurden die Konzentrationen der ohne vorherige Immunopräzipitation gemessenen Aβ-Peptide für die letztgenannten fünf Patienten als das Kollektiv SDS-CSF-3 zusammengefaßt (vergl. Tabelle 20). Bei 27 der NDC-3 Patienten wurde die Konzentration von $A\beta_{1-42}$ im Liquor vergleichend mit einem kommerziellem ELISA (Hulstaert et al., 1999) bestimmt. Innerhalb des Kollektivs NDC-3 wurde zwischen Patienten mit chronisch entzündlichen ZNS-Erkrankungen ("chronic inflammatory CNS disease", CID) und Patienten mit anderen neuropsychiatrischen Erkrankungen ("other neuropsychiatric disease", OND) differenziert.

- OND-3: n=37, Alter = 45.3 ± 16.4 (MW ± SD), Geschlecht: 15/22 (weiblich/männlich).
- CID-3: n=10, Alter = 44.9 ± 14.2 (MW ± SD), Geschlecht: 4/6 (weiblich/männlich).

Das CID-3 Kollektiv setzte sich aus fünf Patienten mit Multipler Sklerose und fünf Patienten mit unklarer Ätiologie des chronisch entzündlichen ZNS-Prozesses zusammen.

Das Kollektiv OND-3 wird in Abhängigkeit des ApoE ε4 Genotypus weiter in die Gruppen OND-3$\varepsilon4^{plus}$ (n=6) und OND-3$\varepsilon4^{minus}$ (n=30) differenziert. Patienten der Gruppe OND-3$\varepsilon4^{plus}$ haben ein oder zwei Allele ε4, Patienten der Gruppe OND-3$\varepsilon4^{minus}$ fehlt dieses Allel. Da 11/12 AD-3 Patienten ein oder zwei ApoE ε4 Allele tragen, kann der Einfluß des ε4 Allels auf das Liquormuster der Aβ-Peptide nicht eliminiert werden, aber ε4 unabhängige und daher mehr AD-spezifische Effekte wurden durch den Vergleich der Patientengruppen AD-3$\varepsilon4^{plus}$ (n=11) und OND-3$\varepsilon4^{plus}$ (n=6) ermittelt.

Die Werte zu den MMSE Testergebnissen der Patienten, Häufigkeiten der ApoE ε4 Allele, sowie absolute and relative Aβ-Peptid Liquorkonzentrationen sind für die Gruppen NCD-3, AD-3, IP-plasma-3, IP-CSF-3, and SDS-CSF-3 in Tabelle 20 zusammengefaßt.

### 3.9.4 NDC-$3^{KP}$ und AD-$3^{KP}$

Bei 15 Patienten der Gruppe NDC-3 und 9 Patienten der Gruppe AD-3 wurde die kältepräzipitationsbedingte Erniedrigung (KP) von $A\beta_{1-42}$ im lumbalen Liquor vergleichend mittels Aβ-SDS-PAGE/Immunoblot-2 und CCD-Kamera untersucht. Die Kollektive werden im folgenden NDC-$3^{KP}$ und AD-$3^{KP}$ genannt. NDC-$3^{KP}$ ist vollständig Teilkollektiv von NDC-3. AD-$3^{KP}$ (n=11) enthält neben neun Patienten von Kollektiv AD-3 zusätzlich zwei weitere Patienten (NP69, NP197). Die Probenvorbereitung für die Bestimmung von $A\beta_{1-42}$nativ und $A\beta_{1-42}$SDS erfolgte wie für das Kollektiv NDC-$2^{KP}$ beschrieben.

Die Diagnosen und Meßwerte der Patienten sind in Tabelle 21 zusammengefaßt. Patienten die gleichzeitig in anderen Kollektiven vertreten sind finden sich in Tabelle 10b, c und d.

- NDC-$3^{KP}$: n=15, Alter = 44.6 ± 15.0 (MW±SD), Geschlecht: 5/10 (weiblich/männlich).
- AD-$3^{KP}$: n = 11, Alter = 70.9 ± 9.0 (MW±SD), Geschlecht: 9/2 (weiblich/männlich).

### 3.10 Statistik

Die Testung auf signifikante Differenzen zwischen unabhängigen Stichproben erfolgte über den Mann-Whitney U-Test und für abhängige (gepaarte) Stichproben über den Wilcoxon-Test. Die nicht-parametrische Regressionsanalyse erfolgte nach Spearman (Korrelationskoeffizient rho oder R). Als Statistiksoftware wurde Statistika (Version 5.0) eingesetzt. Die iterative Berechnung von diagnostischer Spezifität und Sensitivität für die Diagnosestellung AD in Abhängigkeit unterschiedlicher Aβ-Peptidgrenzwerte wurde über eine "receiver operating characteristic (ROC) curve" vorgenommen (Metz, 1978). Das zweiseitige Signifikanzniveau wurde auf $p<0.05$ festgelegt.

## 4 Ergebnisse

### 4.1 Auftrennung und Nachweis von sAPPa und Aβ-Peptiden

#### 4.1.1 Aβ-SDS-PAGE

Mittels Aβ-SDS-PAGE lassen sich folgende synthetische Aβ-Peptide durch harnstoffinduzierte Konformationsänderung von kathodisch (oben) nach anodisch (unten) trennen:

1-33 / 1-34
1-35
1-37
1-38
1-39
1-42 / 2-40 / 3-40
2-42 / 3-42
3p-42* / 3p-40*
* p = Pyroglutamatderivate;

Aβ-Peptide mit fehlender oder nur partieller Trennung stehen in einer Zeile. Der Nachweis erfolgte mittels Silberfärbung der Trenngele.

Mittels Aβ-IPG-2D-PAGE können die Aß-Peptide 2-40 / 3-40 von 1-42 getrennt werden, da das Fehlen von Aspartat den isoelektrischen Punkt um eine pH-Einheit von 5.37 nach 6.37 verschiebt (vergl. Fig. 2a&c und Fig. 24a).

Der gleiche pH-Sprung zeigt sich für die Aß-Peptide 2-42 / 3-42 in Bezug auf 1-42. Zwischen 2-40 /2-42 und 3-40 /3-42 kann über die N-terminal selektiven mAbs 1E8 und 6E10 differenziert werden (vergl. 3.1.2). Bemerkenswert ist, dass N- und C-terminale Veränderungen der Aβ-Peptide, die zu einem erhöhtem Aggregationsverhalten führen (N-terminal: Fehlen von Aspartat und Pyroglutamatbildung; C-terminal: Verlängerung um hydrophobe Aminosäuren) auch zu einer erhöhten elektrophoretischen Mobilität im harnstoffhaltigen Trenngelsystem führen. Damit besteht eine Analogie zwischen den Struktur-Aktivitätsfunktionen *in vitro* und *in vivo*.

Vergleichweise geringfügige Veränderungen der Trenngelmatrix (Polyacrylamid Porengröße, Molarität des Harnstoffs, pH-Wert, Temperatur und Ionenstärke) und der kathodischen SDS-Konzentration verändern signifikant das absolute und relative Laufverhalten der Aβ-Peptide. Veränderungen der Gesamtkonzentration an Acrylamidmonomer (T%) oder des Anteils an Bisacrylamid an der Gesamtkonzentration (%C) um nur 1-2% bei sonst konstanten Bedingungen sind dafür ausreichend. Gleichfalls führt die selektive Reduktion der Harnstoffkonzentration von 8 auf 7 Mol/L oder die Reduktion der kathodischen SDS-Konzentration von 0.25% (w/v) auf 0.1% zu einer veränderten Auftrennung.

Mittels Aβ-SDS-PAGE wird im oberen (kathodischen) Kompartment im harnstoffhaltigem Trenngel sAPPα aufgetrennt, dass mittels Western-Immunoblot-1/2 (mAb 1E8) nachgewiesen werden kann (vergl. Fig. 3). Bedingt durch die Molmasse von > 100000 werden sAPPα Isoformen im Vergleich zu den Aβ-Peptiden weniger effektiv und mit wesentlich höherer Varianz aus den kleinporigen harnstoffhaltigen Trenngelen auf die Nachweismembran geblottet (Intraassay-Variationskoeffizient im Liquor > 20%).

Blotteffizienz und Auftrennung der sAPPα Isoformen können aber wesentlich verbessert werden, wenn das harnstoffhaltige Trenngelsytem mit einem kathodischen nichtharnstoffhaltigem Trenngelkompartment höherer Porengröße aber sonst gleicher Pufferszusammensetzung kombiniert wird (10 T%, 5 C%, kein Harnstoff). Bei unveränderter Trenngellänge des harnstoffhaltigen Kompartiments wird die Qualität der Aβ-Peptidauftrennung nicht beeinträchtigt, da die Aβ-Peptide das großporige Kompartiment noch konzentriert innerhalb der "moving boundary" durchlaufen.

Die Quantifizierung von sAPPα mittels Aβ-SDS-PAGE/Immunoblot-2 und CCDKamera erscheint für die neurochemische Demenzdiagnostik vielversprechend, da sAPPα bei AD im Liquor erniedrigt gefunden wurde und den α-Sekretase Schnitt abbildet. Damit erlaubt der Aβ-SDS-PAGE/Immunoblot die Berechnung von sAPPα/Aβ-Peptid Quotienten, die ein Maß für das Verhältnis von α-Sekretase zu β-/γ-Sekretaseaktivität darstellen.

### 4.1.2 Western-Immunoblot-1/2

Der mAb 1E8 zeigt im Western-Immunoblot-1/2 eine erstaunlich hohe N-terminale Spezifität, da im unteren pg-Bereich (<200 pg) nur Aβ-Petide nachweisbar sind, die N-terminal maximal um eine Aminosäure (Aspartat) verkürzt sind. Entsprechend werden die Aβ-Peptide 3-40, 3p-40, 3-42 und 3p-42 mittels des mAb 1E8 nicht nachgewiesen. Hier gelingt der Nachweis mittels des N-terminal spezifischen mAb 6E10, der kommerziell erhältlich ist, aber in Abhängigkeit der Blotbedingungen etwa zehn- bis dreißigfach weniger empfindlich detektiert.

Die Nachweisempfindlichkeit des Western-Immunoblot-1 (Milchpulver-Block, mAb 1E8) liegt bei 1pg ($A\beta_{1-40}$) bis 2 pg ($A\beta_{1-42}$) bei Filmexposition und bei 3 pg ($A\beta_{1-40}$) bis 6 pg ($A\beta_{1-42}$) bei Signalaufnahme mit der CCD-Kamera. Die Nachweisempfindlichkeit des Western-Immunoblot-2 (Roti-Block, mAb 1E8) liegt bei 0.3 pg ($A\beta_{1-40}$) bis 0.6 pg ($A\beta_{1-42}$) bei Filmexposition und bei 1 pg ($A\beta_{1-40}$) bis 2 pg ($A\beta_{1-42}$) bei Signalaufnahme mit der CCD-Kamera. Durch Entwicklung des Western-Immunoblot-2 konnte die im Vergleich zur Filmexposition etwa dreifach geringere Empfindlichkeit der CCD-Kamera kompensiert werden. Die Nachweisempfindlichkeit des kommerziell erhältlichen N-terminal selektiven mAb 6E10 liegt bei Western-Immunoblot mit Milchpulver-Block bei 10 pg ($A\beta_{1-40}$) bis 20 pg ($A\beta_{1-42}$) bei Filmexposition und bei 30 pg ($A\beta_{1-40}$) bis 60 pg ($A\beta_{1-42}$) bei Signalaufnahme mit der CCD-Kamera. Der mAb 6E10 kann nicht mit Rotiblock verwendet werden. Damit konnte die Nachweisempfindlichkeit gegenüber dem mAb 6E10 um bis zu 30-fach gesteigert werden.

SDS-PAGE Trenngelsysteme mit 8 M Harnstoff können unabhängig von den verwendeten Geldimensionen mit maximal etwa 5 µl Liquor pro $mm^2$ Geloberfläche beladen werden, wenn bei nahezu allen Patientenproben eine optimale elektrophoretische Trennung realisiert werden soll. Dabei handelt es sich um nativ eingefrorenen und anschließend SDS-/Hitzedenaturierten Liquor oder um Liquor, der vor dem Einfrieren SDS-/Hitzedenaturiert wurde. 5 µl pro $mm^2$ entsprechen bei dem verwendeten Minigelsystem einem Liquorvolumen von etwa 10 µl. Damit ergibt sich Empfindlichkeit von 200 pg/ml für den Nachweis von $A\beta_{1-42}$ im humanen Liquor mittels Aβ-SDS-PAGE/Immunoblot-2 und CCD-Kamera. Eine Nachweisempfindlichkeit von mindestens 200 pg/ml ist Voraussetzung für die neurochemische Demenzdiagnostik der AD mittels Bestimmung der Aβ-Peptide im Liquor und kann beispielsweise mit dem kommerziell erhältlichen mAb 6E10 nicht realisiert werden.

Bei Kombination von Immunopräzipitation (RIPA-Detergentien, mAb 1E8) mit Aβ-SDS-PAGE/Immunoblot-2 und CCD-Kamera steigt die Empfindlichkeit für den Nachweis von $A\beta_{1-42}$ auf < 10 pg/ml. Dies ist Voraussetzung für die Quantifizierung von Aβ-Peptiden im Plasma mittels Aβ-SDS-PAGE/Immunoblot. Die Intra- und Interassay-Variationskoeffizienten für den Aβ-SDS-PAGE/ Immunoblot-1 mit densitometrischer Filmauswertung finden sich in Tabelle 11. Die entsprechenden Variationskoeffizienten für den Aβ-SDS-PAGE/Immunoblot-2 mit CCD-Kamera finden sich in Tabelle 18a und b. Für die Quantifizierung von 20 pg Aβ-Peptid wurden Intra- und Interassayvariationskoeffizienten von jeweils weniger als 10% ermittelt.

Die Quantifizierung mittels CCD-Kamera bietet erhebliche Vorteile gegenüber der Filmexposition. Das Lichtsignal kann hier über 3.8 Zehnerpotenzen linear aufgenommen werden und zusätzlich kann die Zeitdauer der Signalaufnahme über einen weiten Bereich exakt gesteuert werden. Entsprechend können APP-Metabolite mit großer Differenz ihrer Signalintensität, wie z.B. sAPPα und $A\beta_{1-42}$, über zwei Messzeiten (z.B. 10 s und 3 min) quantifiziert werden.

### 4.2 Patientenproben

Bisher war bekannt, dass $A\beta_{1-40}$ und $A\beta_{1-42}$ regelhaft und in höherer Konzentration im humanem Liquor vorkommen. Sowohl bei Direktauftrag nach SDS-/Hitzedenaturierung als auch nach vorheriger Immunopräzipitation unter Verwendung N-terminal selektiver Antikörper kann dagegen mittels Aβ-SDS-PAGE/ Immunoblot-1/2 regelhaft ein charakteristisches Aβ-Peptidquintett im humanen lumbalen Liquor nachgewiesen werden (Fig. 3). Oberhalb (kathodenseitig) von $A\beta_{1-40}$ kommen drei weitere Aβ-Peptide zur Darstellung, die initial als $A\beta1\text{-}x^a$, $A\beta1\text{-}x^b$ und $A\beta1\text{-}x^c$ bezeichnet wurden und mittels Aβ-IPG-2D-PAGE/ Immunoblot mit Komigration synthetischer Aβ-Peptide als $A\beta_{1-37/38/39}$ identifiziert werden konnten (Fig. 2). Die Aβ-Peptide 1-37, 1-38 und 1-39 sind bei carboxyterminal selektiver Immunopräzipitation gegen 1-40 und 1-42 im Liquor mittels Aβ-SDS-PAGE/Immunoblot nicht nachweisbar. Neben den Aβ-Peptiden 1-33, 1-34 und 1-35 konnten die Aβ-Peptide 1-37/38/39 im humanem Liquor mittels MALDI-TOF nachgewiesen werden. Die Aβ-Peptide 1-33/1-34 und 1-35 sind im Aβ-SDS-PAGE/Immunoblot oberhalb (kathodenseitig) von 1-37 bei den Patienten im Liquor in der Regel nur an der Nachweisgrenze detektierbar oder nicht nachweisbar. Die Aβ-Peptide 1-37, 1-38, 1-39, 1-40 und 1-42 sind hochgradig und signifikant korreliert, wie aus Fig. 10 entnommen werden kann. Dies spricht für eine enge enzymatische Regulation ihrer Entstehung. Die synthetischen Aβ-Peptide 1-37, 1-38 und 1-39 standen bei Untersuchung der ersten Patientenkollektive (NCD-1, AD-1, $NDC\text{-}2^{KP}$) noch nicht zur Verfügung. Daher wurde hier das Verhältnis $A\beta_{1-42}$ zu $A\beta_{1-38}$ über den Quotienten der densitometrisch ermittelten Flächeneinheiten der jeweiligen Banden einer Gelspur ermittelt. Zum Vergleich wurde auch der Quotient $A\beta_{1-42}/A\beta_{1-40}$ über die Flächeneinheiten ausgedrückt. Da $A\beta_{1-38}$, $A\beta_{1-40}$ und $A\beta_{1-42}$ bei gleicher Konzentration und gleichen Bedingungen im Western-Immunoblot eine unterschiedliche Intensität des ECL-Signals zeigen, können die Quotienten der Flächeneinheiten nicht mit den entsprechenden Quotienten der über die Eichgrade ermittelten Aβ-Peptidkonzentrationen gleichgesetzt werden.

Bei einem Teil der Patienten mit AD wird unterhalb (anodisch) von $A\beta_{1-42}$ mittels Aβ-SDS-PAGE/Immunoblot-2 und CCD-Kamera eine zusätzliche Bande mit dem Retentionsfaktor (Rf) von $A\beta_{2-42}$ nachweisbar (Fig. 23a). Diese Bande konnte im Liquor bei AD nach vorheriger Immunopräzipitation (RIPA-IP, mAb 1E8) mittels Aβ-IPG-2D-PAGE/ Immunoblot-2 und CCD-Kamera als $A\beta_{2-42}$ identifiziert werden (Fig. 24 b). Bei nicht-dementen Kontrollpatienten konnte $A\beta_{2-42}$ bisher nicht nachwiesen werden. $A\beta_{2-42}$ ist auch mit typischer hirnregionaler Verteilung bei Patienten mit sporadischer AD intrazerebral massiv erhöht (vergl. 4.2.5). Für die Bestimmung von Aβ-Peptiden im Liquor bei den unter 3.9 genannten Patientenkollektiven stand noch kein synthetisches $A\beta_{2-42}$ zur Verfügung. Entsprechend liegen für diese Patienten keine quantitativen Daten zu $A\beta_{2-42}$ vor. Zwischenzeitlich wurde aber ein weiteres Patientenkollektiv Patienten gemessen, das unter 3.9 noch nicht aufgeführt ist. Bei einigen der Patienten mit AD-4, bei einigen Patienten mit anderen dementiellen Erkrankungen als AD (nAD-4) und bei einigen Patienten mit nicht-dementiellen Erkrankungen (NDC-4) war $A\beta_{2-42}$ nachweisbar. Zusätzlich war der Quotient $A\beta_{1-42}/A\beta_{1-40}$ bei den $A\beta_{2-42}$ positiven Patienten im Vergleich zu den übrigen Patienten signifikant erniedrigt.

### 4.2.1 NDC-1 und AD-1

Tabelle 12 gibt die klinischen Daten und individuellen Meßwerte der Patienten und Tabelle 13 faßt die statistischen Kennwerte der Patientenkollektive AD-1 und NDC-1 zusammen. Die Analyse der Liquorproben erfolgte mittels Aβ-SDS-PAGE/Immunoblot-1 und densitometrischer Filmauswertung.

Die signifikante Erniedrigung von Aβ1-42 in humanem lumbalen Liquor bei Patienten mit AD-1 im Vergleich zu NDC-1 geht aus Fig. 4 hervor. $A\beta_{1-40}$ ist bei AD-1 gleichfalls signifikant erniedrigt, aber nicht in dem Ausmaß wie dies für $A\beta_{1-42}$ deutlich wird (vergl. Tabelle 14). Entsprechend ist auch der Quotient $A\beta_{1-42}/A\beta_{1-40}$ hochsignifikant erniedrigt (Fig. 5, Tabelle 14). Bisher nicht beschrieben wurde die gleichfalls hochsignifikante Erniedrigung des Quotienten $A\beta_{1-42}$/ $A\beta_{1-38}$ (Fig. 6, Tabelle 14). Die Grenzwerte für den Gruppenvergleich AD-3 versus NDC-3 wurden für $A\beta_{1-42}$ und die beiden letztgenannten Aβ-Peptidquotieten über die jeweiligen "receiver operating characteristics (ROC)" ermittelt (Tabelle 15). Die Differenzierung der Patientenkollektive AD-1 versus NDC-1 bei einem Grenzwert von 802.5 pg/ml $A\beta_{1-42}$ war mit einer Spezifität von 74% und einer Sensitivität von 87% durchführbar. Der Quotient $A\beta_{1-42}/A\beta_{1-40}$ hat eine diagnostische Spezifität von 71% und eine Sensitivität von 93% für die Differenzierung der Kollektive AD-1 versus NDC-1. Die entsprechende Spezifität und Sensitivität für den Quotienten $A\beta_{1-42}/A\beta_{1-38}$ beträgt 84% und 0.86%.

Vergleichend wurde $A\beta_{1-42}$ nach Immunopräzipitation (IP ohne Detergenz, mAb 6E10) und Aβ-SDS-PAGE/ Immunoblot-1 mit densitometrischer Filmauswertung untersucht. Wie aus Fig. 7 und Tabelle 14 hervorgeht, differenziert $A\beta_{1-42}$ nach vorheriger IP weniger gut die Patientenkollektive AD-1 und NDC-1. Die nach Immunopräzipitation und Aβ-SDS-PAGE/Immunoblot-1 mit densitometrischer Filmauswertung ermittelte Konzentration von $A\beta_{1-42}$ im Liquor bei einem Teilkollektiv der Patienten mit AD-1 stimmt gut mit der Konzentration überein, die mittels des kommerziellen ELISA$A\beta_{1-42}$ (Hulstaert et al., 1999) bei AD-1 ermittelt wurde (Tabelle 13). Gleichzeitig stimmt der ELISA Mittelwert bei AD-1 (412 pg/ml) gut mit dem ELISA Medianwerten bei AD (428 und 487 pg/ml) überein, die in einer internationalen Multizenterstudie ermittelt wurden (Hulstaert et al., 1999).

Der Vergleich der Konzentrationen von $A\beta_{1-42}$ im Liquor in Abhängigkeit der Meßmethodik (SDS-/Hitzedenaturierung mit Aβ-SDS-PAGE/Immunoblot-1 versus Immunopräzipitation mit Aβ-SDS-PAGE/Immunoblot-1 oder $ELISA_{A\beta 1-42}$) macht deutlich, dass mittels SDS/Hitzedenaturierung wesentlich mehr $A\beta_{1-42}$ aus dem Liquor extrahiert werden kann als über die Antikörper-abhängigen Verfahren (Immunopräzipitation und ELISA). Im Vergleich zur Immunopräzipitation (IP ohne Detergenz, mAb 6E10) werden nach SDS-/Hitzedenaturierung im Mittel 2.3-fach und im Vergleich zum ELISA (ohne Detergenz) 1.8-fach höhere Konzentrationen von $A\beta_{1-42}$ im Liquor bei AD gemessen. Weiter unten wird gezeigt, dass diese Differenz zwischen ELISA und Aβ-SDS-PAGE/Immunoblot bei NDC-Patienten noch höher ausfällt (vergl. NDC-3).

### 4.2.2 NDC-3 und AD-3

Tabelle 19 gibt die klinischen Daten und individuellen Meßwerte der Patienten und Tabelle 20 faßt die statistischen Kennwerte der Patientenkollektive AD-1 und NDC-1 zusammen. Die Analyse der Liquorproben erfolgte mittels Aβ-SDS-PAGE/Immunoblot-2 und CCD-Kamera.

#### *4.2.2.1 Abhängigkeit der Aβ-Peptid Konzentration im Liquor von der Art der Probenvorbereitung*

Bei fünf Patienten des NDC-3 Kollektivs wurden Aliquots von Liquor vergleichend mit vorheriger Immunopräzipitation (RIPA-IP, mAb 1E8) bzw. mit direkter Probenaufnahme (SDS-/Hitzedenaturierung) untersucht (vergl. Tabelle 20). Nach SDS-/Hitzedenaturierung ergeben sich etwas höhere Aβ-Peptid Konzentrationen. Dieser Effekt ist betont für $A\beta_{1-38}$ und $A\beta_{1-42}$, erreicht aber nicht das Signifikanzniveau. Entsprechend werden mit beiden Methoden der Probenvorbehandlung vergleichbare Aβ-Peptidspiegel im Liquor gemessen, wenn die Immunopräzipitation mit Detergentien durchgeführt wird. Im Gegensatz dazu wird bei 27 der NDC-3 Patienten mit dem kommerziellen ELISA$A\beta_{1-42}$ (ohne Detergenz) im Vergleich zu SDS-/Hitzedenaturierung und Aβ-SDS-PAGE/ Immunoblot-2 mit CCD-Kamera ein ca. 3-fach tieferer Spiegel für $A\beta_{1-42}$ gemessen.

Weiter oben wurde für Patienten des AD-1 Kollektivs nachgewiesen (vergl. 4.2.1), dass mit Immunopräzipitation (mAb 6E10) und Aβ-SDS-PAGE/ Immunoblot-1 deutlich tiefere und mit dem ELISA$A\beta_{1-42}$ vergleichbare Konzentrationen gemessen werden, wenn die Immunopräzipitation ohne Detergenz durchgeführt wird.

Damit ergibt sich der Hinweis, dass $A\beta_{1-42}$ im humanen Liquor in einer Fraktion vorliegt, die monoklonalen Antikörpern ohne vorherige Behandlung mit Detergentien nur zum Teil zugänglich ist.

Die eingesetzten Detergentien können Peptide aus nicht-kovalenten Protein-Peptidbindungen - beispielsweise bedingt durch hydrophobe Wechselwirkung - freisetzen. Entsprechend sind die höheren Liquorspiegel von $A\beta_{1-42}$ nach Einsatz von Detergentien (SDS-Hitzedenaturierung, RIPA-IP) im Vergleich zu Verfahren ohne Einsatz von Detergentien (IP ohne Detergenz, ELISA$A\beta_{1-42}$) wahrscheinlich durch hochaffine Bindung und Epitopmaskierung von $A\beta_{1-42}$ an andere Proteine oder Aβ-Peptidaggregate bedingt. Bei Verwendung von Detergentien ist erwartungsgemäß der Einsatz des ionischen Detergenz SDS bei höherer Konzentrationen (0.5% w/v) und Temperatur (95°C) noch effektiver als der RIPA-Detergentienmix.

Weiter unten wird nachgewiesen, dass $A\beta_{1-42}$ im Vergleich zu $A\beta_{1-40}$ besonders kältepräzipitationsempfindlich ist und krankheitsspezifisch ein unterchiedliches Kältepräzipitationsverhalten bei Patienten mit AD und NDC zeigt (vergl. 4.2.4).

Synthetisches $A\beta_{1-42}$ mit vergleichbarer Konzentration in Wasser gelöst zeigt dagegen eine deutlich geringere Kältepräzipitation. Damit wird wahrscheinlich, dass die kältepräzipitationsbedingte Reduktion von $A\beta_{1-42}$ im humanen Liquor überwiegend auf den Aggregat-gebundenen Anteil des Peptids entfällt. Im Fall vergleichsweise hydrophober Aggregate - beispielweise Lipoprotein-haltiger Komplexe - wäre ein Verlust durch Kältepräzipitation nicht überraschend. In diesem Zusammenhang wird weiter unten gezeigt (4.2.4), dass ε4-positive Patienten des NDC-3KP Kollektivs eine besonders hohe Rate von KP-bedingter Erniedrigung von $A\beta_{1-42}$ zeigen und annähernd gleich tiefe Liquorspiegel wie ε4-positive AD-3KP Patienten haben. Annähernd gleich tiefe $A\beta_{1-42}$ Liquorspiegel werden weiter unten auch für Patienten der Gruppen OND-3ε4plus und AD-3ε4plus nachgewiesen.

*4.2.2.2 Aβ-Peptide im Plasma*

Mittels Immunopräzipitation und Aβ-SDS-PAGE/Immunoblot mit CCD-Kamera konnte das Aβ-Peptidquintett auch im Plasma nachwiesen werden. Im Vergleich zum Liquor sind die Konzentrationen im Plasma 30 bis 60-fach geringer und beide Kompartimente zeigen jeweils spezifische Muster der prozentualen Aβ-Peptidanteile. Besonders das Verhältnis $A\beta_{1-42}/A\beta_{1-38}$ ist ZNS-spezifisch unterschiedlich: Liquor 0.80 (0.79-0.92), Plasma 1.70 (1.69-1.75); Median (Quartil).

*4.2.2.3 Krankheitsspezifische Aβ-Peptidmuster im Liquor und Einfluß des ApoE Genotyp*

Fig. 8 zeigt in Abschnitt A die Konzentrationen der Aβ-Peptide 1-37/38/39/40/42 im humanen Liquor der Patientengruppen OND-3, CID-3 und AD-3. In Abschnitt B ist der prozentuale Anteil der jeweiligen Aβ-Peptidspecies an der Summe aller Aβ-Peptide dargestellt. Die logarithmische Darstellung wurde gewählt, um die deutlich unterschiedlichen Liquorspiegel vergleichend darstellen zu können. Bei den Liquorkonzentrationen der Aβ-Peptide fällt auf, dass das zweithäufigste Aβ-Peptid im humanen Liquor nach $A\beta_{1-40}$ nicht $A\beta_{1-42}$ ist, sondern $A\beta_{1-38}$. Weiter ist bei AD-3 $A\beta_{1-42}$ erniedrigt. Die Aβ-Peptidgesamtkonzentration der untersuchten Gruppen ist weitgehend identisch. Wesentlich deutlichere Unterschiede zwischen den Kollektiven werden dagegen bei Betrachtung der prozentualen Aβ-Peptidanteile deutlich:

- CID-3 und AD-3 zeigen erhöhte Anteile von $A\beta_{1-38}$% und $A\beta_{1-39}$% im Vergleich zu OND-3
- $A\beta_{1-40}$% ist hochsignifikant bei AD-3 erhöht
- $A\beta_{1-42}$% ist hochsignifikant bei AD-3 erniedrigt und differenziert die Patienten mit AD deutlich besser als die zugehörige Aβ-Peptidkonzentration

Aus der Literatur ist bekannt, dass bei familiären AD-Formen mit APP-Punktmutationen in Nähe der β-Sekretaseschnittstelle vermehrt $A\beta_{1-40}$ und $A\beta_{1-42}$ überexpremiert werden, dagegen bei Mutationen in Nähe der γ-Sekretaseschnittstelle vermehrt $A\beta_{1-42}$ anfällt und das Verhältnis $A\beta_{1-42}$ zu $A\beta_{1-40}$ deutlich ansteigt. Es kann daher angenommen werden, dass bei Betrachtung der prozentualen Anteile der Aβ-Peptide vermehrt kranhkeitsspezifische Veränderungen der γ-Sekretaseaktivität abgebildet werden.

In Fig. 9 A und B werden wie unter Fig. 8 beschrieben die Untergruppen OND-3ε4minus, OND-3ε4plus und AD-3ε4plus verglichen. Damit kann zwischen ε4- und AD-abhängigen Effekten auf das Aβ-Peptid Muster im Liquor unterschieden werden. Bei OND-3ε4plus im Vergleich zu OND-3ε4minus sind tendentiell alle Aβ-Peptide im Liquor erniedrigt und entsprechend auch die Aβ-Peptidgesamtkonzentration (Abb 9A). Innerhalb des Aβ-Peptidquintetts ist die Erniedrigung von $A\beta_{1-42}$ besonders deutlich. Bei AD-3ε4plus ist dagegen selektiv $A\beta_{1-42}$ erniedrigt, allerdings in dem gleichen Ausmaß wie bei OND-3ε4plus. Die Aβ-Peptidgesamtmenge ist hier nicht erniedrigt (Fig. 9A). Damit können die ε4-positiven NDC-3 Patienten nicht über die alleinige Bestimmung von $A\beta_{1-42}$ im Liquor von den ε4-positiven AD-3 Patienten getrennt werden. Dies ist jedoch über die prozentualen Aβ-Peptidanteile möglich (Fig. 9B). Bedingt durch die selektive Erniedrigung von $A\beta_{1-42}$ bei AD-3ε4plus ist hier $A\beta_{1-42}$% besonders stark erniedrigt und das Kollektiv AD-3ε4plus kann über diesen Parameter ohne Überschneidung von den Gruppen OND-3ε4plus und OND-3ε4minus getrennt werden. Gleichzeitig ist der prozentuale Anteil von $A\beta_{1-40}$ bei AD-3ε4plus besonders stark erhöht.

Wie aus der Korrelationsmatrix in Fig. 10 hervorgeht ist das Aβ-Peptidquintett im Liquor eng miteinander korreliert und die prozentualen Anteile der Aβ-peptide and ihrer Gesamtkonzentration haben für biologische Parameter erstaunlich geringe Variationskoeffizienten. Diese Befunde legen eine enge enzymatische Regulation der Konzentration der fünf Aβ-Peptide durch β- und γ-Sekretase nahe. In diesem Zusammenhang wird weiter unten nachgewiesen (4.5.2), dass neben $A\beta_{1-40}$ und $A\beta_{1-42}$ besonders stark ausgeprägt die Entstehung der carboxyterminal verkürzten Aβ-Peptide durch synthetische Inhibitoren der β- und γ-Sekretase reduziert wird.

*4.2.2.4 Krankheitsspezifische Muster der prozentualen Aβ-Peptidanteile: Einzelfalldarstellung der Patientenkollektive*

Aus Fig. 11-13 kann entnommen werden, dass über $A\beta_{1-38}$%, $A\beta_{1-40}$% und $A\beta_{1-42}$% Patienten mit AD-3 und CID-3 von OND-3 Patienten differenziert werden können.

Fig. 11 weist eine signifikante negative Korrelation zwischen $A\beta_{1-38}$% und $A\beta_{1-42}$% für AD-3 Patienten nach. Der AD-3 Patient 143 wurde bei der Berechnung der Regressionsgerade nicht berücksichtigt und wird hier, wie auch nachfolgend (vergl. Fig. 12 & 13), als Ausreißer identifiziert. Klinisch zeigte dieser Patient ein Frühstadium der AD (MMSE 27/30).

Differentialdiagnostisch war in der Vorgeschichte eine depressive Pseudodemenz diskutiert worden.

In Richtung der Pfeilspitze der Regressiongraden nimmt der Schweregrad der Demenz zu. Auf den Zusammenhang zwischen den prozentualen Aβ-Peptidanteilen und Schweregrad der Demenz wird weiter unten näher eingegangen (vergl. Fig. 14 & 15). Bei einer Grenzkonzentration von $A\beta_{1-42}$% = 8.5 kann ohne Überschneidung das AD-3 Kollektiv von den Gruppen CID-3 und OND-3 getrennt werden. Der spezifische Zusammenhang zwischen $A\beta_{1-38}$% und $A\beta_{1-42}$% bei AD legt aber nahe, dass Patienten mit AD noch besser über eine Funktion ähnlich wie die Regressionsgerade zwischen $A\beta_{1-38}$% und $A\beta_{1-42}$% differenziert werden können. Dies hat unmittelbare Relevanz für die neurochemische AD-Diagnostik, denn über die Regressionsgerade als Grenzlinie würde beispielweise ein Patient mit $A\beta_{1-42}$% = 9.5 noch als AD erkannt, wenn gleichzeitig sein Wert für $A\beta_{1-38}$% 13.5 beträgt, wie von der Regressionsgerade vorhergesagt (vergl. Fig. 11). Entsprechendes gilt für den weiter unten gezeigten Zusammenhang zwischen den Quotienten Ab1-30/$A\beta_{1-40}$ und $A\beta_{1-42}$/$A\beta_{1-38}$ (Fig. 16). Die Grenzkonzentrationen für die Differenzierung des CID-3 Kollektivs lauten: $A\beta_{1-38}$% = 15.5 und $A\beta_{1-42}$% = 9.6%. Auf diese Weise werden fälschlich sechs Patienten als CID-3 klassifiziert. Diese Patieten sind durch ihre Codierung identifiziert. Bemerkenswert ist, dass bei genauer Analyse der klinischen Befunde bei einigen dieser Patienten (3/6) retrospektiv ein chronisch-entzündlicher Prozeß wahrscheinlich wird.

Fig. 12 zeigt $A\beta_{1-40}$% in Abhängigkeit von $A\beta_{1-42}$%. Eine AD-spezifische Korrelation zwischen diesen Parametern ist zwar signifikant (ohne Patient 143) aber weniger eng. Der Schweregrad der Demenz steigt in Pfeilrichtung.

Die Grenzwertkonzentrationen für AD-3 sind: $A\beta_{1-40}$% = 63 und $A\beta_{1-42}$% = 8.5.

Fig. 13 zeigt $A\beta_{1-40}$% in Abhängigkeit von $A\beta_{1-38}$%.

Die Grenzwertlinien $A\beta_{1-38}$% = 15.5 und $A\beta_{1-40}$% = 60.0 beziehen sich auf das Kollektiv CID-3. Eine AD-spezifische Korrelation zwischen diesen Parametern ist signifikant (ohne Patient 143). Der Schweregrad der Demenz steigt in Pfeilrichtung. Die Grenzwertlinien $A\beta_{1-38}$% = 16.0 und $A\beta_{1-40}$% = 63 definieren AD-3 Patienten mit schwerer Demenz.

Bemerkenswert ist hier, dass kein NDC-3 Patient oberhalb von $A\beta_{1-40}$% = 63 liegt und der Schnittpunkt dieser Grenzwertlinie mit der Regressionsgeraden, gleichzeitig die Grenzwertlinie $A\beta_{1-38}$% = 16 vorhersagt.

Aus Fig. 14 wird deutlich, dass AD-3 Patienten mit $A\beta_{1-38}$% < 16.0 oder $A\beta_{1-40}$% > 63 überwiegend eine schwere Demenzausgrägung zeigen (MMSE ≤ 10), andernfalls einen mittleren bis leichtgradigen Schweregrad der Demenz (MMSE > 10). Besonders ausgeprägt ist dieser Zusammenhang bei Patienten, die gleichzeitig beide Grenzwerte unter- bzw. überschreiten (Fig. 14; vergl. auch Fig. 13).

Die Korrelationsmatrix für den Zusammenhang zwischen den prozentualen Aβ-Peptidanteilen und Schweregrad der Demenz ist in Fig. 15 dargestellt. Signifikante Zusammenhänge finden sich für $A\beta_{1-37}$% und $A\beta_{1-40}$%. Auffällig ist, dass die Gruppe der carboxyterminal verkürzten Aß-Peptide im Gegensatz zu $A\beta_{1-40}$% positive Korrelationskoeffizienten für den letztgenannten Zusammenhang zeigt. Zwischen den absoluten Konzentrationen der Aβ-Peptide im Liquor und dem Schweregrad der Demenz wurde kein signifikanter Zusammenhang gefunden, d.h. auch hier werden krankheitsspezifische Zusammenhänge erst bei Betrachtung der prozentualen Aβ-Peptidanteile deutlich.

*4.2.2.5 Krankheitsspezifische Muster von Aβ-Peptidquotienten: Einzelfalldarstellung der Patientenkollektive*

Die Aβ-Peptidquotienten $A\beta_{1-38}$/$A\beta_{1-40}$, $A\beta_{1-42}$/$A\beta_{1-38}$ und $A\beta_{1-42}$/$A\beta_{1-40}$ erlauben eine Differenzierung zwischen den Kollektiven AD-3, CID-3 und OND-3. Dies hat den Vorteil, dass jetzt nur drei Aβ-Peptide quantifiziert werden müssen, um die drei Patientenkollektive zu differenzieren, führt aber zu einem gewissen Verlust an diagnostischer Trennschärfe. Damit bietet sich an, für die neurochemische Demenzdiagnostik und Identifizierung von Patienten mit chronisch entzündlichen ZNS-Erkrankungen ein ELISA-Triplett zu entwickeln ($A\beta_{1-38}$, $A\beta_{1-40}$, $A\beta_{1-42}$). Dieser Ansatz sollte mit einer detergentien-abhängigen Probenvorbereitung kombiniert werden (vergl. 4.2.4).

Fig. 16 zeigt $A\beta_{1-38}$/$A\beta_{1-40}$ in Abhängigkeit von $A\beta_{1-42}$/$A\beta_{1-38}$. Es besteht ein signifikanter und spezifischer Zusammenhang zwischen diesen beiden Parametern bei AD. In Richtung der Pfeilspitze der Regressionsgeraden nimmt die Schwere der Demenz zu (vergl. Fig. 18). Über die Grenzwertlinie $A\beta_{1-38}/A\beta_{1-40} = -0.5 (A\beta_{1-42}/A\beta_{1-38}) + 0.52$

wird erneut Patient 143 als Ausreißer identifiziert. Die übrigen AD-3 Patienten werden korrekt klassifiziert und kein NDC-3 Patient wird fälschlich dem Kollektiv AD-3 zugeordnet. Die Grenzwertlinien $A\beta_{1-38}/A\beta_{1-40}$ = 0.26 und $A\beta_{1-42}/A\beta_{1-38}$ = 0.57 beziehen sich auf das CID-3 Kollektiv.

Fig. 17 zeigt $A\beta_{1-38}/A\beta_{1-40}$ in Abhängigkeit von $A\beta_{42}/A\beta_{1-40}$. Es besteht ein signifikanter und spezifischer Zusammenhang zwischen diesen beiden Parametern bei AD. In Richtung der Pfeilspitze der Regressionsgeraden nimmt die Schwere der Demenz zu (vergl. Fig. 18). Über die Grenzwertlinie $A\beta_{1-42}/A\beta_{1-42}$ = 0.14 werden alle AD-3 Patienten korrekt klassifiziert und kein NDC-3 Patient fälschlich der AD-3 Gruppe zugeordnet. Die Grenzwertlinien $A\beta_{1-38}/A\beta_{1-40}$ = 0.26 und $A\beta_{1-42}/A\beta_{1-40}$ = 0.16 beziehen sich auf das CID-3 Kollektiv.

Fig. 18 macht deutlich, dass AD-3 Patienten mit einem Quotieten von $A\beta_{1-38}/ A\beta_{1-40}$ von weniger als 0.26 im Mittel eine schwere AD zeigen ((MMSE ≤ 10), sonst dagegen einen mittleren bis leichtgradigen Schweregrad der Demenz aufweisen (MMSE > 10).

### 4.2.3 NDC-2$^{KP}$

Die im Liquor erniedrigte Konzentration von $A\beta_{1-42}$ bei Patienten mit AD wurde bisher in zuvor bereits eingefrorenen Proben gefunden. Es wurde daher zunächst an Patienten des Kollektivs NCD-2$^{KP}$ untersucht, ob $A\beta_{1-42}$ im Liquor im Vergleich zu anderen Aβ-Peptiden besonders empfindlich für Kältepräzipitation ist. In diesem Zusammenhang wurde frisch gewonnener Liquor aliquotiert. Ein Aliquot wurde unbehandelt bei -80° C eingefroren. Mit dem anderen Aliquot wurde der als Trockensubstanz in Eppendorf Probengefäßen vorgelegte SDS-SB aufgenommen. Dieses Aliquot wurde nach SDS-/Hitzedenaturierung eingefroren. Mindestens 24 Stunden nach Lagerung bei -80° C erfolgte dann die vergleichende Analyse mittels Aβ-SDS-PAGE/Immunoblot-1 und densitometrischer Filmauswertung. Es wurden zehn neuropsychiatrische Kontrollpatienten ohne Alzheimer-Demenz untersucht. Bei neun dieser Patienten konnten $A\beta_{1-40}$ und $A\beta_{1-42}$ bestimmt werden. Bei einem Patienten konnte lediglich $A\beta_{1-40}$ ausgewertet werden.

Tabelle 16a macht deutlich, dass selektiv betont für $A\beta_{1-42}$ mit hoher interindividueller Varianz kältepräzipitationsbedingt ein Anteil des Peptids verloren geht. Der prozentuale Anteil von $A\beta_{1-42}$ im nativ eingefrorenen Liquor, der verloren geht, wenn die Kältepräzipitation nicht durch Vorbehandlung mit SDS-/Hitzedenaturierung vermindert wird, wurde wie folgt berechnet:

$$\%\Delta A\beta_{1-42} = ([A\beta_{1-42}nativ]_{conc.} - [A\beta_{1-42}SDS]_{conc.})/[A\beta_{1-42}SDS]_{conc.} \times 100.$$

Ein Wert für $\%\Delta A\beta_{1-42}$ von "-10" bedeutet beispielweise, dass $A\beta_{1-42}$ durch Einfrieren von nativem Liquor kältepräzipitationsbedingt um 10% im Vergleich zur "protektiven" Vorbehandlung mit SDS-/Hitzedenaturierung reduziert wurde. Da die Proben nicht vor dem Einfrieren gemessen werden konnten, kann nicht ausgeschlossen werden, dass in den Proben durch Kältepräzipitation ein zusätzlicher Anteil von $A\beta_{1-42}$ verloren geht, der auch durch die Vorbehandlung mit SDS-/Hitzedenaturierung nicht verhindert werden kann.

Tabelle 16b macht deutlich, dass $A\beta_{1-42}$ nach Einfrieren von nativem Liquor KP-bedingt im Mittel um etwa 30% reduziert wird (ΔAβ42%: -29.9 ± 10.9, MW ± SD; p=0.005). Die maximal beobachteten absoluten und prozentualen Abfälle für $A\beta_{1-42}$ betragen -798.3 pg bzw. -44.5%. Der leichte Abfall von $A\beta_{1-40}$ (ΔAβ40%: -3.5 ± 6.3; MW ± SD; p=n. s.) ist dagegen nicht signifikant, entsprechend aber das Verhältnis $A\beta_{1-42}/A\beta_{1-40}$ (ΔAβ42/40%: -27.0 ± 10.2; MW ± SD; p=0.008).

### 4.2.4 NDC-3$^{KP}$ und AD-3$^{KP}$

Anschließdend wurde anhand der Patientenkollektive NDC-3$^{KP}$ und AD-3$^{KP}$ untersucht, ob sich im Liquor krankheitsspezifische Unterschiede in der Kältepräzipitation von $A\beta_{1-42}$ zeigen. Die Quantifizierung von $A\beta_{1-42}$ im Liquor erfolgte mittels Aβ-SDS-PAGE/Immunoblot-2 und CCD-Kamera. Die differentielle Probenvorbehandlung erfolgte wie für das Kollektiv NDC-2$^{KP}$ ausgeführt.

Die Konzentrationen für $A\beta_{1-42}$ in Abhängigkeit der Probenvorbehandlung sind für die beiden Patientenkollektive in Tabelle 21 zusammengefaßt.

In Fig. 19 ist die Konzentrationen von $A\beta_{1-42}$ nach Einfrieren von nativem Liquor in Abhängigkeit ($A\beta_{1-42}$nativ) von der Kältepräzipitation dargestellt. Durch die Bestimmung von $A\beta_{1-42}$ im nativ eingefrorenem Liquor können die Patientenkollektive NDC-3$^{KP}$ und AD-3$^{KP}$ signifikant getrennt werden (p=0.0013). Dennoch besteht nach Fig. 19 eine deutliche Überschneidung der beiden Patientenkollektive. Demnach haben 6/15 Patienten des NDC-3$^{KP}$ Kollektivs $A\beta_{1-42}$ Liquorspiegel unterhalb der Grenzkonzentration (2100 pg/ml) des AD-3$^{KP}$ Kollektivs. Bei zusätzlicher Berücksichtigung der Grenzkonzentration von $\Delta A\beta_{1-42}$% (-17% bis -20%) wird dagegen nur noch ein NDC-3$^{KP}$ Patient fälschlich dem AD-3KP Kollektiv zugeordnet. Gleichzeitig werden alle Patienten des AD-3$^{KP}$ Kollektivs korrekt zugeordnet.

Die mittlere kältepräzipitationsbedingte Erniedrigung von $A\beta_{1-42}$ beträgt bei NDC-3$^{KP}$ -24.6% ± 18.8 (MW ± SD), was gut mit dem oben genannten Daten für die NDC-$2^{KP}$ Patienten übereinstimmt (-29.9 ± 10.9, MW ± SD). Dabei wird erneut bei den NDC-$3^{KP}$ Patienten eine erhebliche interindividuelle Varianz für das Ausmaß der kältepräzipitationsbedingten Erniedrigung von $A\beta_{1-42}$ im Liquor deutlich. Dabei wird das Ausmaß der kältepräzipitationsbedingten Erniedrigung von $A\beta_{1-42}$ bei NDC-$3^{KP}$ Patienten anscheinend wesentlich durch das Vorliegen des ApoE ε4 Allels bestimmt: Von den 4 Patienten mit der höchsten KP-bedingten Erniedrigung von $A\beta_{1-42}$ ($\Delta A\beta_{1-42}$% < -40%) tragen 3 Patienten ein ApoE ε4 Allel (vergl. Fig. 19)

AD Patienten zeigen dagegen keine nennenswerte kältepräzipitationsbedingte Erniedrigung von $A\beta_{1-42}$ und aus Fig. 19 geht hervor, dass die %$\Delta A\beta_{1-42}$ Werte hier um die Null-Achse streuen (-1.6 ± 10.2, MW ± SD). Entsprechend ist der Gruppenvergleich NDC-$3^{KP}$ versus AD-$3^{KP}$ für $\Delta A\beta_{1-42}$% signifikant (p=0.0025). Bemerkenswert ist, dass die KP-bedingte Erniedrigung von $A\beta_{1-42}$ bei AD-$3^{KP}$ fehlt, obwohl 9/11 Patienten ε4-positiv sind. Bei zwei AD-$3^{KP}$ Patienten ist der ApoE Genotypus nicht bekannt. Weiter fällt auf, dass AD-$3^{KP}$ und NDC-$3^{KP}$ Patienten mit mindestens einem ε4 Allel etwa gleich tiefe $A\beta_{1-42}$ Liquorspiegel haben. Dieser Zusammenhang wurde weiter oben auch bei dem Vergleich der $A\beta_{1-42}$ Liquorspiegel der Kollektive OND-3ε4plus und AD-3ε4plus deutlich (vergl. 4.2.2.3).

Gleichzeitig unterscheiden sich diese beiden Patientenuntergruppen besonders deutlich in ihrer KP-bedingten Erniedrigung von $A\beta_{1-42}$. Diese ist besonders stark bei den ε4-positiven NDC-3KP Patienten und fehlt fast vollständig bei den ε4-positiven AD-3KP Patienten. Demnach haben die ε4-positiven Patienten aus dem AD-$3^{KP}$ Kollektiv im Gegensatz zu den ε4-positiven Patienten aus dem NDC-$3^{KP}$ Kollektiv tiefe Liquorspiegel von $A\beta_{1-42}$ trotz "protektiver" SDS-/Hitzedenaturierung vor dem Einfrieren. Entsprechend sollten sich die beiden Patientenkollektive AD-$3^{KP}$ und NCD-$3^{KP}$ nach Vorbehandlung mit SDS/Hitzedenaturierung über die Bestimmung von $A\beta_{1-42}$ im Liquor ($A\beta_{1-42}$SDS) wesentlich besser differenzieren lassen. Fig. 20 bestätigt diese Annahme:
Die KP-bedingte Erniedrigung von $A\beta_{1-42}$ wird um den Anteil vermindert, der durch die "protektive" SDS-/Hitzedenaturierung verhindert werden kann, wodurch die NDC-$3^{KP}$ Patienten jetzt im Mittel deutlich höhere $A\beta_{1-42}$ Liquorspiegel ($A\beta_{1-42}$SDS) haben. Die $A\beta_{1-42}$ Liquorspiegel bei AD-3KP bleiben dagegen weitgehend unverändert tief.

Entsprechend verbessert sich das Signifikanzniveau des Gruppenvergleichs NDC-$3^{KP}$ versus AD-$3^{KP}$ bei Differenzierung der Kollektive über $A\beta_{1-42}$SDS deutlich ($p=1.81x10^{-6}$). Alle NDC Patienten (15/15) und nur ein AD Patient (1/11) liegen jetzt oberhalb der Grenzkonzentration von $A\beta_{1-42}$SDS = 2100 pg/ml.

Wie oben ausgeführt, ist dieser Effekt bei den Trägern von ε4 Allelen innerhalb des NDC-$3^{KP}$ Kollektivs besonders stark ausgeprägt. Fig. 19 und 20 machen aber deutlich, dass dieser Effekt nicht ausschließlich über das Vorliegen des ε4 Allels bestimmt wird. Einzelne Patienten mit beispielweise dem ApoE Genotypus 3/3 zeigen gleichfalls eine ausgeprägte KP-bedingte Erniedrigung von $A\beta_{1-42}$, die durch SDS-/Hitzedenaturierung vor dem Einfrieren vermindert werden kann. Zusammenfassend lassen sich folgende Aß-Peptidgrenzwerte angeben:

$A\beta_{1-42}$nativ = 2100 pg/ml & %$\Delta A\beta_{1-42}$ = -17%: Alle AD Patienten (11/11) werden richtig und ein NDC-Patient (1/15) wird falsch klassifiziert.
$A\beta_{1-42}$nativ = 2300 pg/ml & %$\Delta A\beta_{1-42}$ = -20%: Alle AD Patienten (11/11) werden richtig und zwei NDC-Patienten (2/15) werden falsch klassifiziert.
Und
$A\beta_{1-42}$SDS = 2100 pg/ml & %$\Delta A\beta_{1-42}$ = -17%: 10/11 AD-$3^{KP}$ Patienten werden richtig und kein NDC-$3^{KP}$ Patient (0/15) wird falsch klassifiziert.
$A\beta_{1-42}$SDS = 2300 pg/ml & %$\Delta A\beta_{1-42}$ = -20%: Alle AD Patienten (11/11) werden richtig und zwei NDC-Patienten (2/15) werden falsch klassifiziert.

Die Bestimmung von %$\Delta A\beta_{1-42}$ zusätzlich zu $A\beta_{1-42}$SDS wird voraussichtlich die neurochemische AD-Diagnostik weiter verbessern:
Bei einem $A\beta_{1-42}$SDS Grenzwert von 2300 pg/ml statt 2100 pg/ml werden drei NDC Patienten (3/15) falsch und alle AD Patienten korrekt klassifiziert. Wird zusätzlich der Grenzwert %$\Delta A\beta_{1-42}$ = -20% berücksichtigt, werden nur zwei NDC Patienten (2/15) falsch und alle AD Patienten korrekt klassifiziert. Damit kann die $A\beta_{1-42}$SDS Schwellenkonzentration um 200 pg/ml steigen, ohne dass sich gleichzeitig die diagnostische Spezifität verringert.

Zusammenfassend können aus den oben genannten Befunden zur KP-bedingten Erniedrigung von Aβ-Peptiden folgende Hypothesen abgeleitet werden:
$A\beta_{1-42}$ liegt im humanen Liquor im Vergleich zu $A\beta_{1-40}$ vermehrt in einer Fraktion vor, die KP-abhängig reduziert werden kann. Durch Einsatz von Detergentien kann $A\beta_{1-42}$ zumindest partiell aus dieser Fraktion freigesetzt werden und die KP-bedingte Erniedrigung reduziert werden. Bei dieser $A\beta_{1-42}$ bindenden Fraktion handelt es sich wahrscheinlich um vergleichsweise hydrophobe höhermolekulare Komplexe unter Beteiligung von $A\beta_{1-42}$ und wahrscheinlich anderer Proteine (z.B. Lipoproteine).
(Anmerkung: Durch Analyse von Fraktionen aus der Gelfiltration (SEC-FPLC) von humanem Liquor mittels Aβ-SDS-PAGE/Immunoblot konnte gezeigt werden, dass selektiv betont für $A\beta_{1-42}$ ein beträchtlicher Anteil in einer hochmolekularen Fraktion transportiert wird).

Bei AD - im Gegensatz zu NDC - läßt sich $A\beta_{1-42}$ auch durch starke Detergentien kaum aus dieser Fraktion verdrängen, was auf eine AD-spezifische Zusammensetzung dieses Komplexes hinweist. In diesem Fall wäre zu erwarten, dass $A\beta_{1-42}$ im Liquor bei AD auch dann spezifisch erniedrigt wäre, wenn die Proben nach Detergentienbehandlung direkt gemessen würden, d.h. ohne vorheriges Einfrieren. Damit könnten die Proben nach Detergentienbehandlung bei Raumtemperatur in Anwesenheit von SDS und Proteaseinhibitoren (3.1.3.1b, SDS-SB-3) bis zur Messung gelagert werden, da diese sehr effektiv vor Autoaggregation, Präzipitation, unspezifischer Proteaseaktivität und Keimbesiedlung geschützt wären.

Alternativ kann angenommen werden, dass die Erniedrigung von $A\beta_{1-42}$ bei AD im wesentlichen dadurch bedingt ist, dass der Liquor bei AD insgesamt weniger $A\beta_{1-42}$ enthält.

Bei hochaffiner detergentien-stabiler Bindung von $A\beta_{1-42}$ an einen Komplex wird das Peptid in dieser Bindung vermehrt dem enzymatischem Katabolismus entzogen. Damit stellt dieser Komplexes auch ein Target für die Entwicklung von Medikamenten gegen die Alzheimer-Demenz dar, da Substanzen die mit der Bindung von Aβ-Peptiden an diesen Komplex konkurieren, Aβ-Peptide vermehrt dem enzymatischen Katabolismus zuführen könnten. Die Reduktion von $A\beta_{1-42}$ im Liquor bei einem Teil der Patienten mit Creutzfeldt-Jakob-Demenz, einer weiteren Amyloidose oder Proteinfaltungskrankheit des ZNS, legt nahe, dass dieser Komplex bei beiden Erkrankungen ein vergleichbare Zusammensetzung aufweisen könnte.

Die oben genannten Befunde sind auch für die Frühdiagnostik oder präklinische Diagnostik der AD relevant. Hier stellt sich die Frage, ob Patienten, die trotz "protektiver " SDS/Hitzedenaturierung ($A\beta_{1-42}$SDS) tiefe $A\beta_{1-42}$ Liquorspiegel zeigen und gleichzeitig durch eine geringe KP-bedingte Abnahme von $A\beta_{1-42}$ ($\Delta A\beta_{1-42}$%) auffallen, ein besonders hohes Risiko für die Entwicklung einer AD tragen. Diese Fragestellung könnte durch eine prospektive Untersuchung bei Patienten mit leichten kognitiven Störungen (ICD10 F06.7) beantwortet werden, da diese Patienten in bis zu 30% der Fälle innerhalb von zwei Jahren eine AD entwickeln. Hier wäre eine einmalige Liquorpunktion mit anschließdender Verlaufsbeurteilung (Klinik, Neuropschologie, Bildgebung) ausreichend, da der prädiktive Wert der Parameter retrospektiv bestimmt werden könnte.

Damit liegt nahe, prinzipiell bei jedem Patienten ein A- (nativ eingefroren) und B-Aliquot (SDS-/Hitzedenaturierung vor dem Einfrieren) der Liquorprobe zu gewinnen. Gegebenenfalls reicht es aus, die Proben unter Kontrolle der Temperatur der individuellen Probe standardisiert auf beispielweise 0° C abzukühlen.

Allgemein wird man die oben dargestellte differentielle Probenvorbereitung auch mit ELISA-Methoden oder Einsatz der Fluoreszenzkorrelationsspektroskopie (FCS) für die Bestimmung von $A\beta_{1-42}$ im Liquor kombinieren können.

Die Nachweisempfindlichkeit des ELISA der Firma BioSource für die Bestimmung von $A\beta_{1-42}$ im humanen Liquor liegt beispielsweise bei 10 pg/ml. Diese Nachweisempfindlichkeit erlaubt bei Auftrag von 100 µl Probe, den SDS-/Hitzedenaturierten Liquor vor der Messung mindestens fünfach zu verdünnen. Die resultierende Konzentration von 0.1% SDS (w/v) beinträchtigt nach eigenen Ergebnissen nicht die in diesem ELISA im ersten Schritt verwendeten N-terminalen Fangantikörper. Analog kann dies auch für die innerhalb der RIPA-IP eingesetzten N-terminal selektiven Antikörper 1E8 und 6E10 belegt werden.

Bei der FCS mit Kreuzkorrelation unter Verwendung von Fluorenzenz-markierten N-terminal und C-terminal selektiven Antikörpern ist die Signalintensität proportional der innerhalb solcher Aggregate gebundenen Aβ-Peptide. Die Sensitivität der Methodik erlaubt auch hier eine Verdünnung der Probe nach SDS-/Hitzedenaturierung auf SDS-Konzentrationen von beispielweise 0.1% w/v. Sollte $A\beta_{1-42}$ selektiv bei AD detergentien-stabil an höhermolekulare Aggregate gebunden vorliegen wird durch Vorbehandlung der Liquorproben mit Detergentien die Spezifität der Messung erhöht, da die Aβ-Peptide bei den NDC Patienten im Gegensatz zu den AD Patienten aus den höhermolekularen Aggregaten freigesetzt werden. Die verminderte Reduktion des Fluoreszenzsignals der FCS (Kreuzkorrelation) nach Detergentienbehandlung bei Patienten mit AD im Vergleich zu Patienten mit NDC könnte damit für die neurochemische AD Diagnostik relevant werden.

### 4.2.5 Hirnhomogenate von Patienten mit AD, frontotemporaler Demenz, Lewy-Körperchen-Demenz und Kontrollen

Himgewebe (frontotemporaler Cortex, Cerebellum) von Patienten mit AD, frontotemporaler Demenz (FTD), Lewy-Körperchen-Demenz (LBD) und nicht-dementen Kontrollen wurde wurde in Anwesenheit von RIPA Detergenzienpuffer homogenisiert (3.4.6). Anschließend wurde eine Immunopräzipitation in Anwesenheit von RIPA durchgeführt. In der RIPA-Detergenz extrahierbaren Fraktion der Aβ-Peptide ließen sich $A\beta_{1-38}$, $A\beta_{1-40}$, $A\beta_{1-42}$ und $A\beta_{2-42}$ nachweisen (Fig. 21a,b). $A\beta_{2-42}$ wurde durch MALDI-TOF Analyse direkt von der Blotmembran (Daten nicht gezeigt), Aβ-SDS-PAGE/Immunoblot-2 (Fig. 23b) und Aβ-IPG-2D-PAGE/ Immunoblot-2 (Fig. 24c) identifiziert. $A\beta_{2-42}$ wird auch in Liquorproben bei AD (Fig. 23a) und in Zellkulturüberständen nachgewiesen (Fig. 23a, Fig. 28a,b).

Patienten mit AD waren im Vergleich zu nicht-dementen Kontrollen und Patienten mit FTD durch eine massiven Anstieg von $A\beta_{1-42}$ und $A\beta_{2-42}$ gekennzeichnet. Dieser Anstieg war im frontotemporalen Kortex weitaus ausgeprägter als im Zerebellum (Fig. 22). Patienten mit LBD zeigten Anstiege von $A\beta_{1-42}$ und $A\beta_{2-42}$ in Abhängigkeit der Anzahl zusätzlich vorliegender Alzheimer-typischer β-Amyloidplaques, die über die CERAD Klassifikation erfasst werden (Fig. 21b): Patieten mit LBD CERAD A hatten deutlich weniger $A\beta_{1-42}$ und $A\beta_{2-42}$ als Patienten mit LBD CERAD C.

Bei Alzheimer-Demenz fielen vergleichsweise hohe Konzentrationen von $A\beta_{1-38}$ auf (Fig. 21a,b). Gleichzeitig war eine gewebsspezifische Expression von $A\beta_{1-38}$ bei AD auffällig, da im Zerebellum relativ zum frontotemporalen Kortex $A\beta_{1-38}$ deutlich geringer war oder nicht nachweisbar war und zusätzlich bei einigen Patienten eine bisher nicht charakterisierte Bande unterhalb von $A\beta_{1-38}$ meßbar wurde (Fig. 22). Da der carboxyterminale Schnitt durch γ-Sekretase(n) erfolgt, ergibt sich damit ein Hinweis auf eine ggf. gewebsspezifisch unterschiedliche Expression von γ-Sekretase(n). Da sich im Zerebellum im Vergleich zu anderen Hirnregionen bei AD bekanntlich wenig Alzheimer-typische neuropathologische Veränderungen zeigen, könnte dieser Befund pathophysiologisch relevant sein.

Die extrem hohen Konzentrationen von $A\beta_{2-42}$, zum Teil in Höhe von $A\beta_{1-42}$, in einer RIPAetrahierbaren Himpräparation wurde bisher nicht beschrieben. Bei einigen Patienten war zusätzlich $A\beta_{1-40}$ vergleichsweise stark erhöht. Damit kann der Aβ-SDS-PAGE/Immunoblot für die neuropathologische Differentialdiagnostik dementieller Erkrankungen eingesetzt werden und ggf. über eine biochemische Phänotypisierung zur Differenzierung von Subgruppen der sporadischen AD beitragen.

### 4.3 Cisternaler Liquor von Kaninchen und Meerschweinchen.

Auch im cisternalen Liquor des adulten Meerschweinchens (Fig. 25a) und Kaninchens (Fig. 25b) sind $A\beta_{1-37/38/39}$ neben $A\beta_{1-40}$ und $A\beta_{1-42}$ nachweisbar. Die Varianz der Meßwerte wird deutlich reduziert, wenn analog zu den Patientenproben vor dem Einfrieren der Proben die SDS-/Hitzedenaturierung durchgeführt wird. Diese Probenvorbehandlung ist für Wirkstoffindungsprojekte unter Einsatz von Meerschweinchen oder Kaninchen als Tiermodell relevant, da bestimmte Substanzeffekte (z.B. Sekretaseinhibition) auf diese Weise schon mit deutlich weniger Tieren pro Behandlungs- und Kontrollgruppe nachgewiesen werden können.

### 4.4 Hippokampale Gewebeschnitte des adulten Meerschweinchens mit Kurzzeitkultur

In Kurzzeitkulturen hippokampaler Gewebeschnitte des adulten Meerschweinchens werden $A\beta_{1-37}$/38/39 neben $A\beta_{1-40}$ und $A\beta_{1-42}$ in den Überstand sezerniert und sind auch intrazellulär nachweisbar (Fig. 26).

### 4.5 Zellkultur

#### 4.5.1 Primäre telencephale Hühnchenkultur

Da die Aβ-Peptid Aminsäuresequenz des Hühnchens und die humane Sequenz übereinstimmen, wurde ein primär neuronales Zellkultursystem aus den Vorderhinbläschen von Hühnerembryonen etabliert (vergl. 3.7). Dabei ergab sich, dass neben $A\beta_{1-40}$ und $A\beta_{1-42}$ die C-terminal verkürzten Aβ-Peptide 1-37/38/39 in die Zellkulturüberstände freigesetzt werden, und die relative Verteilung der Aβ-Peptide gut mit der im humanen Liquor übereinstimmt.

#### 4.5.2 Transgene $APP751_{Sw}$ Neuroglioma Zellinie

Vergleichend wurde das Aβ-Peptidmuster in Neuroglioma-Zellen (H4) untersucht, die mit ${}_{human}APP751_{Sw}$ transfiziert wurden. Fig. 28a&b zeigt, dass auch hier neben $A\beta_{1-40}$ und $A\beta_{1-42}$, die C-terminal verkürzten Aβ-Peptide 1-37/38/39 in die Zellkulturüberstände freigesetzt werden. Zusätzlich kann $A\beta_{2-42}$ identifiziert werden. Nach Behandlung mit Inhibitoren der β-/γ-Sekretasen (Calpain-Inhibitor I&II, Calpeptin, MG132, Leupeptin) werden neben $A\beta_{1-40}$ und $A\beta_{1-42}$ auch die C-terminal verkürzten Aβ-Peptide 1-37/38/39 und das N-terminal verkürzte $A\beta_{2-42}$ reduziert (Fig. 28a,b). Fig. 28b zeigt die dosisabhängige Reduktion unter Calpaininhibitor-1.

Entsprechend kann angenommen werden, dass die Aβ-Peptide 1-37/38/39, wie für 1-40/42 bekannt, auch durch den kombinierten β-/γ-Sekretaseschnitt entstehen. Die Reduktion von 2-42 kann durch Hemmung von β-/γ-Sekretaseaktivität bedingt sein, oder durch vermindertes Substratangebot ($A\beta_{1-42}$) für eine nachgeschaltete N-terminale Aminopeptidase (siehe aber unten).

Aus Fig. 29a&b geht hervor, dass die C-terminal verkürzten Aβ-Peptide 1-37, 1-38 und 1-39 mit einer anderen Kinetik gehemmt werden als $A\beta_{1-40}$ und $A\beta_{1-42}$. Der Unterschied in der Kinetik ist besonders ausgeprägt für $A\beta_{1-37}$. Dieser Befund zeigt an, dass über das Aβ-Peptidmuster eine Heterogenität der γ-Sekretaseaktivität abgebildet werden kann, was für die Wirkstoffindung selektiver γ-Sekretaseinhibitoren relevant ist. Weiter ist bemerkenswert, dass der aus der Literatur bekannte paradoxe Anstieg von $A\beta_{1-42}$ bei niedrigen Inhibitorkonzentrationen nicht mit einem Anstieg von $A\beta_{2-42}$ korreliert ist. Dies spricht gegen eine sekundäre Entstehung von $A\beta_{2-42}$ aus $A\beta_{1-42}$.

Für die Entstehung von $A\beta_{1-37}$ muß eine weitere Alternative berücksichtigt werden. Kürzlich wurde beschrieben, dass die Neutrale Endopeptidase (NEP) durch den kombinierten Schnitt 10/11 und 37/38 am Katabolismus von Aβ-Peptiden wesentlich beteiligt ist (Iwata et al., 2000). Damit könnte $A\beta_{1-37}$ auch durch die Kombination BACE-Schnitt und NEP-Schnitt 37/38 entstehen.

**Liste der Abkürzungen :**

| | |
|---|---|
| Aβ - | β-Amyloid |
| AD - | Alzheimer Demenz |
| APP - | Amyloidvorläuferprotein |
| FAD - | familiäre AD, d.h. genetisch bedingt |
| PS-1 - | Presenilin 1 |
| PS-2 - | Presenilin 2, |
| Bis - | N, N'-Methylenbisacrylamide |
| Bicin - | N,N'-bis-[2-Hydroxyethyl]-Glycin |
| %T - | Gesamtacrylamid Monomer Konzentration (w/v) |
| %C - (w/w) | Anteil von Bis an der Gesamtmenge des Acrylamid Monomers |
| Aβ-SDS-PAGE - | β-Amyloid Natrium Laurylsulfat Polyacrylamidgelelektrophorese |
| Aβ-2D-PAGE - | β-Amyloid-zweidimensionale-Polyacrylamidgelelektrophorese |
| IPG - | immobilisierter pH Gradient |
| Aβ1-n - | $A\beta_{1-n}$ |

**5. Literatur**

Görg A., Boguth G., Obermaier C., Posch A. and Weiss W. (1995) Two-dimensional polyacrylamide gel electrophoresis with immobilized pH gradients in the first dimension (IPG-Dalt): the state of the art and the controversy of vertical versus horizontal systems. *Electrophoresis* **16,** 1079-86.

Görg A., Obermaier C., Boguth G., Csordas A., Diaz J.J. and Madjar J.J. (1997) Very alkaline immobilized pH gradients for two-dimensional electrophoresis of ribosomal and nuclear proteins. *Electrophoresis* **18,** 328-37.

Heukeshoven J. and Dernick R. (1988) Improved silver staining procedure for fast staining in PhastSystem Development Unit. I. Staining of sodium dodecyl sulfate gels. *Electrophoresis* **9,** 28-32.

Hulstaert F., Blennow K., Ivanoiu A., Schoonderwaldt H.C., Riemenschneider M., De Deyn P.P., Bancher C., Cras P., Wiltfang J., Mehta P.D., Iqbal K., Pottel H., Vanmechelen E. and Vanderstichele H. (1999) Improved discrimination of AD patients using beta-amyloid(1-42) and tau levels in CSF. *Neurology* **52,** 1555-62.

Ida N., Hartmann T., Pantel J., Schroder J., Zerfass R., Forstl H., Sandbrink R., Masters C.L. and Beyreuther K. (1996) Analysis of heterogeneous A4 peptides in human cerebrospinal fluid and blood by a newly developed sensitive Western blot assay. *J Biol Chem* **271,** 22908-14.

Klafki H., Abramowski D., Swoboda R., Paganetti P.A. and Staufenbiel M. (1996) The carboxyl termini of beta-amyloid peptides 1-40 and 1-42 are generated by distinct gammasecretase activities. *J Biol Chem* **271,** 28655-9.

Klafki H.W., Wiltfang J. and Staufenbiel M. (1996) Electrophoretic separation of betaA4 peptides (1-40) and (1-42). *Anal Biochem* **237,** 24-9.

Kuo Y.M., Emmerling M.R., Woods A.S., Cotter R.J. and Roher A.E. (1997) Isolation, chemical characterization, and quantitation of A beta 3- pyroglutamyl peptide from neuritic plaques and vascular amyloid deposits. *Biochem Biophys Res Commun* **237,** 188-91.

Laemmli U. (1970) Cleavage of structural proteins during the assembly of the head of bacteriophage T4. *Nature* **227,** 680-685.

McKhann G., Drachman D., Folstein M., Katzman R., Price D. and Stadlan E.M. (1984) Clinical diagnosis of Alzheimer's disease: report of the NINCDS-ADRDA Work Group under the auspices of Department of Health and Human Services Task Force on Alzheimer's Disease. *Neurology* **34,** 939-44.

Metz C.E. (1978) Basic principles of ROC analysis. *Semin Nucl Med* **8,** 283-98.

O'Farrell P., Goodman H. and O'Farrell P. (1977) High resolution two-dimensional electrophoresis of basic as well as acidic proteins. *Cell* **12,** 1133-41.

O'Farrell P.H. (1975) High resolution two-dimensional electrophoresis of proteins. *J Biol Chem* **250,** 4007-21.

Righetti P.G. and Bossi A. (1997) Isoelectric focusing in immobilized pH gradients: recent analytical and preparative developments. *Anal Biochem* **247,** 1-10.

Russo C., Saido T.C., DeBusk L.M., Tabaton M., Gambetti P. and Teller J.K. (1997) Heterogeneity of water-soluble amyloid beta-peptide in Alzheimer's disease and Down's syndrome brains. *FEBS Lett* **409,** 411-6.

Schagger H. and von Jagow G. (1987) Tricine-sodium dodecyl sulfate-polyacrylamide gel electrophoresis for the separation of proteins in the range from 1 to 100 kDa. *Anal Biochem* **166**,368-79.

Tamaoka A., Sawamura N., Fukushima T., Shoji S., Matsubara E., Shoji M., Hirai S., Furiya Y., Endoh R. and Mori H. (1997) Amyloid beta protein 42(43) in cerebrospinal fluid of patients with Alzheimer's disease. *J Neurol Sci* **148,** 41-5.

Thome J., Kornhuber J., Munch G., Schinzel R., Taneli Y., Zielke B., Rosler M. and Riederer P. (1996) [New hypothesis on etiopathogenesis of Alzheimer syndrome. Advanced glycation end products (AGEs)]. *Nervenarzt* **67,** 924-9.

Thome J. M.G., Schinzel R., Kornhuber J., Blum-Degen D., Sitzmann L., Rösler M., Heidland A., Riederer P. (1996) Advanced glycation endproducts- associated parameters in the peripheral blood of patients with Alzheimer's disease. *Life Sci.* **59,** 679-685.

Wiltfang J., Arold N. and Neuhoff V. (1991) A new multiphasic buffer system for sodium dodecyl sulfate- polyacrylamide gel electrophoresis of proteins and peptides with molecular masses 100,000-1000, and their detection with picomolar sensitivity. *Electrophoresis* **12,** 352-66.

Wiltfang J., Smirnov A., Schnierstein B., Kelemen G., Matthies U., Klafki H.W., Staufenbiel M., Huther G., Ruther E. and Kornhuber J. (1997) Improved electrophoretic separation and immunoblotting of beta-amyloid (A beta) peptides 1-40, 1-42, and 1-43. *Electrophoresis* **18,** 527-32.

Wiltfang J., Otto M., Rüther E., Kornhuber J. (1998) Klinisch-chemische Früh- und Differentialdiagnostik der Alzheimer Demenz, *psycho* **24,** 726-31.

Wiltfang J., Esselmann H., Smirnov A., Maler M.J., Bleich S., Otto M., Bibl M., Rüther E., Kornhuber J. (2000) Therapieansätze in der Alzheimer-Demenz, *Notfallmedizin* **26,** 246-51.

**Tabelle 1:** Stammlösungen und Puffer für die Aβ-SDS-PAGE

| Lösung | Zusammensetzung |
|---|---|
| Trenngelpuffer | 1.6 M Tris, 0.4 M $H_2SO_4$ |
| Sammelgelpuffer | 0.8 M Bistris, 0.2 M $H_2SO_4$ |
| Kammgelpuffer | 0.72 M Bistris, 0.32 M Bicin |
| Kathodenpuffer | 0.2 M Bicin, 0.1 M NaOH, 0.25 % w/v SDS |
| Anodenpuffer | 0.2 M Tris, 0.05 M $H_2SO_4$ |
| 1%ige SDS | 1% (w/v) SDS |
| 10%ige SDS | 10% (w/v) SDS |
| Acrylamid/Bis 60 %T / 3 %C[1] | 58.2 % (w/v) Acrylamid, 1.8 % (w/v) Bis |
| Acrylamid/Bis 60 %T / 5 %C[1] | 57% (w/v) Acrylamid, 3% (w/v) Bis |

[1] Die Acrylamid Stammlösung wird für 30 min mit AG 501-X8D Mischbettionenaustauscher Matrix (Bio-Rad, RichmNDC, CA, USA) gerührt, gefiltert, und bei Raumtemperatur im Dunkeln aufbewahrt.

**Table 2:** Zusammensetzung von Kamm-, Sammel-, und Trenngel für die Aβ-SDS-PAGE

| Lösung | Kammgel 9%T / 5%C | Sammelgel 6%T / 5% C | Trenngel 12%T / 5%C / 8M Harnstoff |
|---|---|---|---|
| Kammgelpuffer | 2000 µl | | |
| Sammelgelpuffer | | 2000 µl | |
| Trenngelpuffer | | | 2500 µl |
| Acrylamid/Bis (60 %T / 3 %C) | 400 µl | 400 µl | |
| Acrylamid/Bis (60 %T / 5 %C) | | | 2000 µl |
| Harnstoff | | | 4,80 g |
| 1% (w/v) SDS | 1000 µl | 1000 µl | |
| 10% (w/v) SDS | | | 250 µl |
| $H_2O$ | 600 µl | 600 µl | ad 10 ml (ca. 1.51 ml) |
| 10% AMPS | 24 µl | 24 µl | 40 µl |
| TEMED | 8 µl | 8 µl | 5 µl |
| Bromphenol Blau, 1% (w/v) | 15 µl | | |

**Tabelle 3**: Zusammensetzung der Puffer für die Probenaufnahme (SDS-SB)

| Reagenz | SDS-SB-1* | SDS-SB-2* | SDS-SB-3* |
|---|---|---|---|
| Bistris | 0,36 M | 0,72 M | 0,120 M |
| Bicine | 0,16 M | 0,32 M | 0,053 M |
| Saccharose | 15,0% (w/v) | 30,0 % (w/v) | 5,0% (w/v) |
| SDS | 1,0% (w/v) | 2,0% (w/v) | 0,5% (w/v) |
| Bromphenol Blau | 0,004% (w/v) | 0,008% (w/v) | 0,002% (w/v) |
| Proteinase inhibitor cocktail tablets | | | 1 Tabl. / 10 ml |

* direkt vor Aß-SDS-PAGE mit 2,5% (v/v) 2-Mercaptoethanol 5 min bei 95°C aufkochen

**Tabelle 4a**: Zusammensetzung des IEF-SB

| Reagenz | IEF-SB |
|---|---|
| Harnstoff | 8 M |
| CHAPS | 0,27% (w/v) |
| NP-40 | 0,13% (v/v) |
| Servalyt pH 3-10 (40%ig) | 1% (v/v) |
| Servalyt pH 4-7 oder pH 5-6 | 1% (v/v) |
| 2-Mercaptoethanol | 5% (v/v) |

**Tabelle 4b**: Zusammensetzung des Trägerampholyt-IEF-Rundgels

| Lösung | Volumen | IEF-Rundgel |
|---|---|---|
| Acrylamid/Bis (60%T / 3%C) | 89 µl | 5,4%T / 3%C |
| $H_2O_{dd}$ | 280 µl | |
| Harnstoff | 480 mg | 8 M |
| 2% (w/v) CHAPS | 135 µl | 0,27% |
| 2% (w/v) NP-40 | 65 µl | 0,13% |
| Servalyt pH 3-10 (40%ig) | 25 µl | 1% |
| Servalyt pH 5-6 oder 4-7 | 25 µl | 1% |
| 1% (w/v) AMPS | 20 µl | |
| 10% (v/v) TEMED | 10 µl | |

**Tabelle 4c:** Zusammensetzung von Anolyte und Katholyte für die Trägerampholyt-IEF

| Lösung | Zusammensetzung |
|---|---|
| Katholyt | 20 mM NaOH |
| Anolyt | 10 mM $H_3PO_4$ |

**Tabelle 4d:** Zusammensetzung des IEF-Equilibrierungspuffers und der IEF-Agaroselösung

| Reagenz | IEF-Equilibrierungspuffer | IEF-Agaroselösung |
|---|---|---|
| Bicin | 0,16 M | 0,16 M |
| Bistris | 0,36 M | 0,36 M |
| SDS | 1% (w/v) | 1% (w/v) |
| Bromphenol Blau | | 0,004% (w/v) |

**Tabelle 5a:** Zusammensetzung von IPG-SB (Lysispuffer) und IPG-Rehydrierungspuffer

| Reagenz | IPG-SB (Lysispuffer) | IPG-Rehydrierungslösung |
|---|---|---|
| Harnstoff | 9,0 M | 8,0 M |
| Serdolit MB-1 | 1,0% (w/v) | 1,0% (w/v) |
| CHAPS | 2,0% (w/v) | 0,5% (w/v) |
| DTT | 1,0% (w/v) | 0,2% (w/v) |
| Pharmalyte pH 3-10 (40%) | 0,8% (v/v) | 0,8 (v/v) |

**Tabelle 5b:** Zusammensetzung der IPG-Equilibrierungspuffers

| Reagenz | IPG-Equilibrierungspuffer |
|---|---|
| Harnstoff | 6,0 M |
| Glycerol (100%) | 20,0% (w/v) |
| SDS | 2,0% (w/v) |
| Bistris | 0,36 M |
| Bicin | 0,16 M |

1. 10 min in IPG-Equilibrierungspuffer mit 1% (w/v) DTT bei Raumtemperatur unter leichtem Schütteln inkubieren

2. 10 min in IPG-Equilibrierungspuffer mit 4,8% (w/v) Iodoacetamid bei Raumtemperatur unter leichtem Schütteln inkubieren

**Tabelle 5c:** Zusammensetzung der IPG-Agaroselösung

| Reagenz | IPG-Agaroselösung |
|---|---|
| Agarose | 1,0% (w/v) |
| SDS | 0,25% (w/v) |
| Bromphenol Blau | 0,002% (w/v) |
| Bistris | 0,36 M |
| Bicin | 0,16 M |

**Tabelle 6:** Zusammensetzung der RIPA-Puffer

| Reagenz | *$RIPA_{1x}$ | *$RIPA_{0.5x}$ |
|---|---|---|
| HEPES/NaOH pH 7.4 | 50 mM | 50 mM |
| NP40 | 1,0% (v/v) | 0,5% (v/v) |
| Na-Deoxycholat | 0,5% (w/v) | 0,25% (w/v) |
| SDS | 0,1% (w/v) | 0,05% (w/v) |
| NaCl | 150 mM (w/v) | 150 mM (w/v) |

*Zusatz: 1 Tablette Protease Inhibitor Mix pro 10 ml $RIPA_{0.5x}$ bzw. $RIPA_{1x}$ -Puffer

**Table 7:** Silberfärbung nach Glutardialdehydfixierung

| Lösungen | Zeit (min) |
|---|---|
| kaltes (4°C) $H_2O_{dd}$ | 3 x 10 |
| 30% (v/v) EtOH, 10% (v/v) Hac[1] | 1 x 60 |
| 30% (v/v) EtOH, 0.5 M Natriumazetat, 0.5% (v/v) Glutardialdehyd, 0.2% $Na_2S_2O_3$ | 1 x 60 |
| kaltes (4°C) $H_2O_{dd}$ | 3 x 15 |
| 0.1% (w/v) $AgNO_3$, 0.02% (v/v) Formaldehyd | 1 x 60 |
| 2.5% (w/v) $Na_2CO_3$, 0.02% (v/v) Formaldehyd | 5-10 |
| 0.05 M (w/v) Glycin | 30 |

[1] Gele nach Glutardialdehydfixierung (Wiltfang et al., 1997, Electrophoresis 18: 527-32) können in dieser Lösung bei 4°C gelagert werden, wenn die Silberfärbung später durchgeführt werden soll. Die hier untersuchten Aβ-Peptide und Proteine können auch direkt in EtOH/Hac fixiert werden.

**Tabelle 8:** Zusammensetzung der Blotpuffer für den Western-Immunoblot

| Reagenz | Blotpuffer A | Blotpuffer B | Blotpuffer C |
|---|---|---|---|
| Tris | 0.21 M | 25 mM | 25 mM |
| Methanol | 30% (v/v) | 30% (v/v) | |
| SDS | | | 0.025% (w/v) |
| pH | 10,4 | 10,4 | 9,0* |

* mit 0.5 M Borsäure eingestellt

**Tabelle 9a:** Stammlösungen für den Immunoblot

| Lösung | Zusammensetzung |
|---|---|
| $PBS_{10x}$ (10x konz.) | 95.5 g Phosphat-gepuffertes NaCl auf 1000 ml $H_2O_{dd}$ |
| PBS-T (0.075% v/v Tween-20 in PBS) | 75 ml Tween-20 ad 1000 ml $PBS_{1x}$ |
| PBS-T-M | 2.5 g Milchpulver ad 100 ml PBS-T |

**Tabelle 9b:** Puffer, Lösungen und Antikörper für Western-Immunoblot-1 und -2

| Immunoblot-Schritt | Zeit | Western-Immunoblot-1 | Western-Immunoblot-2 |
|---|---|---|---|
| Blockierung unspezifischer Bindungsstellen | 1 h | 2.5% (w/v) Milchpulver in PBS-T | 10% (v/v) Roti-Block in $H_2O_{dd}$ |
| Inkubation mit primärem mAb bei 4°C über Nacht | 15 h | a) mAb 1E8, 1:4000 in Milchpulver-PBS-T; mAb 6E10, 1:1000 in Milchpulver-PBS-T | a) mAb 1E8, 1:4000 in 10% (v/v) Roti-Block / $H_2O_{dd}$ |
| Waschschritt | 3x10 min | PBS-T | PBS-T |
| Inkubation mit sekundärem Ab bei Raumtemperatur | 1 h | biotinylierter anti-Maus-Antikörper, 1:3000 in Milchpulver-PBS-T | biotinylierter anti-Maus-Antikörper, 1:3000 in PBS-T |
| Waschschritt | 3x10 min | PBS-T | PBS-T |
| Inkubation mit Streptavidin-POD bei Raumtemperatur | 1 h | Streptavidin-POD-Komplex,1:3000 in PBS-T | Streptavidin-POD-Komplex,1:3000 in PBS-T |
| Waschschritt | 3x10 min | PBS-T | PBS-T |

Tabelle 10a-d:

| Zusammenstellung der Kollektive mit Schnittmengen gemeinsamer Patienten.<br>NDC-$3^{KP}$ ist vollständig Teilkollektiv von NDC-3.<br>AD-$3^{KP}$ ist weitgehend Teilkollektiv von AD-3 | |
|---|---|
| **Tabelle 5a)** | **NDC1 (n=30)** |
| **NDC-$2^{KP}$ (n=10)** | Schnittmenge (n=2): NP55, NP57 |

Tabelle 10a-d: (fortgesetzt)

| Zusammenstellung der Kollektive mit Schnittmengen gemeinsamer Patienten.<br>NDC-$3^{KP}$ ist vollständig Teilkollektiv von NDC-3.<br>AD-$3^{KP}$ ist weitgehend Teilkollektiv von AD-3 | |
|---|---|
| **Tabelle 5a)** | **NDC1 (n=30)** |
| **Tabelle 5b)** | **NDC-3 (n=47)** |
| **NDC-$3^{KP}$ (n=15; Teilmenge** von **NDC-3)** | Schnittmenge (n=15):<br>NP213, NP344, NP345,<br>NP352, NP355, NP356,<br>NP364, NP374, NP402,<br>NP412, NP419, NP421,<br>NP457, NP490, NP 526 |
| **Tabelle 5c)** | **AD-1 (n=35)** |
| **AD-3 (n=12)** | Schnittmenge (n=3): NP37, NP52, NP66 |
| **AD-$3^{KP}$ (n=11)** | Schnittmenge (n=3): NP52, NP66, NP69 |
| **Tabelle 5d)** | **AD-3 (n=12)** |
| **AD-$3^{KP}$ (n=11; Schnittmenge plus NP69/NP197)** | Schnittmenge (n=9):<br>NP45, NP52, NP58, NP66,<br>NP111, NP143, NP190,<br>NP319, NP320 |

**Tabelle 11:** Aβ-SDS-PAGE/Immunoblot-1 von synthetischen Aβ-Peptiden nach direkter Aufnahme der Proben in SDS-PAGE-Probenpuffer und ECL-Detektion mittels Filmbelichtung: Inter- und Intraassayvariationskoeffizienten

| Aβ-Peptide | Interassay-VK* | Intraassay-VK* |
|---|---|---|
| $A\beta_{1-40}$ (100 pg) | 8,5 | 4,8 |
| $A\beta_{1-40}$ (75 pg) | 8,7 | 6,1 |
| $A\beta_{1-40}$ (50 pg) | 10,8 | 5 |
| $A\beta_{1-40}$ (20 pg) | 15,4 | 8,8 |
| $A\beta_{1-42}$ (25 pg) | 10,9 | 11,1 |
| $A\beta_{1-42}$ (15 pg) | 11,7 | 16,5 |
| $A\beta_{1-42}$ (10 pg) | 19,6 | 16,4 |
| $A\beta_{1-42}$ (5 pg) | 15,1 | 22,4 |
| $A\beta_{1-42}/A\beta_{1-40}$ (25pg/100pg) | 6,9 | 7,5 |
| $A\beta_{1-42}/A\beta_{1-40}$ (15pg/75pg) | 11 | 12,5 |
| $A\beta_{1-42}/A\beta_{1-40}$ (10pg/50pg) | 15,6 | 14,3 |
| $A\beta_{1-42}/A\beta_{1-40}$ (5pg/25pg) | 16,7 | 19,1 |

* VK: Variationskoeffizient (MW/SDx100; %); Ausreißer wurden nicht eliminiert.

**Tabelle 12:** Aβ-SDS-PAGE/Immunoblot-1 von Aβ-Peptiden im humanen Liquor bei NDC-1 und AD-1

| Diagnose | P-CODE | D-Code | Alter | Geschl. | $A\beta_{1-40}$ (ng/ml) | $A\beta_{1-42}$ (pg/ml) | $A\beta_{1-42}$ IP§ (pg/ml) | $A\beta_{1-42}/A\beta_{1-40}$* | $A\beta_{1-42}/A\beta_{1-38}$* |
|---|---|---|---|---|---|---|---|---|---|
| Tonisch-klonischer epileptischer Anfall | NP6 | NDC | 49 | weibl. | 10,24 | 1916,3 | 499,6 | 0,2895 | 0,4937 |
| Tonisch-klonischer epileptischer Anfall | NP7 | NDC | 77 | weibl. | 12,97 | 1203,3 | 268,3 | 0,1863 | 0,295 |
| Tonisch-klonischer epileptischer Anfall | NP9 | NDC | 71 | männl. | 14,71 | 2744,2 | 815,7 | 0,3408 | 0,5239 |
| Entzündl. ZNS-Prozeß unkl. Genese | NP10 | NDC | 72 | männl. | 13,21 | 2658,3 | 598,7 | 0,2666 | 0,42 |
| Hirnstamm-Ischämie | NP12 | NDC | 79 | männl. | 12,61 | 2579,2 | 504,2 | 0,2567 | 0,4065 |
| Zerebrale Ischämie | NP13 | NDC | 69 | weibl. | 14,38 | 1970,4 | 312,8 | 0,1506 | 0,2355 |
| Neuropathie unkl. Genese | NP17 | NDC | 61 | weibl. | 6,61 | 2009,2 | 669,6 | 0,2569 | 0,4919 |
| Depression | NP21 | NDC | 58 | weibl. | 11 | 3217,5 | 738,3 | 0,4164 | 0,7197 |
| Chlamydien-assoziierte Vaskulitis | NP22 | NDC | 56 | weibl. | 8,01 | 1962,9 | 350,2 | 0,2765 | 0,5004 |
| Depression mit psychotischenMerkmalen | NP25 | NDC | 67 | weibl. | 8,61 | 1844,2 | 392,1 | 0,2603 | 0,5099 |
| Entzündl. ZNS-Prozeß unkl. Genese | T17 | NDC | 37 | weibl. | 3,38 | 802,5 | | 0,1023 | 0,2575 |
| Akute organische Psychose | T27 | NDC | 42 | männl. | 2,93 | 1237,1 | | 0,109 | 0,3375 |
| Katatone Schizophrenie | T28 | NDC | 42 | weibl | 2,35 | 979,2 | | 0,1041 | 0,3071 |
| vaskuläre Demenz | T29 | NDC | 62 | männl. | 6,32 | 2631,3 | | 0,2358 | 0,4547 |
| Zerebrale Ischämie | T38, NP55 | NDC | 59 | weibl. | 7,28 | 3350,8 | | 0,2498 | 0,4924 |
| Katatones Syndrom | T40, NP57 | NDC | 59 | weibl. | 3,19 | 1356,3 | | 0,1287 | 0,3424 |
| Parkinson-Demenz-Komplex | A59 | NDC | 78 | weibl. | 10,38 | 734,2 | 230,4 | 0,0861 | 0,1448 |
| Parkinson-Demenz-Komplex | A185 | NDC | 74 | männl. | 5,5 | 1007,1 | 376,8 | 0,2123 | 0,4624 |
| Pseudodemenz bei Depression | A209 | NDC | 36 | weibl. | 9,01 | 1475,8 | 619,5 | 0,2501 | 0,4627 |
| Demenz bei subcortikaler Enzephalopathie | A266 | NDC | 68 | männl. | 7,48 | 689,6 | 222,5 | 0,076 | 0,1649 |
| Parkinson-Demenz-Komplex | A340 | NDC | 75 | weibl. | 10,32 | 1645,4 | 387 | 0,1606 | 0,2753 |
| Parkinson-Demenz-Komplex | A368 | NDC | 67 | männl. | 8,38 | 967,9 | 364,3 | 0,1114 | 0,209 |
| Pseudodemenz bei Depression | A458 | NDC | 67 | weibl. | 4,9 | 1329,8 | 382,1 | 0,1428 | 0,3644 |
| Parkinson-Demenz-Komplex | A473 | NDC | 44 | weibl. | 5,6 | 1590,8 | 431 | 0,1833 | 0,4918 |
| Parkinson-Demenz-Komplex | A546 | NDC | 77 | weibl. | 5,1 | 1602,9 | 518,9 | 0,1919 | 0,4068 |
| Parkinson-Demenz-Komplex | A582 | NDC | 77 | weibl. | 10,79 | 2918,3 | 490 | 0,3774 | 0,6543 |
| vaskuläre Demenz | T5 | NDC | 97 | männl. | 5,09 | 1040,6 | | 0,1331 | |
| vaskuläre Demenz | T13 | NDC | 73 | männl. | 4,85 | 546,7 | | 0,0697 | 0,1237 |
| Demenz bei Pick-Krankheit | T23 | NDC | 53 | männl. | 2,55 | 1064,2 | | 0,1112 | 0,3601 |
| vaskuläre Demenz | T24 | NDC | 61 | weibl. | 4,44 | 1248,8 | | 0,1065 | 0,2668 |
| Alzheimer-Demenz | A72 | AD | 60 | weibl. | 2,69 | 620 | 120,3 | 0,1301 | 0,4299 |
| Alzheimer-Demenz | A130 | AD | 75 | weibl. | 6,02 | 611,7 | 131,4 | 0,0827 | 0,1799 |
| Alzheimer-Demenz | A139 | AD | 53 | weibl. | 6,99 | 668,7 | 206,3 | 0,1204 | 0,2879 |
| Alzheimer-Demenz | A193 | AD | 71 | weibl. | 2,44 | 274,6 | 338,2 | 0,0427 | 0,2047 |
| Alzheimer-Demenz | A257 | AD | 67 | männl. | 6,88 | 615,4 | 298,8 | 0,0685 | 0,1535 |
| Alzheimer-Demenz | A279 | AD | 56 | weibl. | 9,42 | 1868,8 | 363,5 | 0,1899 | 0,3691 |
| Alzheimer-Demenz | A291 | AD | 81 | weibl. | 3,55 | 411,9 | 530,5 | 0,047 | 0,2108 |
| Alzheimer-Demenz | A391 | AD | 76 | männl. | 8,65 | 654,6 | 559,1 | 0,0612 | 0,1525 |
| Alzheimer-Demenz | A454 | AD | 59 | männl. | 11,87 | 706,3 | 287,6 | 0,123 | 0,2307 |
| Alzheimer-Demenz | A477 | AD | 65 | weibl. | 27,94 | 1357,9 | 373 | 0,1784 | 0,3132 |
| Alzheimer-Demenz | T1 | AD | 70 | männl. | 5,21 | 536,7 | | 0,0628 | 0,1145 |
| Alzheimer-Demenz | T2 | AD | 70 | männl. | 5,99 | 1108,8 | | 0,0868 | 0,162 |
| Alzheimer-Demenz | T3 | AD | 72 | weibl. | 4,53 | 440 | | 0,0639 | 0,1542 |
| Alzheimer-Demenz | T4 | AD | 68 | weibl. | 5,97 | 925,8 | | 0,1153 | 0,2101 |
| Alzheimer-Demenz | T6 | AD | 86 | weibl. | 5,56 | 498,1 | | 0,061 | |
| Alzheimer-Demenz | T7 | AD | 77 | männl. | 3,91 | 319,2 | | 0,0361 | 0,0934 |
| Alzheimer-Demenz | T8 | AD | 76 | männl. | 5,22 | 607,1 | | 0,0807 | 0,1438 |
| Alzheimer-Demenz | T9 | AD | 83 | männl. | 8,35 | 1844,2 | | 0,192 | 0,3362 |
| Alzheimer-Demenz | T10 | AD | 65 | männl. | 2,34 | 419,2 | | 0,0746 | 0,1616 |
| Alzheimer-Demenz | T11 | AD | 61 | weibl. | 4,85 | 481,3 | | 0,0555 | 0,1032 |
| Alzheimer-Demenz | T12 | AD | 76 | weibl. | 4,57 | 356,3 | | 0,0535 | 0,1038 |
| Alzheimer-Demenz | T14 | AD | 71 | männl. | 2,67 | 377,5 | | 0,0656 | 0,2175 |
| Alzheimer-Demenz | T15 | AD | 63 | männl. | 3,75 | 290 | | 0,0509 | 0,1134 |
| Alzheimer-Demenz | T16 | AD | 85 | weibl. | 4,21 | 515,8 | | 0,0647 | 0,139 |
| Alzheimer-Demenz | T18 | AD | 66 | weibl. | 6,56 | 542,9 | | 0,0577 | 0,1007 |
| Alzheimer-Demenz | T19 | AD | 76 | männl. | 4,03 | 586,9 | | 0,0465 | |
| Alzheimer-Demenz | T20 | AD | 82 | weibl. | 5,43 | 1628,3 | | 0,1186 | 0,2001 |
| Alzheimer-Demenz | T21 | AD | 59 | weibl. | 4,67 | 1036,3 | | 0,0774 | 0,1445 |
| Alzheimer-Demenz | T22 | AD | 58 | männl. | 2,33 | 780 | | 0,0897 | 0,1393 |
| Alzheimer-Demenz | T26 | AD | 78 | männl. | 4,43 | 735,4 | | 0,0509 | 0,1012 |
| Alzheimer-Demenz | T30, NP52 | AD | 68 | weibl. | 4,49 | 1065,8 | | 0,0773 | 0,1807 |
| Alzheimer-Demenz | T32, NP66 | AD | 66 | weibl. | 4,32 | 1206,7 | | 0,0809 | 0,2132 |
| Alzheimer-Demenz | T33, NP69 | AD | 65 | männl. | 1,93 | 475 | | 0,0553 | |
| Alzheimer-Demenz | T34, NP35 | AD | 75 | männl. | 3,77 | 300 | | 0,0253 | |
| Alzheimer-Demenz | T35, NP37 | AD | 70 | männl. | 3,58 | 378,8 | | 0,0222 | 0,0624 |

§ Aβ-SDS-PAGE/Immunoblot nach Immunopräzipitation * Quotient der Flächeneinheiten

**Tabelle 13:** Aβ-SDS-PAGE/Immunoblot-1 von Aβ-Peptiden im humanen lumbalen Liquor bei NDC-1 und AD-1 nach direkter Aufnahme der Proben in SDS-PAGE-Probenpuffer oder nach vorheriger Immunopräzipitation (Teilkollektiv). Zusätzlich wurde die $A\beta_{1-42}$ Konzentration im Liquor bei AD-1 mittels eines kommerziellen $ELISA_{A\beta 1-42}$ ermittelt; Statistische Kennwerte: gültiges N, Mittelwert, 95%-Konfidenzintervall, Median, Unteres-Oberes Quartil, Standardabweichung

| | Kollektiv | Gült. N | Mittelw. | Konfid. -95% | Konfid. +95% | Median | Unteres Quartil | Oberes Quartil | Stdabw. |
|---|---|---|---|---|---|---|---|---|---|
| ALTER | AD | 35 | 69,7 | 66,7 | 72,7 | 68,0 | 64,7 | 76,0 | 8,7 |
| | NDC | 30 | 63,5 | 58,2 | 68,9 | 66,9 | 56,3 | 74,4 | 14,2 |
| $A\beta_{1-40}$ (ng/ml) | AD | 35 | 5,6 | 4,1 | 7,1 | 4,6 | 3,7 | 6,0 | 4,4 |
| | NDC | 30 | 7,6 | 6,2 | 9,0 | 6,6 | 4,9 | 10,4 | 3,7 |
| $A\beta_{1-42}$ (pg/ml) | AD | 35 | 721,2 | 575,0 | 867,5 | 607,1 | 419,2 | 925,8 | 425,7 |
| | NDC | 30 | 1677,5 | 1384,0 | 1970,9 | 1533,3 | 1040,6 | 2009,2 | 785,9 |
| $A\beta_{1-42}$,IP, (pg/ml)$ | AD | 10 | 320,7 | 215,0 | 426,4 | 317,4 | 206,3 | 373,0 | 147,7 |
| | NDC | 20 | 457,5 | 380,4 | 534,6 | 411,6 | 356,1 | 557,8 | 164,8 |
| $A\beta_{1-42}$ ELISA[1] (pg/ml) | AD | 35 | 412,4 | 361,1 | 463,7 | 371,0 | 316,0 | 445,0 | 149,3 |
| $A\beta_{1-37}$ (FE*) | AD | 35 | 1031,8 | 794,0 | 1269,6 | 840,3 | 660,0 | 1243,3 | 692,3 |
| | NDC | 30 | 1517,8 | 1173,1 | 1862,5 | 1299,7 | 759,7 | 2375,0 | 923,1 |
| $A\beta_{1-38}$ (FE*) | AD | 35 | 3975,3 | 3445,1 | 4505,5 | 3931,7 | 2977,3 | 4907,7 | 1543,5 |
| | NDC | 30 | 5123,3 | 4420,2 | 5826,3 | 5035,3 | 3421,7 | 6658,3 | 1882,8 |
| $A\beta_{1-39}$ (FE*) | AD | 35 | 942,8 | 749,5 | 1136,2 | 923,0 | 545,7 | 1186,7 | 562,7 |
| | NDC | 30 | 1389,1 | 1101,9 | 1676,2 | 1178,7 | 726,0 | 2115,0 | 769,0 |
| $A\beta_{1-40}$ (FE*) | AD | 35 | 8499,1 | 7841,0 | 9157,3 | 8293,0 | 7545,5 | 9335,0 | 1916,0 |
| | NDC | 30 | 9748,3 | 9007,0 | 10489,5 | 9689,8 | 8556,0 | 11384,0 | 1985,2 |
| $A\beta_{1-42}$ (FE*) | AD | 35 | 732,7 | 539,7 | 925,6 | 563,3 | 416,3 | 811,7 | 561,6 |
| | NDC | 30 | 2003,1 | 1556,7 | 2449,5 | 1864,2 | 908,7 | 2593,7 | 1195,5 |
| $A\beta_{42}/A\beta_{40}$** | AD | 35 | 0,0803 | 0,0657 | 0,0948 | 0,0656 | 0,0535 | 0,0897 | 0,0424 |
| | NDC | 30 | 0,1947 | 0,1603 | 0,2291 | 0,1848 | 0,1112 | 0,2569 | 0,0922 |
| $A\beta_{42}/A\beta_{38}$** | AD | 31 | 0,1847 | 0,1532 | 0,2163 | 0,1616 | 0,1145 | 0,2132 | 0,0861 |
| | NDC | 29 | 0,3853 | 0,3302 | 0,4404 | 0,4065 | 0,2753 | 0,4919 | 0,1448 |

$ Aβ-SDS-PAGE/Immunoblot nach Immunopräzipitation (mAb 6E10)   * Flächeneinheiten

** Quotient der Flächeneinheiten   [1] Hulstaert et al., 1999, Neurology 52: 1555-62.

**Tabelle 14:** Vergleich der Patientenkollektive AD-1 und NDC-1 mittels Mann-Whitney U-Test

| | AD-1 (n) | NDC-1 (n) | p-Niveau |
|---|---|---|---|
| ALTER | 35 | 30 | 0,1098 |
| $A\beta_{1-40}$ | 35 | 30 | 0,0055 |
| $A\beta_{1-42}$ | 35 | 30 | 1,23E-07 |
| $A\beta_{1-42}$ $IP^{\$}$ | 10 | 20 | 0,0387 |
| $A\beta_{42}/A\beta_{40}$* | 35 | 30 | 4,77E-08 |
| $A\beta_{42}/A\beta_{38}$* | 31 | 29 | 3,49E-07 |

$ Aβ-SDS-PAGE/Immunoblot nach Immunopräzipitation (mAb 6E10)
* Quotient der Flächeneinheiten

**Tabelle 15:** Synopsis der diagnostischen Kennwerte (Spezifität, Sensitivität, max. Youden-Index, Grenzwertkonzentrationen) für $A\beta_{1-42}$ und zugehörige Aβ-Peptidquotienten für die Differenzierung der Patientenkollektive AD-1 und NDC-1.

| | Grenzwert | Spezifität | Sensitivität | maximaler Youden-Index |
|---|---|---|---|---|
| $A\beta_{1-42}$ (pg/ml) | 802.5 | 0.74 | 0.87 | 0.61 |
| $A\beta_{42}/A\beta_{40}$* | 0.086 | 0.71 | 0.93 | 0.65 |
| $A\beta_{42}/A\beta_{38}$* | 0.231 | 0.84 | 0.86 | 0.70 |

* Quotient der Flächeneinheiten

**Tabelle 16a:** Aβ-SDS-PAGE/Immunoblot-1 von $A\beta_{1-42}$ und $A\beta_{1-40}$ im Liquor, Kollektiv NDC-2[KP]: Vergleich der Kältepräzipitation nach Einfrieren unbehandelter Liquorproben (nativ*) versus Vorbehandlung mit SDS-/Hitzedenaturierung (SDS**).

| Patienten | Diagnose | $A\beta_{1-42}$SDS** pg/ml | $A\beta_{1-40}$SDS** pg/ml | $A\beta_{42}/A\beta_{40}$SDS** | $A\beta_{1-42}$nativ* pg/ml | $A\beta_{1-40}$nativ* pg/ml | $A\beta_{42}/A\beta_{40}$nativ* |
|---|---|---|---|---|---|---|---|
| NP51 | Angsterkrankung | 1543,0 | 7830,0 | 0,1971 | 856,3 | 7473,0 | 0,1146 |
| NP53 | Epilepsie | 1888,0 | 7869,0 | 0,2399 | 1348,3 | 7714,0 | 0,1748 |
| NP47 | Horner-Syndrom | 1588,7 | 7753,3 | 0,2049 | 991,7 | 7156,7 | 0,1386 |
| NP48 | Depression | 1926,3 | 7911,3 | 0,2435 | 1128,0 | 6821,7 | 0,1654 |
| NP54 | Discusprolaps (BWS) | 1387,3 | 5257,0 | 0,2639 | 1277,0 | 5032,7 | 0,2537 |
| NP55 | Zerebrale Ischämie | 2578,0 | 7985,0 | 0,3229 | 1707,0 | 8103,7 | 0,2106 |
| NP56 | Depression | 2183,3 | | | 1437,7 | | |
| NP57 | Katatones Syndrom | 1477,7 | 4292,0 | 0,3443 | 1261,7 | 4744,3 | 0,2659 |
| NP59 | primär progrediente Demenz ungekl. Genese | 2285,3 | 8377,7 | 0,2728 | 1734,7 | 7942,0 | 0,2184 |
| NP60 | Neurosarkoidose | 965,7 | 6818,0 | 0,1416 | 653,0 | 6411,7 | 0,1018 |
| **MW** | | **1782,3** | **7121,5** | **0,2479** | **1239,5** | **6822,2** | **0,1827** |
| SD | | 457,1 | 1332,6 | 0,0592 | 328,8 | 1150,6 | 0,0553 |
| VK | | 25,6 | 18,7 | 23,9 | 26,5 | 16,9 | 30,3 |

**Tabelle 16b:** Absolute und prozentuale kältepräzipitationsbedingte Erniedrigung von $A\beta_{1-40}$, $A\beta_{1-42}$ und des Aβ-Peptidquotienten für das Kollektiv NDC-2[KP] (Differenzwerttabelle zu Tabelle 10a)

| Patienten | Diagnose | $\Delta A\beta_{1-42}$ | $\Delta A\beta_{1-42}$%* | $\Delta A\beta_{1-40}$ | $\Delta A\beta_{1-40}$%* | $\Delta A\beta_{42}/A\beta_{40}$ | $\Delta A\beta_{42}/A\beta_{40}$%* |
|---|---|---|---|---|---|---|---|
| NP51 | Angsterkrankung | -686,7 | -44,5 | -357,0 | -4,6 | -0,0825 | -41,9 |
| NP53 | Epilepsie | -539,7 | -28,6 | -155,0 | -2,0 | -0,0651 | -27,1 |
| NP47 | Horner-Syndrom | -597,0 | -37,6 | -596,7 | -7,7 | -0,0663 | -32,4 |
| NP48 | Depression | -798,3 | -41,4 | -1089,7 | -13,8 | -0,0781 | -32,1 |
| NP54 | Discusprolaps (BWS) | -110,3 | -8,0 | -224,3 | -4,3 | -0,0102 | -3,8 |
| NP55 | Zerebrale Ischämie | -871,0 | -33,8 | 118,7 | 1,5 | -0,1122 | -34,8 |
| NP56 | Depression | -745,7 | -34,2 | | | | |
| NP57 | Katatones Syndrom | -216,0 | -14,6 | 452,3 | 10,5 | -0,0784 | -22,8 |
| NP59 | primär progrediente Demenz ungekl. Genese | -550,7 | -24,1 | -435,7 | -5,2 | -0,0544 | -19,9 |
| NP60 | Neurosarkoidose | -312,7 | -32,4 | -406,3 | -6,0 | -0,0398 | -28,1 |
| **MW** | | **-542,8** | **-29,9** | **-299,3** | **-3,5** | **-0,0652** | **-27,0** |
| SD | | 241,8 | 10,9 | 410,0 | 6,3 | 0,0272 | 10,2 |
| VK | | 44,5 | 36,6 | 137,0 | 180,8 | 41,7 | 37,9 |

* $\Delta A\beta_{Peptid}\% = (A\beta_{Peptid}Nativ - A\beta_{Peptid}SDS) / A\beta_{Peptid}SDS * 100$:
Negatives (positives) $\Delta A\beta_{1-42}$ oder $\Delta A\beta_{1-40}$ bedeutet niedrigere (höhere) Aβ-Peptidkonzentration der nativ eingefrorenen Liquorprobe bezogen auf Vorbehandlung der Probe mit SDS-/Hitzedenaturierung

**Tabelle 17:** Das Signifikanzniveau der kältepräzipitationsbedingten Erniedrigung der Aβ-Peptide wurde für das Kollektiv NDC-2[KP] mittels des Wilcoxon Test für gepaarte Proben ermittelt (vergl. Tabelle 10a):

| $A\beta_{1-42}$SDS versus $A\beta_{1-42}$Nativ | $A\beta_{1-40}$SDS versus $A\beta_{1-40}$Nativ | $A\beta_{42}/A\beta_{40}$SDS versus $A\beta_{42}/A\beta_{40}$Nativ | |
|---|---|---|---|
| 10 | 9 | 9 | n |
| 0,0051 | 0,0858 | 0,0077 | p-Niveau |

**Tabelle 18a:** Intraassay-Variationskoeffizient bei Aβ-SDS-PAGE/Immunoblot-2 unter Verwendung von Rotiblock und ECL-Detektion mittels CCD-Kamera. Die Aβ-Peptide wurden pro Western-Immunoblot (M1-M4) jeweils vierfach aufgetragen.

| | 80pg | | | | | 20pg | | | | |
|---|---|---|---|---|---|---|---|---|---|---|
| | $A\beta_{1-38}$ | $A\beta_{1-40}$ | $A\beta_{1-42}$ | $A\beta_{42}/A\beta_{40}$ | $A\beta_{42}/A\beta_{38}$ | $A\beta_{1-38}$ | $A\beta_{1-40}$ | $A\beta_{1-42}$ | $A\beta_{42}/A\beta_{40}$ | $A\beta_{42}/A\beta_{38}$ |
| Intraassay-VK M1 | 5,47 | 4,47 | 4,28 | 2,32 | 2,14 | 5,34 | 5,92 | 3,15 | 6,22 | 7,48 |
| Intraassay-VK M2 | 6,19 | 4,05 | 5,90 | 4,02 | 4,68 | 4,41 | 8,88 | 8,97 | 3,67 | 7,92 |
| Intraassay-VK M3 | 5,75 | 5,39 | 2,59 | 5,43 | 5,52 | 12,82 | 4,12 | 1,27 | 3,01 | 12,21 |
| Intraassay-VK M4 | 6,16 | 6,79 | 2,87 | 4,28 | 4,53 | 2,20 | 4,90 | 4,86 | 2,63 | 5,27 |
| **Mean Intraassay-VK** | **5,89** | **5,18** | **3,91** | **4,01** | **4,22** | **6,19** | **5,95** | **4,56** | **3,88** | **8,22** |
| SD Intraassay-VK | 0,30 | 1,05 | 1,32 | 1,11 | 1,26 | 3,99 | 1,80 | 2,84 | 1,40 | 2,51 |

**Tabelle 18b:** Interassay-Variationskoeffizient bei Aβ-SDS-PAGE/Immunoblot-2 unter Verwendung von Rotiblock und ECL-Detektion mittels CCD-Kamera. Die Meßwerte beziehen sich auf die Volumendatensätze einer Bande nach Hintergrundkorrektur und entsprechen den Mittelwerten (MW) einer Vierfachbestimmung pro Western-Immunoblot (vergl. Tabelle xx). Die Western-Immunoblots (M1-M4) entsprechen unabhängig durchgeführten Elektrophoresen.

| | 80pg | | | | | 20pg | | | | |
|---|---|---|---|---|---|---|---|---|---|---|
| | $A\beta_{1-38}$ | $A\beta_{1-40}$ | $A\beta_{1-42}$ | $A\beta_{42}/A\beta_{40}$ | $A\beta_{42}/A\beta_{38}$ | $A\beta_{1-38}$ | $A\beta_{1-40}$ | $A\beta_{1-42}$ | $A\beta_{42}/A\beta_{40}$ | $A\beta_{42}/A\beta_{38}$ |
| MW M1 | 46452,20 | 52114,60 | 28568,20 | 0,55 | 0,62 | 6817,50 | 7106,25 | 2963,50 | 0,42 | 0,44 |
| MW M2 | 46415,80 | 51925,00 | 27217,20 | 0,52 | 0,59 | 6622,50 | 6774,00 | 3253,75 | 0,48 | 0,49 |
| MW M3 | 51619,25 | 55606,75 | 30266,50 | 0,55 | 0,59 | 7430,75 | 7544,25 | 3028,00 | 0,40 | 0,41 |
| MW M4 | 45169,00 | 50431,50 | 29304,00 | 0,58 | 0,65 | 7511,00 | 8251,60 | 3767,60 | 0,46 | 0,50 |
| Total Mean | 47414,06 | 52519,46 | 28838,98 | 0,55 | 0,61 | 7095,44 | 7419,03 | 3253,21 | 0,44 | 0,46 |
| SD | 2482,22 | 1897,91 | 1113,28 | 0,02 | 0,03 | 382,77 | 552,89 | 315,93 | 0,03 | 0,04 |
| **Interassay-VK** | **5,24** | **3,61** | **3,86** | **3,81** | **4,23** | **5,39** | **7,45** | **9,71** | **7,09** | **8,02** |

**Tabelle 19:** Aβ-SDS-PAGE/Immunoblot-2 von Aβ-Peptiden im humanen Liquor bei NDC-3 und AD-3

| P-Code | Alter | Geschlecht | Diagnose | D-Code | MMSE[1] | $A\beta_{1-42}$ ELISA[2] (ng/ml) | $A\beta_{1-37}$ (ng/ml) | $A\beta_{1-38}$ (ng/ml) | $A\beta_{1-39}$ (ng/ml) | $A\beta_{1-40}$ (ng/ml) | $A\beta_{1-42}$ (ng/ml) | total Aβ[3] (ng/ml) | $A\beta_{1-37}$[4] (%) | $A\beta_{1-38}$[4] (%) | $A\beta_{1-39}$[4] (%) | $A\beta_{1-40}$[4] (%) | $A\beta_{1-42}$[4] (%) | $R_{42/40}$[5] | $R_{42/38}$[5] | $R_{38/40}$[5] | ApoE[6] |
|---|---|---|---|---|---|---|---|---|---|---|---|---|---|---|---|---|---|---|---|---|---|
| NP207 | 76 | M | Polyneuropathie | NDC (OND) | 29 | | 1,20 | 2,66 | 1,44 | 11,38 | 1,95 | 18,66 | 6,44 | 14,37 | 7,71 | 61,01 | 10,47 | 0,1717 | 0,7290 | 0,2366 | 3/3 |
| NP208 | 65 | F | Meningoenzephalitis | NDC (OND) | 30 | | 1,68 | 3,37 | 1,48 | 13,44 | 2,14 | 22,11 | 7,61 | 15,28 | 6,71 | 60,78 | 9,66 | 0,1590 | 0,6332 | 0,2511 | 3/3 |
| NP213 | 32 | M | Depressive Störung mit psychotischen Merkmalen | NDC (OND) | 30 | 0,72 | 1,23 | 2,66 | 1,33 | 10,72 | 2,07 | 18,00 | 6,82 | 14,77 | 7,38 | 59,54 | 11,48 | 0,1928 | 0,7772 | 0,2481 | 3/3 |
| NP230 | 38 | F | Epilepsie | NDC (OND) | 30 | | 1,06 | 2,36 | 1,10 | 8,18 | 1,44 | 14,16 | 7,52 | 16,64 | 7,79 | 57,78 | 10,19 | 0,1764 | 0,6124 | 0,2881 | 3/3 |
| NP231 | 42 | M | Epilepsie | NDC (OND) | 30 | | 1,34 | 3,04 | 1,46 | 12,19 | 2,21 | 20,24 | 6,62 | 15,02 | 7,20 | 60,23 | 10,93 | 0,1815 | 0,7277 | 0,2495 | 3/3 |
| NP234 | 28 | F | Embolische transitorisch ischämische Hirnstammattacke | NDC (OND) | 30 | | 1,13 | 2,42 | 1,15 | 9,43 | 1,38 | 15,52 | 7,30 | 15,62 | 7,43 | 60,73 | 8,93 | 0,1471 | 0,5720 | 0,2571 | 3/3 |
| NP235 | 19 | M | Psychotische Störung (nicht spezifiziert) und leichte kognitive Störung | NDC (OND) | 28 | | 1,64 | 3,28 | 1,77 | 14,24 | 4,12 | 25,04 | 6,56 | 13,10 | 7,05 | 56,85 | 16,43 | 0,2891 | 1,2543 | 0,2305 | 2/3 |
| NP237 | 78 | F | Subkortikale arteriosklerotische Enzephalopathie | NDC (OND) | 30 | | 1,22 | 2,47 | 1,10 | 8,73 | 1,29 | 14,82 | 8,21 | 16,70 | 7,43 | 58,94 | 8,72 | 0,1480 | 0,5224 | 0,2833 | 3/4 |
| NP243 | 47 | F | Depressive Störung | NDC (OND) | 29 | | 1,21 | 2,53 | 1,13 | 9,28 | 1,77 | 15,92 | 7,61 | 15,88 | 7,10 | 58,31 | 11,10 | 0,1903 | 0,6988 | 0,2724 | 3/3 |
| NP271 | 31 | M | Morbus Meniere | NDC (OND) | 30 | | 1,60 | 3,49 | 1,48 | 12,82 | 2,46 | 21,84 | 7,33 | 15,97 | 6,78 | 58,68 | 11,25 | 0,1916 | 0,7041 | 0,2722 | 3/3 |
| NP272 | 52 | M | Gutartiger paroxysmaler Lagerungsschwindel | NDC (OND) | 30 | | 1,23 | 2,71 | 1,11 | 10,53 | 1,97 | 17,55 | 7,00 | 15,44 | 6,33 | 60,03 | 11,20 | 0,1866 | 0,7252 | 0,2573 | 3/3 |
| NP274 | 59 | M | Multiple Sklerose | NDC (CID) | 27 | | 1,02 | 2,14 | 0,96 | 7,29 | 1,25 | 12,66 | 8,04 | 16,89 | 7,57 | 57,63 | 9,87 | 0,1713 | 0,5846 | 0,2931 | 3/4 |
| NP120 | 30 | M | Generalisierte Angsterkrankung | NDC (OND) | 30 | 1,17 | 2,29 | 4,58 | 2,23 | 19,36 | 4,26 | 32,72 | 7,00 | 13,94 | 6,83 | 59,22 | 13,01 | 0,2197 | 0,9336 | 0,2364 | 3/3 |
| NP121 | 37 | M | Benzodiazepinabhängigkeit | NDC (OND) | 30 | 0,81 | 1,12 | 2,40 | 1,09 | 10,47 | 1,97 | 17,05 | 6,59 | 14,07 | 6,42 | 61,38 | 11,55 | 0,1881 | 0,8208 | 0,2292 | NA |
| NP123 | 61 | F | Subkortikale arteriosklerotische Enzephalopathie | NDC (OND) | 30 | 0,91 | 1,58 | 3,57 | 1,66 | 14,54 | 3,06 | 24,45 | 6,47 | 14,58 | 6,66 | 59,48 | 12,60 | 0,2119 | 0,8642 | 0,2452 | 3/3 |
| NP238 | 54 | M | Schweres Schädel-Hirn-Trauma mit Hydrozephalus und posttraumatischer Epilepsie | NDC (OND) | 25 | | 0,85 | 1,77 | 0,96 | 7,82 | 1,44 | 12,83 | 6,60 | 13,82 | 7,45 | 60,93 | 11,20 | 0,1836 | 0,8108 | 0,2267 | 2/3 |
| NP245 | 38 | M | Spannungskopfschmerz | NDC (OND) | 30 | | 0,99 | 2,23 | 1,00 | 10,20 | 1,81 | 16,22 | 6,11 | 13,73 | 6,14 | 62,87 | 11,15 | 0,1773 | 0,8120 | 0,2184 | 3/3 |
| NP288 | 63 | M | chronisch entzündliche ZNS-Erkrankung | NDC (CID) | 30 | | 0,77 | 1,56 | 0,74 | 5,18 | 1,00 | 9,26 | 8,36 | 16,88 | 8,03 | 55,94 | 10,78 | 0,1928 | 0,6388 | 0,3018 | 3/3 |
| NP276 | 38 | M | Subkortikale arteriosklerotische Enzephalopathie | NDC (OND) | 30 | | 1,34 | 2,93 | 1,48 | 11,67 | 1,97 | 19,60 | 6,85 | 14,96 | 7,58 | 60,56 | 10,06 | 0,1690 | 0,6718 | 0,2471 | 2/4 |
| NP278 | 56 | M | Posttraumatische Epilepsie | NDC (OND) | 24 | | 2,48 | 5,28 | 2,76 | 20,02 | 4,85 | 35,34 | 6,97 | 14,87 | 7,81 | 56,64 | 13,71 | 0,2421 | 0,9218 | 0,2626 | 3/3 |
| NP101 | 67 | M | Depressive Störung und leichte kognitive Störung | NDC (OND) | 30 | | 1,29 | 2,91 | 1,46 | 13,09 | 2,22 | 20,97 | 6,17 | 13,90 | 6,98 | 62,40 | 10,58 | 0,1696 | 0,7610 | 0,2227 | 3/3 |
| NP110 | 39 | M | Depressive Störung | NDC (OND) | 30 | 0,94 | 1,45 | 3,11 | 1,78 | 13,10 | 2,49 | 21,92 | 6,62 | 14,20 | 8,03 | 59,79 | 11,36 | 0,1901 | 0,8002 | 0,2375 | 3/3 |
| NP119 | 45 | F | Depressive Störung | NDC (OND) | NA | 1,27 | 2,24 | 5,12 | 2,99 | 20,93 | 4,62 | 35,90 | 6,23 | 14,27 | 8,34 | 58,30 | 12,87 | 0,2208 | 0,9017 | 0,2448 | 2/3 |
| NP290 | 19 | M | Manische Episode mit psychotischen Merkmalen bei bipolar affektiver Psychose,Typ I | NDC (OND) | 30 | | 1,26 | 2,71 | 1,27 | 10,53 | 1,81 | 17,57 | 7,18 | 15,40 | 7,22 | 59,93 | 10,28 | 0,1715 | 0,6673 | 0,2570 | 3/3 |
| NP292 | 70 | F | Hirnmetastasierung | NDC (OND) | 27 | | 0,96 | 2,27 | 0,95 | 7,57 | 1,43 | 13,19 | 7,31 | 17,21 | 7,22 | 57,41 | 10,86 | 0,1891 | 0,6307 | 0,2996 | 3/3 |
| NP296 | 51 | M | Motor Neuron Erkrankung | NDC (OND) | 30 | | 2,31 | 5,51 | 2,46 | 19,46 | 4,58 | 34,32 | 6,72 | 16,06 | 7,17 | 56,71 | 13,33 | 0,2351 | 0,8303 | 0,2831 | 3/3 |
| NP297 | 48 | F | Multiple Sklerose | NDC (CID) | 30 | 0,83 | 1,33 | 3,33 | 1,46 | 12,96 | 2,08 | 21,16 | 6,30 | 15,75 | 6,88 | 61,26 | 9,82 | 0,1603 | 0,6234 | 0,2571 | 3/3 |
| NP300 | 31 | F | Sinusvenenthrombose mit rezidivierenden transitorisch ischämischen Attacken | NDC (OND) | 30 | 0,18 | 0,70 | 1,45 | 0,74 | 4,35 | 0,88 | 8,12 | 8,64 | 17,83 | 9,16 | 53,59 | 10,78 | 0,2012 | 0,6048 | 0,3326 | 3/4 |
| NP309 | 39 | F | Neurologische Erkrankung (nicht spezifiziert) ohne Demenz | NDC (OND) | 30 | 0,47 | 1,24 | 2,71 | 1,11 | 10,42 | 1,63 | 17,11 | 7,27 | 15,82 | 6,48 | 60,91 | 9,52 | 0,1564 | 0,6019 | 0,2598 | 2/3 |
| NP362 | 45 | F | chronisch entzündliche ZNS-Erkrankung | NDC (CID) | 29 | 0,67 | 2,51 | 5,92 | 2,63 | 19,38 | 4,59 | 35,02 | 7,17 | 16,89 | 7,50 | 55,34 | 13,10 | 0,2368 | 0,7759 | 0,3052 | 3/3 |
| NP426 | 17 | F | Schädel Trauma | NDC (OND) | NA | 0,43 | 1,16 | 2,47 | 1,11 | 9,24 | 1,33 | 15,30 | 7,58 | 16,15 | 7,23 | 60,38 | 8,66 | 0,1434 | 0,5362 | 0,2675 | 3/4 |
| NP330 | 58 | M | Motor Neuron Erkrankung | NDC (OND) | NA | 0,73 | 1,09 | 2,45 | 1,15 | 10,08 | 2,16 | 16,93 | 6,46 | 14,48 | 6,77 | 59,54 | 12,75 | 0,2142 | 0,8808 | 0,2432 | 3/3 |
| NP344 | 36 | F | Somatoforme (konversionsneurotische) Störung | NDC (OND) | 28 | 0,59 | 1,18 | 2,67 | 1,23 | 10,86 | 2,01 | 17,94 | 6,58 | 14,86 | 6,85 | 60,50 | 11,21 | 0,1853 | 0,7546 | 0,2457 | 3/3 |
| NP345 | 34 | M | Panikstörung mit Agoraphobie | NDC (OND) | NA | 0,68 | 1,60 | 3,59 | 1,67 | 15,11 | 2,88 | 24,85 | 6,43 | 14,44 | 6,72 | 60,82 | 11,59 | 0,1905 | 0,8023 | 0,2375 | 3/3 |
| NP366 | 31 | F | zerebrale transitorisch ischämische Attacke | NDC (OND) | 30 | 0,78 | 2,54 | 5,23 | 2,73 | 20,22 | 4,93 | 35,66 | 7,11 | 14,66 | 7,67 | 56,71 | 13,83 | 0,2439 | 0,9424 | 0,2589 | 3/3 |
| NP368 | 65 | F | Polyzythämia vera mit transitorisch ischämischen Attacken | NDC (OND) | 30 | 0,64 | 0,97 | 2,15 | 1,10 | 8,92 | 1,29 | 14,42 | 6,70 | 14,90 | 7,62 | 61,85 | 8,91 | 0,1441 | 0,5980 | 0,2410 | 3/4 |
| NP384 | 58 | M | Epilepsie | NDC (OND) | 28 | 0,79 | 1,40 | 3,08 | 1,59 | 13,28 | 2,45 | 21,80 | 6,41 | 14,13 | 7,31 | 60,91 | 11,24 | 0,1845 | 0,7961 | 0,2320 | 2/3 |
| NP374 | 43 | M | chronisch entzündliche ZNS-Erkrankung | NDC (CID) | 30 | 0,76 | 1,12 | 2,49 | 1,17 | 9,32 | 1,56 | 15,66 | 7,14 | 15,91 | 7,48 | 59,49 | 9,98 | 0,1677 | 0,6272 | 0,2674 | 3/4 |
| NP402 | 66 | F | Hemicranie | NDC (OND) | 30 | 0,51 | 1,15 | 2,78 | 1,28 | 11,62 | 1,71 | 18,55 | 6,20 | 15,00 | 6,90 | 62,66 | 9,24 | 0,1475 | 0,6161 | 0,2395 | 3/4 |
| NP412 | 48 | M | Depressive Störung bei subkortikaler arteriosklerotischer Enzephalopathie | NDC (OND) | 30 | 0,54 | 1,07 | 2,44 | 1,06 | 9,75 | 1,56 | 15,88 | 6,76 | 15,36 | 6,69 | 61,36 | 9,82 | 0,1601 | 0,6394 | 0,2504 | 3/3 |
| NP419 | 48 | M | chronisch entzündliche ZNS-Erkrankung | NDC (CID) | NA | 0,39 | 0,93 | 2,36 | 1,11 | 8,43 | 1,41 | 14,24 | 6,51 | 16,57 | 7,80 | 59,21 | 9,91 | 0,1674 | 0,5983 | 0,2798 | 3/3 |
| NP421 | 60 | M | chronisch entzündliche ZNS-Erkrankung mit primär progressiver Aphasie | NDC (CID) | 30 | 0,54 | 1,34 | 3,12 | 1,42 | 11,90 | 2,15 | 19,92 | 6,72 | 15,64 | 7,12 | 59,75 | 10,77 | 0,1803 | 0,6887 | 0,2618 | 3/3 |
| NP458 | 42 | F | chronisch entzündliche ZNS-Erkrankung | NDC (CID) | 27 | 0,6794 | 1,93 | 5,09 | 2,59 | 18,81 | 3,72 | 32,13 | 6,01 | 15,83 | 8,04 | 58,54 | 11,58 | 0,1978 | 0,7308 | 0,2708 | 4/4 |
| NP457 | 24 | M | Depressive Episode bei bipolar affektiver Psychose, Typ I | NDC (OND) | 30 | 0,7451 | 1,23 | 2,93 | 1,78 | 12,85 | 2,16 | 20,75 | 5,93 | 14,10 | 8,59 | 60,96 | 10,43 | 0,1712 | 0,7372 | 0,2316 | 2/3 |
| NP466 | 25 | F | chronisch entzündlicher ZNS-Prozeß | NDC (CID) | 30 | 0,7458 | 1,16 | 3,3 | 1,94 | 14,24 | 2,38 | 23,02 | 5,06 | 14,33 | 8,44 | 61,82 | 10,36 | 0,1674 | 0,7212 | 0,2317 | 3/3 |
| NP490 | 20 | M | chronisch entzündlicher ZNS-Prozeß | NDC (CID) | 30 | 0,7196 | 2,33 | 6,18 | 2,64 | 20,27 | 4,35 | 35,77 | 6,51 | 17,27 | 7,37 | 56,68 | 12,17 | 0,2146 | 0,7039 | 0,3049 | 3/3 |
| NP526 | 61 | M | Depressive Störung bei subkortikaler arteriosklerotischer Enzephalopathie | NDC (OND) | 29 | 0,82 | 1,62 | 4,53 | 2,28 | 18,40 | 3,40 | 30,23 | 5,36 | 14,99 | 7,56 | 60,87 | 11,24 | 0,1848 | 0,7506 | 0,2462 | 3/3 |
| NP37 | 71 | M | Alzheimer Demenz | AD | 17 | | 1,37 | 3,11 | 1,37 | 11,94 | 0,94 | 18,73 | 7,32 | 16,59 | 7,32 | 63,76 | 5,01 | 0,0786 | 0,3020 | 0,2602 | 44 |
| NP45 | 76 | F | Alzheimer Demenz | AD | 5 | | 1,04 | 2,40 | 1,06 | 11,55 | 1,46 | 17,50 | 5,93 | 13,71 | 5,99 | 66,00 | 8,36 | 0,1267 | 0,6100 | 0,2077 | 34 |
| NP52 | 68 | F | Alzheimer Demenz | AD | 11 | | 1,65 | 3,73 | 2,00 | 14,21 | 1,59 | 23,18 | 7,12 | 16,11 | 8,63 | 61,30 | 6,84 | 0,1118 | 0,4247 | 0,2629 | 34 |
| NP66 | 67 | F | Alzheimer Demenz | AD | 1 | | 1,22 | 3,06 | 1,34 | 13,06 | 1,50 | 20,17 | 6,05 | 15,17 | 6,65 | 64,69 | 7,43 | 0,1148 | 0,4897 | 0,2345 | 34 |
| NP112 | 77 | F | Alzheimer Demenz | AD | 14 | | 1,23 | 2,95 | 1,36 | 12,56 | 1,55 | 19,65 | 6,27 | 15,01 | 6,93 | 63,92 | 7,87 | 0,1231 | 0,5244 | 0,2347 | 33 |
| NP190 | 79 | F | Alzheimer Demenz | AD | 19 | | 0,80 | 1,77 | 0,85 | 6,40 | 0,66 | 10,48 | 7,64 | 16,86 | 8,15 | 61,07 | 6,27 | 0,1027 | 0,3720 | 0,2761 | 34 |
| NP143 | 55 | M | Alzheimer Demenz | AD | 27 | | 0,86 | 1,75 | 0,79 | 6,38 | 0,89 | 10,67 | 8,09 | 16,42 | 7,41 | 59,77 | 8,31 | 0,1390 | 0,5069 | 0,2747 | 24 |
| NP58 | 83 | F | Alzheimer Demenz | AD | 20 | | 2,27 | 4,92 | 2,61 | 19,46 | 2,07 | 31,32 | 7,25 | 15,69 | 8,32 | 62,12 | 6,62 | 0,1066 | 0,4219 | 0,2528 | 34 |
| NP319 | 72 | F | Alzheimer Demenz | AD | 28 | | 1,01 | 2,42 | 1,25 | 9,08 | 0,87 | 14,63 | 6,86 | 16,56 | 8,54 | 62,07 | 5,96 | 0,0960 | 0,3600 | 0,2666 | 44 |
| NP320 | 70 | F | Alzheimer Demenz | AD | 25 | | 1,97 | 4,50 | 2,29 | 17,28 | 1,55 | 27,55 | 7,14 | 16,32 | 8,19 | 62,72 | 5,63 | 0,0898 | 0,3451 | 0,2602 | 24 |
| NP111 | 84 | F | Alzheimer Demenz | AD | 15 | | 3,02 | 7,18 | 3,49 | 25,39 | 2,02 | 41,10 | 7,34 | 17,48 | 8,49 | 61,77 | 4,92 | 0,0796 | 0,2812 | 0,2830 | 34 |
| NP128 | 74 | M | Alzheimer Demenz | AD | 8 | | 1,36 | 3,36 | 1,33 | 13,63 | 1,48 | 21,20 | 6,49 | 15,85 | 6,27 | 64,30 | 6,98 | 0,1086 | 0,4378 | 0,2460 | 34 |

[1] Mini-Mental-Status Test [2] Hulstaert et al., 1999, Neurology 52: 1555-62 [3] Summe der Aβ-Peptidspecies (ng/ml)
[4] Prozent der jeweiligen Aβ-Peptidspecies von total Aβ [5] Aβ-Peptidquotienten (Aβ-SDS-PAGE/Immunoblot)
[6] ApoE-Genotypisierung

Tabelle 20: Aβ-SDS-PAGE/Immunoblot-2; Aβ-Peptide im Liquor, ApoE-Genotypisierung und MMSE Testergebnisse der Patientenkollektive NDC-3 und AD-3 mit zugehörigen Untergruppen.

| Gruppen | Patienten | MMSE* | $A\beta_{1-42}$ ELISA[&] | $A\beta_{1-37}$ | $A\beta_{1-38}$ | $A\beta_{1-39}$ | $A\beta_{1-40}$ | $A\beta_{1-42}$ | total $A\beta$[1] | $A\beta_{1-37}$[2] | $A\beta_{1-38}$[2] | $A\beta_{1-39}$[2] | $A\beta_{1-40}$[2] | $A\beta_{1-42}$[2] | $R_{42/40}$[3] | $R_{42/38}$[3] | $R_{38/40}$[3] | ApoE |
|---|---|---|---|---|---|---|---|---|---|---|---|---|---|---|---|---|---|---|
| | | Score | (ng/ml) | (ng/ml) | (ng/ml) | (ng/ml) | (ng/ml) | (ng/ml) | (ng/ml) | (%) | (%) | (%) | (%) | (%) | | | | |
| | Anzahl | Median | Median | Median | Median | Median | Median | Median | Median | Median | Median | Median | Median | Median | Median | Median | Median | no ε4 |
| | M | p25 | p25 | p25 | p25 | p25 | p25 | p25 | p25 | p25 | p25 | p25 | p25 | p25 | p25 | p25 | p25 | 1 or 2 ε4 |
| | F | p75 | p75 | p75 | p75 | p75 | p75 | p75 | p75 | p75 | p75 | p75 | p75 | p75 | p75 | p75 | p75 | n.a. |
| NDC-3[a] | 47 | 30 | 0,720 | 1,230 | 2,783 | 1,418 | 11,622 | 2,087 | 18,658 | 6,721 | 15,024 | 7,311 | 59,786 | 10,933 | 0,184 | 0,725 | 0,251 | 37 |
| | 28 | 29 | 0,545 | 1,119 | 2,440 | 1,107 | 9,319 | 1,580 | 15,664 | 6,438 | 14,367 | 6,861 | 58,296 | 9,977 | 0,167 | 0,627 | 0,238 | 9 |
| | 19 | 30 | 0,813 | 1,602 | 3,489 | 1,766 | 14,240 | 2,880 | 24,447 | 7,268 | 15,972 | 7,668 | 60,908 | 11,581 | 0,199 | 0,802 | 0,272 | 1 |
| AD-3[b] | 12 | 16,0 | n.a. | 1,303 | 3,083 | 1,354 | 12,814 | 1,489 | 19,925 | 7,129 | 16,216 | 7,783 | 62,422 | 6,732 | 0,108 | 0,423 | 0,260 | 1 |
| | 3 | 9,5 | n.a. | 1,022 | 2,412 | 1,149 | 10,318 | 0,912 | 16,069 | 6,383 | 15,430 | 6,796 | 61,536 | 5,796 | 0,093 | 0,353 | 0,241 | 11 |
| | 9 | 22,5 | n.a. | 1,809 | 4,115 | 2,128 | 15,743 | 1,569 | 25,363 | 7,331 | 16,575 | 8,401 | 64,113 | 7,648 | 0,119 | 0,498 | 0,271 | 0 |
| IP-plasma-3[c] | 5 | 30 | n.a. | 0,034 | 0,047 | 0,050 | 0,330 | 0,080 | 0,540 | 5,877 | 8,722 | 9,181 | 61,604 | 14,598 | 0,237 | 1,708 | 0,142 | 4 |
| | 3 | 29 | n.a. | 0,031 | 0,043 | 0,048 | 0,327 | 0,076 | 0,527 | 5,870 | 8,218 | 8,948 | 61,329 | 14,559 | 0,237 | 1,695 | 0,133 | 1 |
| | 2 | 30 | n.a. | 0,037 | 0,057 | 0,057 | 0,384 | 0,091 | 0,628 | 6,185 | 8,769 | 9,410 | 61,714 | 14,861 | 0,241 | 1,752 | 0,143 | 0 |
| IP-CSF-3[d] | 5 | 30 | n.a. | 1,345 | 2,805 | 1,928 | 13,230 | 2,730 | 22,038 | 6,494 | 13,729 | 8,728 | 60,032 | 12,387 | 0,206 | 0,795 | 0,221 | 4 |
| | 3 | 29 | n.a. | 1,272 | 2,689 | 1,544 | 12,192 | 2,041 | 19,587 | 6,104 | 13,582 | 7,882 | 53,350 | 10,769 | 0,173 | 0,793 | 0,218 | 1 |
| | 2 | 30 | n.a. | 2,299 | 4,735 | 2,538 | 15,250 | 3,763 | 28,584 | 8,041 | 16,048 | 8,750 | 62,248 | 13,165 | 0,247 | 0,920 | 0,306 | 0 |
| SDS-CSF-3[e] | 5 | 30 | n.a. | 1,620 | 4,530 | 2,280 | 16,400 | 3,400 | 30,230 | 5,929 | 14,890 | 8,037 | 60,865 | 11,238 | 0,185 | 0,732 | 0,246 | 4 |
| | 3 | 29 | n.a. | 1,230 | 3,300 | 1,940 | 14,240 | 2,380 | 23,020 | 5,351 | 14,330 | 7,556 | 58,541 | 10,434 | 0,171 | 0,722 | 0,232 | 1 |
| | 2 | 30 | n.a. | 1,930 | 5,090 | 2,580 | 18,810 | 3,720 | 32,130 | 6,013 | 15,829 | 8,443 | 60,949 | 11,581 | 0,198 | 0,740 | 0,271 | 0 |

*n.a.: nicht erhältlich* * MMSE Testergebnisse liegen bei 5 der 47 NDC-3 Patienten nicht vor [1] Aβ-Peptidgesamtkonz.

[2] Prozentualer Anteil der jeweiligen Aβ-Peptidspecies an der Gesamtkonzentration [3] Aβ-Peptidquotienten

[a] Nicht-demente neuropsychiatrische Kontrollpatienten [b] Alzheimer-Demenz [&] Hulstaert et al., 1999, Neurology 52: 1555-62

[c,d] Immunopräzipitation und Aβ-SDS-PAGE/Immunoblot-2 von gepaarten [c] Plasma / [d] Liquorproben bei fünf Patienten der Gruppe NDC-3

[e] SDS-/Hitzedenaturierung mit Aβ-SDS-PAGE/Immunoblot-2 von Liquorproben der fünf NDC-3 Patienten, für die gepaarte Plasma/Liquorproben vorliegen

**Tabelle 21:** Bestimmung von $A\beta_{1-42}$ im Liquor mittels Aβ-SDS-PAGE/Immunoblot-2 und CCD-Kamera: Vergleich der Kältepräzipitation nach Einfrieren unbehandelter Liquorproben (nativ*) versus Vorbehandlung mit SDS-/Hitzedenaturierung (SDS**) für die Kollektive NDC-3[KP] und AD-3[KP]

| P-CODE | Alter | Geschlecht | Diagnose | D-Code | MMSE& | $A\beta_{1-42}$nativ* pg/ml | $A\beta_{1-42}$SDS** pg/ml | $\Delta A\beta_{1-42}$%$ | ApoE§ |
|---|---|---|---|---|---|---|---|---|---|
| NP45 | 76 | F | Alzheimer Demenz | AD | 5 | 1464,00 | 1431,75 | 2,25 | 3/4 |
| NP52 | 68 | F | Alzheimer Demenz | AD | 11 | 1586,00 | 1390,50 | 14,06 | 3/4 |
| NP58 | 83 | F | Alzheimer Demenz | AD | 20 | 2074,00 | 1775,50 | 16,81 | 3/4 |
| NP66 | 67 | F | Alzheimer Demenz | AD | 1 | 1498,25 | 1572,00 | -4,69 | 3/4 |
| NP69 | 62 | M | Alzheimer Demenz | AD | n.a. | 960,75 | 901,75 | 6,54 | n.a. |
| NP111 | 84 | F | Alzheimer Demenz | AD | 15 | 2020,00 | 2182,50 | -7,45 | 3/4 |
| NP143 | 55 | M | Alzheimer Demenz | AD | 27 | 886,00 | 965,00 | -8,19 | 2/4 |
| NP190 | 79 | F | Alzheimer Demenz | AD | 19 | 657,50 | 716,50 | -8,23 | 3/4 |
| NP197 | 64 | F | Alzheimer Demenz | AD | n.a. | 699,75 | 825,00 | -15,18 | n.a. |
| NP213 | 32 | M | Depressive Störung mit psychotischen Merkmalen | NDC (OND) | 30 | 2066,50 | 2166,25 | -4,60 | 3/3 |
| NP319 | 72 | F | Alzheimer Demenz | AD | 28 | 872,25 | 913,00 | -4,46 | 4/4 |
| NP320 | 70 | F | Alzheimer Demenz | AD | 25 | 1551,25 | 1705,00 | -9,02 | 2/4 |
| NP344 | 36 | F | Somatoforme (konversionsneurotische) Störung | NDC (OND) | 28 | 2012,00 | 2688,00 | -25,15 | 3/3 |
| NP345 | 34 | M | Panikstörung mit Agoraphobie | NDC (OND) | n.a. | 2879,75 | 3788,75 | -23,99 | 3/3 |
| NP352 | 45 | F | chronisch entzündliche ZNS-Erkrankung | NDC (CID) | 29 | 4589,75 | 5878,75 | -21,93 | 3/3 |
| NP355 | 31 | F | zerebrale transitorisch ischämische Attacke | NDC (OND) | 30 | 4931,75 | 5266,75 | -6,36 | 3/3 |
| NP356 | 65 | F | Polyzythämia vera mit transitorisch ischämischen Attacken | NDC (OND) | 30 | 1285,50 | 2858,00 | -55,02 | 3/4 |
| NP364 | 58 | M | Epilepsie | NDC (OND) | 28 | 2450,25 | 2982,50 | -17,85 | 2/3 |
| NP374 | 43 | M | chronisch entzündliche ZNS-Erkrankung | NDC (CID) | 30 | 1562,75 | 3326,25 | -53,02 | 3/4 |
| NP402 | 66 | F | Hemicranie | NDC (OND) | 30 | 1714,50 | 3004,50 | -42,94 | 3/4 |
| NP412 | 48 | M | Depressive Störung bei subkortikaler arteriosklerotischer Enzephalopatie | NDC (OND) | 30 | 1560,25 | 2961,75 | -47,32 | 3/3 |
| NP419 | 46 | M | chronisch entzündliche ZNS-Erkrankung | NDC (CID) | n.a. | 1411,75 | 2154,50 | -34,47 | 3/3 |
| NP421 | 60 | M | chronisch entzündliche ZNS-Erkrankung mit primär progressiver Aphasie | NDC (CID) | 30 | 2145,50 | 2967,75 | -27,71 | 3/3 |
| NP457 | 24 | M | Depressive Episode bei bipolar affektiver Psychose, Typ I | NDC (OND) | 30 | 2164,67 | 2156,67 | 0,37 | 2/3 |
| NP490 | 20 | M | chronisch entzündlicher ZNS-Prozeß | NDC (CID) | 30 | 4349,50 | 4789,75 | -9,19 | 3/3 |
| NP526 | 61 | M | Depressive Störung bei subkortikaler arteriosklerotischer Enzephalopatie | NDC (OND) | 29 | 3397,00 | 3412,75 | -0,46 | 3/3 |

$ $\Delta A\beta_{Peptid}\% = (A\beta_{Peptid}Nativ - A\beta_{Peptid}SDS) / A\beta_{Peptid}SDS * 100$:
Negatives (positives) $\Delta A\beta_{1-42}$ bedeutet niedrigere (höhere) Aβ-Peptidkonzentration der nativ eingefrorenen Liquorprobe bezogen auf Vorbehandlung der Probe mit SDS-/Hitzedenaturierung

§ ApoE-Genotypisierung    & Mini Mental Status Examination    n.a. = nicht vorhanden

## Patentansprüche

1. Monoklonaler Antikörper, der als mAb 1E8 bezeichnet wird, der von Hybridomen produziert wird, die bei der DSMZ-Deutsche Sammlung von Mikroorganismen und Zellkulturen GmbH, Braunschweig, am 19.12.2000 hinterlegt wurden und denen die DSMZ-Aufnahmenummer DSM ACC2485 zugeordnet wurde.

2. Antikörper mAb 1E8 nach Anspruch 1, der radioaktiv markiert ist.

3. Verwendung des Antikörpers mAb 1E8 nach Anspruch 1 oder 2 zum Nachweis von Aβ-Peptiden Aβ1-x und/oder Aβ2-x und/oder sAPPα.

4. Verwendung des Antikörpers mAb 1E8 nach Anspruch 3 in einem Western-Immunoblot.

5. Verwendung des Antikörpers mAb 1E8 nach Anspruch 4, **dadurch gekennzeichnet, dass** unspezifische Bindungsstellen zuvor mit einem Blockiermittel blockiert werden.

6. Verwendung des Antikörpers mAb 1E8 nach einem der Ansprüche 3 bis 5, **dadurch gekennzeichnet, dass** die Aβ-Peptide und das sAPPα zuvor durch eine Natrium-Laurylsulfat-Polyacrylamid-Gelelektrophorese (SDS-PAGE) voneinander getrennt werden, wobei durch Harnstoffzugabe eine Aminosäureprimärsequenz-spezifische Konformationsänderung der Aβ-Peptide induziert wird.

7. Verwendung des Antikörpers mAb 1E8 nach Anspruch 6, **dadurch gekennzeichnet, dass** die Aβ Peptide zuvor durch isoelektrische Fokussierung separiert werden.

8. Verwendung des Antikörpers mAb 1E8 nach Anspruch 6 oder 7, **dadurch gekennzeichnet, dass** in einer Probe, die aus der Gruppe ausgewählt ist, welche Liquor, Hirnhomogeniesaat, Plasma und Mischungen davon umfasst, eine Konzentration des Aβ-Peptids Aβ1-42 bestimmt wird.

9. Verwendung des Antikörpers mAb 1E8 nach Anspruch 6 oder 7, **dadurch gekennzeichnet, dass** eine Probe, die aus der Gruppe ausgewählt ist, welche Liquor, Hirnhomogeniesaat, Plasma und Mischungen davon umfasst, auf das Vorliegen einer nachweisbaren Konzentration des Aβ-Peptids Aβ2-42 überprüft wird, die bei 100 pg/ml oder höher liegt.

10. Verwendung des Antikörpers mAb 1E8 nach Anspruch 8 und 9, **dadurch gekennzeichnet, dass** ein Verhältnis zwischen der Konzentration des Aβ-Peptids Aβ2-42 zu der Konzentration des Aβ-Peptids Aβ1-42 in der Probe bestimmt wird.

11. Verwendung des Antikörpers mAb 1E8 nach Anspruch 6 oder 7, **dadurch gekennzeichnet, dass** in einer Probe, die aus der Gruppe ausgewählt ist, welche Liquor, Himhomogeniesaat, Plasma und Mischungen davon umfasst, mindestens ein Konzentrationsverhältnis bestimmt wird, das aus der Gruppe ausgewählt ist, welche ein Verhältnis zwischen einer Konzentration des Aβ-Peptids Aβ1-42 zu einer Konzentration des Aβ-Peptids Aβ1-40, ein Verhältnis zwischen einer Konzentration des Aβ-Peptids Aβ1-42 zu einer Konzentration des Aβ-Peptids Aβ1-38 und ein Verhältnis zwischen einer Konzentration des Aβ-Peptids Aβ1-38 zu einer Konzentration des Aβ-Peptids Aβ1-40 umfasst.

12. Verwendung des Antikörpers mAb 1E8 nach Anspruch 6 oder 7, **dadurch gekennzeichnet, dass** in einer Probe, die aus der Gruppe ausgewählt ist, welche Liquor, Hirnhomogeniesaat, Plasma und Mischungen davon umfasst, mindestens ein relativer Anteil Aβ1-n% einer Konzentration eines Aβ-Peptids Aβ1-n an einer Konzentration von Aβ-Peptiden Aβ1-x bestimmt wird, wobei der relative Anteil Aβ1-n% aus der Gruppe ausgewählt ist, welche einen relativen Anteil Aβ1-42% einer Konzentration des Aβ-Peptids Aβ1-42, einen relativen Anteil Aβ1-40% einer Konzentration des Aβ-Peptids Aβ1-40 und einen relativen Anteil Aβ1-38% einer Konzentration des Aβ-Peptids Aβ1-38 umfasst, und wobei die Konzentration von Aβ-Peptiden Aβ1-x mindestens die Konzentration der Aβ-Peptide Aβ1-38, Aβ1-40 und Aβ1-42 aus der Gruppe der Aβ-Peptide Aβ1-37, Aβ1-38, Aβ1-39, Aβ1-40 und Aβ1-42 umfasst.

13. Verwendung des Antikörpers mAb 1E8 nach einem der Ansprüche 1 bis 12, **dadurch gekennzeichnet, dass** die Aβ-Peptide zuvor durch eine Behandlung mit einem Detergenz aufgeschlossen werden.

14. Verwendung des Antikörpers mAb 1E8 nach Anspruch 13, **dadurch gekennzeichnet, dass** die Aβ-Peptide durch eine SDS-Hitzedenaturierung aufgeschlossen werden.

15. Verwendung des Antikörpers mAb 1E8 nach Anspruch 8 und 13 oder 14, **dadurch gekennzeichnet, dass** die Probe in mindestens zwei Teilproben unterteilt wird, dass eine erste Teilprobe der Probenbehandlung mit dem Detergenz vor oder statt einer Präzipitationsbehandlung unterworfen wird, die zumindest auf Teilmengen des Aβ-Peptids Aβ1-42 gerichtet ist, dass eine zweite Teilprobe der Präzipitationsbehandlung vor oder statt der Probenbehandlung mit dem Detergenz unterworfen wird, und dass eine Differenz ΔAβ1-42 zwischen den in beiden Probenteilen bestimmten Konzentrationen des Aβ-Peptids Aβ1-42 bestimmt wird.

16. Verwendung des Antikörpers mAb 1E8 nach einem der Ansprüche 1 bis 15, **dadurch gekennzeichnet, dass** die Aβ-Peptide, an die der Antikörper mAb 1E8 gebunden ist, mit einem gegen den Antikörper mAb 1E8 gerichtete sekundären Antikörper markiert werden.

17. Verwendung des Antikörpers mAb 1E8 nach Anspruch 16, **dadurch gekennzeichnet, dass** der gegen den Antikörper mAb 1E8 gerichtete sekundäre Antikörper bereits mit einem mengenmäßig registrierbaren Marker versehen ist oder nach seiner Immunreaktion mit dem Antikörper mAb 1E8 mit einem mengenmäßig registrierbaren Marker versehen wird.

18. Verwendung des Antikörpers mAb 1E8 nach Anspruch 16 oder 17, **dadurch gekennzeichnet, dass** die Menge des markierten Antikörper mAb 1E8 photometrisch mit einer CCD-Kamera bestimmt wird.

19. Verwendung des Antikörpers mAb 1E8 nach Anspruch 1 oder 2 zum Aufkonzentrieren von Aβ-Peptiden Aβ1-x und Aβ2-x.

20. Verwendung des Antikörpers mAb 1E8 nach Anspruch 1 oder 2 zur Unterscheidung von Aβ-Peptiden Aβ1-x und Aβ2-x von Aβ-Peptiden Aβn-x mit n>2.

## Claims

1. A monoclonal antibody which is referred to as mAb 1E8, which is produced by hybridomas which were deposited at the DSMZ-Deutsche Sammlung von Mikroorganismen und Zellkulturen GmbH, Braunschweig, on Dec. 19, 2000, and which were assigned the DSMZ accession number DSM ACC2485.

2. An antibody mAb 1E8 as claimed in claim 1, which is radiolabeled.

3. The use of the antibody mAb 1E8 as claimed in claim 1 or 2 for detecting Aβ peptides Aβ1-x and/or Aβ2-x and/or sAPPα.

4. The use of the antibody mAb 1E8 as claimed in claim 3 in a Western immunoblot.

5. The use of the antibody mAb 1E8 as claimed in claim 4, **characterized in that** nonspecific binding sites are previously blocked with a blocking agent.

6. The use of the antibody mAb 1E8 as claimed in any of claims 3 to 5, **characterized in that** the Aβ peptides and the sAPPα are previously separated from one another by a sodium lauryl sulfate polyacrylamide gel electrophoresis (SDS-PAGE), inducing an amino acid primary sequence-specific conformational change in the Aβ peptides by addition of urea.

7. The use of the antibody mAb 1E8 as claimed in claim 6, **characterized in that** the Aβ peptides are previously separated by isoelectric focusing.

8. The use of the antibody mAb 1E8 as claimed in claim 6 or 7, **characterized in that** a concentration of the Aβ peptide Aβ1-42 is determined in a sample which is selected from the group which comprises CSF, brain homogenius seed, plasma and mixtures thereof.

9. The use of the antibody mAb 1E8 as claimed in claim 6 or 7, **characterized in that** a sample which is selected from the group which comprises CSF, brain homogenius seed, plasma and mixtures thereof is investigated for the presence of a detectable concentration of the Aβ peptide Aβ2-42 which is at 100 pg/ml or above.

**10.** The use of the antibody mAb 1E8 as claimed in claim 8 and 9, **characterized in that** a ratio between the concentration of the Aβ peptide Aβ2-42 to the concentration of the Aβ peptide Aβ1-42 in the sample is determined.

**11.** The use of the antibody mAb 1E8 as claimed in claim 6 or 7, **characterized in that** at least one concentration ratio which is selected from the group which comprises a ratio between a concentration of the Aβ peptide Aβ1-42 to a concentration of the Aβ peptide Aβ1-40, a ratio between a concentration of the Aβ peptide Aβ1-42 to a concentration of the Aβ peptide Aβ1-38 and a ratio between a concentration of the Aβ peptide Aβ1-38 to a concentration of the Aβ peptide Aβ1-40 is determined in a sample which is selected from the group which comprises CSF, brain homogenius seed, plasma and mixtures thereof.

**12.** The use of the antibody mAb 1E8 as claimed in claim 6 or 7, **characterized in that** at least one relative proportion Aβ1-n% of a concentration of an Aβ peptide Aβ1-n in a concentration of Aβ peptides Aβ1-x, where the relative proportion Aβ1-n% is selected from the group which comprises a relative proportion Aβ1-42% of a concentration of the Aβ peptide Aβ1-42, a relative proportion Aβ1-40% of a concentration of the Aβ peptide Aβ1-40 and a relative proportion Aβ1-38% of a concentration of the Aβ peptide Aβ1-38, and where the concentration of Aβ peptides Aβ1-x comprises at least the concentration of the Aβ peptides Aβ1-38, Aβ1-40 and Aβ1-42 from the group of Aβ peptides Aβ1-37, Aβ1-38, Aβ1-39, Aβ1-40 and Aβ1-42 is determined in a sample which is selected from the group which comprises CSF, brain homogenius seed, plasma and mixtures thereof.

**13.** The use of the antibody mAb 1E8 as claimed in any of claims 1 to 12, **characterized in that** the Aβ peptides are previously digested by a treatment with a detergent.

**14.** The use of the antibody mAb 1E8 as claimed in claim 13, **characterized in that** the Aβ peptides are digested by an SDS-thermal denaturation.

**15.** The use of the antibody mAb 1E8 as claimed in claim 8 and 13 or 14, **characterized in that** the sample is divided into at least two part-samples, wherein a first part-sample is subjected to the sample treatment with the detergent before or instead of a precipitation treatment which is directed at at least part portions of the Aβ peptide Aβ1-42, and that a second part-sample is subjected to the precipitation treatment before or instead of the sample treatment with the detergent, and that a difference ΔAβ1-42 between the concentrations of the Aβ peptide Aβ1-42 determined in the two part-samples is determined.

**16.** The use of the antibody mAb 1E8 as claimed in any of claims 1 to 15, **characterized in that** the Aβ peptides to which the antibody mAb 1E8 is bound are labeled with a secondary antibody directed against the antibody mAb 1E8.

**17.** The use of the antibody mAb 1E8 as claimed in claim 16, **characterized in that** the secondary antibody directed against the antibody mAb 1E8 is already provided with a marker whose quantity can be recorded, or is provided after its immune reaction with the antibody mAb 1E8 with a marker whose quantity can be recorded.

**18.** The use of the antibody mAb 1E8 as claimed in claim 16 or 17, **characterized in that** the amount of the labeled antibody mAb 1E8 is determined by photometry with a CCD camera.

**19.** The use of the antibody mAb 1E8 as claimed in claim 1 or 2 for concentrating Aβ peptides Aβ1-x and Aβ2-x.

**20.** The use of the antibody mAb 1E8 as claimed in claim 1 or 2 for distinguishing Aβ peptides Aβ1-x and Aβ2-x from Aβ peptides Aβn-x with n>2.

## Revendications

**1.** Anticorps monoclonal désigné par mAb 1E8, qui est produit à partir d'hybridomes qui ont été déposés auprès de la DSMZ (Deutsche Sammlung von Mikro organismen und Zellkulturen GmbH, Braunschweig) le 19.12.2000 et auxquels le numéro d'enre gistrement DSM ACC2485 a été attribué par DSMZ.

**2.** Anticorps mAb 1E8 selon la revendication 1, qui est marqué radioactivement.

**3.** Utilisation de l'anticorps mAb 1E8 selon la revendication 1 ou 2 pour la détection de peptides Aβ1-x et/ou Aβ2-x et/ou sAPPα.

**4.** Utilisation de l'anticorps mAb 1E8 selon la revendication 3 dans un immunoblot Western.

**5.** Utilisation de l'anticorps mAb 1E8 selon la revendication 4, **caractérisée en ce que** les emplacements de liaison non spécifiques sont préalablement bloqués à l'aide d'un agent de blocage.

**6.** Utilisation de l'anticorps mAb 1E8 selon l'une quelconque des revendications 3 à 5, **caractérisée en ce que** les peptides Aβ et le peptide sAPPα sont d'abord séparés l'un de l'autre par une électrophorèse sur gel de polyac rylamide et de laurylsulfate de sodium (SDS-PAGE), une modification de la conformation des peptides Aβ, spécifique à la séquence d'acides aminés primaires, étant induite par addition d'urée.

**7.** Utilisation de l'anticorps mAb 1E8 selon la revendication 6, **caractérisée en ce que** les peptides Aβ sont préalablement séparés par focalisation isoélectrique.

**8.** Utilisation de l'anticorps mAb 1E8 selon la revendication 6 ou 7, **caractérisée en ce que** dans un échantillon qui est sélectionné dans le groupe qui compre nd le liquide amniotique, un homogénéisat de cerveau, du plasma et des mélanges de ceux -ci, on détermine une concentration du peptide Aβ1-42.

**9.** Utilisation de l'anticorps mAb 1E8 selon la revendication 6 ou 7, **caractérisée en ce que** l'on vérifie sur un é chantillon qui est sélectionné dans le groupe qui comprend le liquide amniotique, un homogénéisat de cerveau, du plasma et des mélanges de ceux -ci, la présence d'une concentration détectable du peptide Aβ2-42 qui est située à 100 pg/ml ou plus haut.

**10.** Utilisation de l'anticorps mAb 1E8 selon les revendications 8 et 9, **caractérisée en ce que** l'on détermine un rapport entre la concentration du peptide Aβ2-42 et la concentration du peptide Aβ1-42 dans l'échantillon.

**11.** Utilisation de l'anticorps mAb 1E8 s elon les revendications 6 ou 7, **caractérisée en ce que** dans un échantillon qui est sélectionné dans le groupe qui comprend le liquide amniotique, un homogénéisat de cerveau, du plasma et des mélanges de ceux-ci, on détermine au moins un rapport de concentrations qui est sélectionné dans le groupe qui comprend un rapport entre une concentration du peptide Aβ1-42 et une concentration du peptide Aβ1-40, un rapport entre une concentration du peptide Aβ1-42 et une concentration du peptide Aβ1-38 et un rapport en tre une concentration du peptide Aβ1-38 et une concentration du peptide Aβ1-40.

**12.** Utilisation de l'anticorps mAb 1E8 selon la revendication 6 ou 7, **caractérisée en ce que** dans un échantillon qui est sélectionné dans le groupe qui comprend le liquide amn iotique, un homogénéisat de cerveau, le plasma et des mélanges de ceux-ci, on détermine au moins une proportion relative Aβ1-n% d'une concentration d'un peptide Aβ1-n et une concentration de peptides Aβ1-x, la proportion relative Aβ1-n% étant sélectionnée dans le groupe qui comprend une proportion relative Aβ1-42% d'une concentration du peptide Aβ1-42, une proportion relative Aβ1-40% d'une concentration du peptide Aβ1-40 et une proportion relative Aβ1-38% d'une concentration du peptide Aβ1-38, la concentration des peptides Aβ1-x comprenant au moins la concentration du peptide Aβ1-38, du peptide Aβ1-40 et du peptide Aβ1-42 du groupe des peptides Aβ1-37, Aβ1-38, Aβ1-39, Aβ1-40 et Aβ1-42.

**13.** Utilisation de l'anticorps mAb 1E8 selon l'une quelconque des revendications 1 à 12, **caractérisée en ce que** les peptides Aβ sont préalablement dissous par un traitement à l'aide d'un détergent.

**14.** Utilisation de l'anticorps mAb 1E8 selon la revendication 13, **caractérisée en ce que** les peptides Aβ sont dissous par une dénaturisation SDS à la chaleur.

**15.** Utilisation de l'anticorps mAb 1E8 selon les revendications 8 et 13 ou 14, **caractérisée en ce que** l'échantillon est divisé en au moins deux échantillons partiels, **en ce qu'**un premier échantillon partiel subit le traitement d'échantillon à l'aide du détergent avant ou à la place d'un traitement par précipitation qui est appliqué sur au moins une partie de la quantité du peptide Aβ1-42, **en ce qu'**un deuxième échantillon partiel subit le traitement par précipitation avant ou à la place du traitement de l'échantillon à l'aide du détergent et **en ce que** l'on détermine la différence ΔAβ1-42 entre les concentrations du peptide Aβ1-42 déterminées dans les deux parties de l'échantillon.

**16.** Utilisation de l'anticorps mAb 1E8 selon l' une quelconque des revendications 1 à 15, **caractérisée en ce que** les peptides Aβ auxquels l'anticorps mAb 1E8 est lié sont marqués par un anticorps secondaire dirigé contre l'anticorps mAb 1E8.

**17.** Utilisation de l'anticorps mAb 1E8 selon la revendication 16, **caractérisée en ce que** l'anticorps secondaire dirigé contre l'anticorps mAb 1E8 est déjà doté d'un marqueur enregistrable quantitativement ou est doté d'un marqueur enregistrable quantitativement après son immunoréaction avec l'anticorps mAb 1E8.

**18.** Utilisation de l'anticorps mAb 1E8 selon la revendication 16 ou 17, **caractérisée en ce que** la quantité de l'anticorps marqué mAb 1E8 est déterminée photométriquement à l'aide d'une caméra à CCD.

**19.** Utilisation de l'anticorps mAb 1E8 selon la revendication 1 ou 2 pour la concentration de peptides Aβ1-x et Aβ2-x.

**20.** Utilisation de l'anticorps mAb 1E8 selon la revendication 1 ou 2 pour distinguer les peptides Aβ1-x et A β2-x des peptides Aβn-x pour lesquels n>2.

Fig. 1

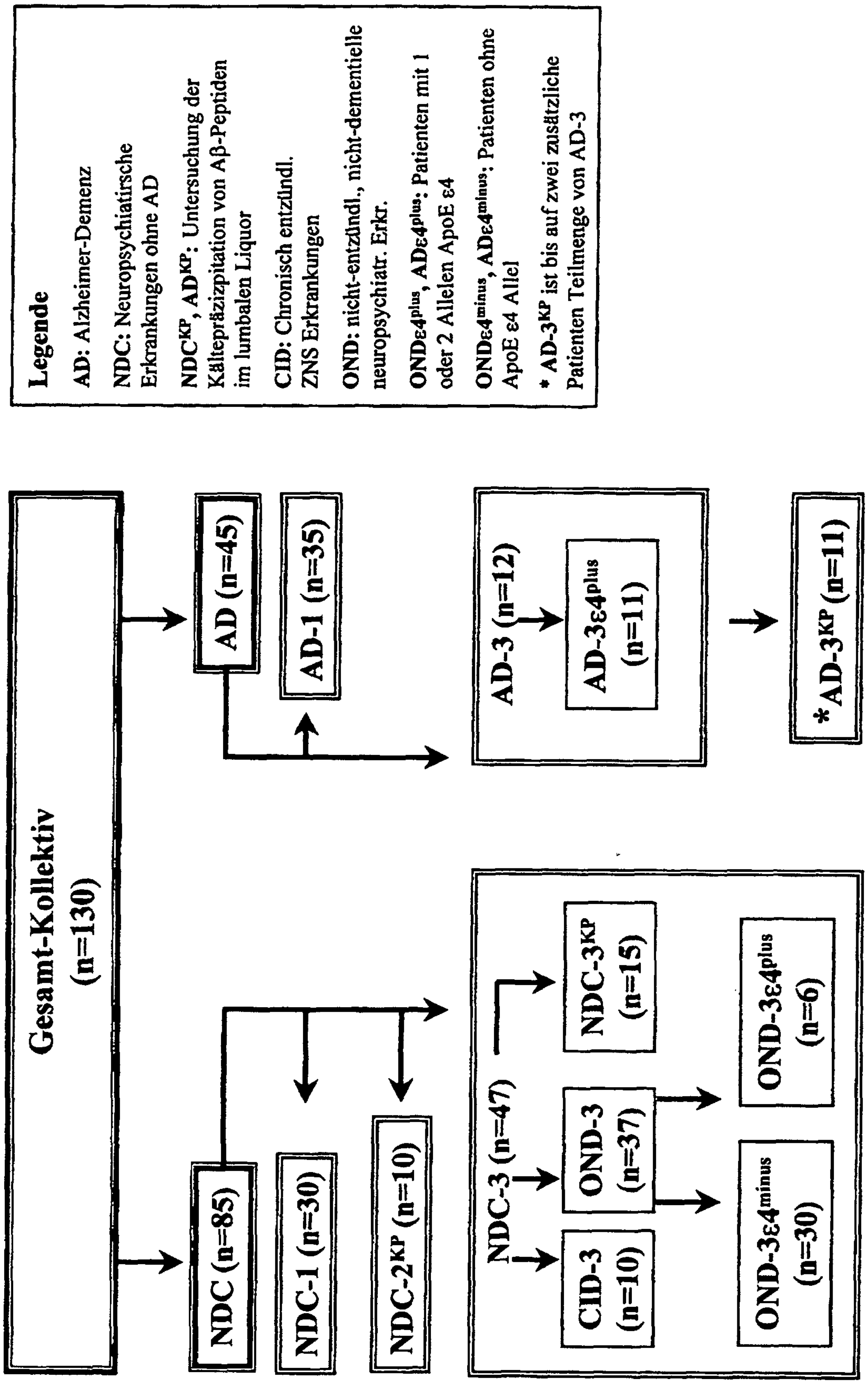
Gesamt-Kollektiv (n=130)
NDC (n=85)
NDC-1 (n=30)
NDC-$2^{KP}$ (n=10)
NDC-3 (n=47)
CID-3 (n=10)
OND-3 (n=37)
NDC-$3^{KP}$ (n=15)
OND-3$\varepsilon 4^{minus}$ (n=30)
OND-3$\varepsilon 4^{plus}$ (n=6)
AD (n=45)
AD-1 (n=35)
AD-3 (n=12)
AD-3$\varepsilon 4^{plus}$ (n=11)
* AD-$3^{KP}$ (n=11)
Legende
AD: Alzheimer-Demenz
NDC: Neuropsychiatirsche Erkrankungen ohne AD
$NDC^{KP}$, $AD^{KP}$: Untersuchung der Kältepräzizpitation von Aβ-Peptiden im lumbalen Liquor
CID: Chronisch entzündl. ZNS Erkrankungen
OND: nicht-entzündl., nicht-dementielle neuropsychiatr. Erkr.
OND$\varepsilon 4^{plus}$, AD$\varepsilon 4^{plus}$: Patienten mit 1 oder 2 Allelen ApoE ε4
OND$\varepsilon 4^{minus}$, AD$\varepsilon 4^{minus}$: Patienten ohne ApoE ε4 Allel
* AD-$3^{KP}$ ist bis auf zwei zusätzliche Patienten Teilmenge von AD-3

Fig. 2

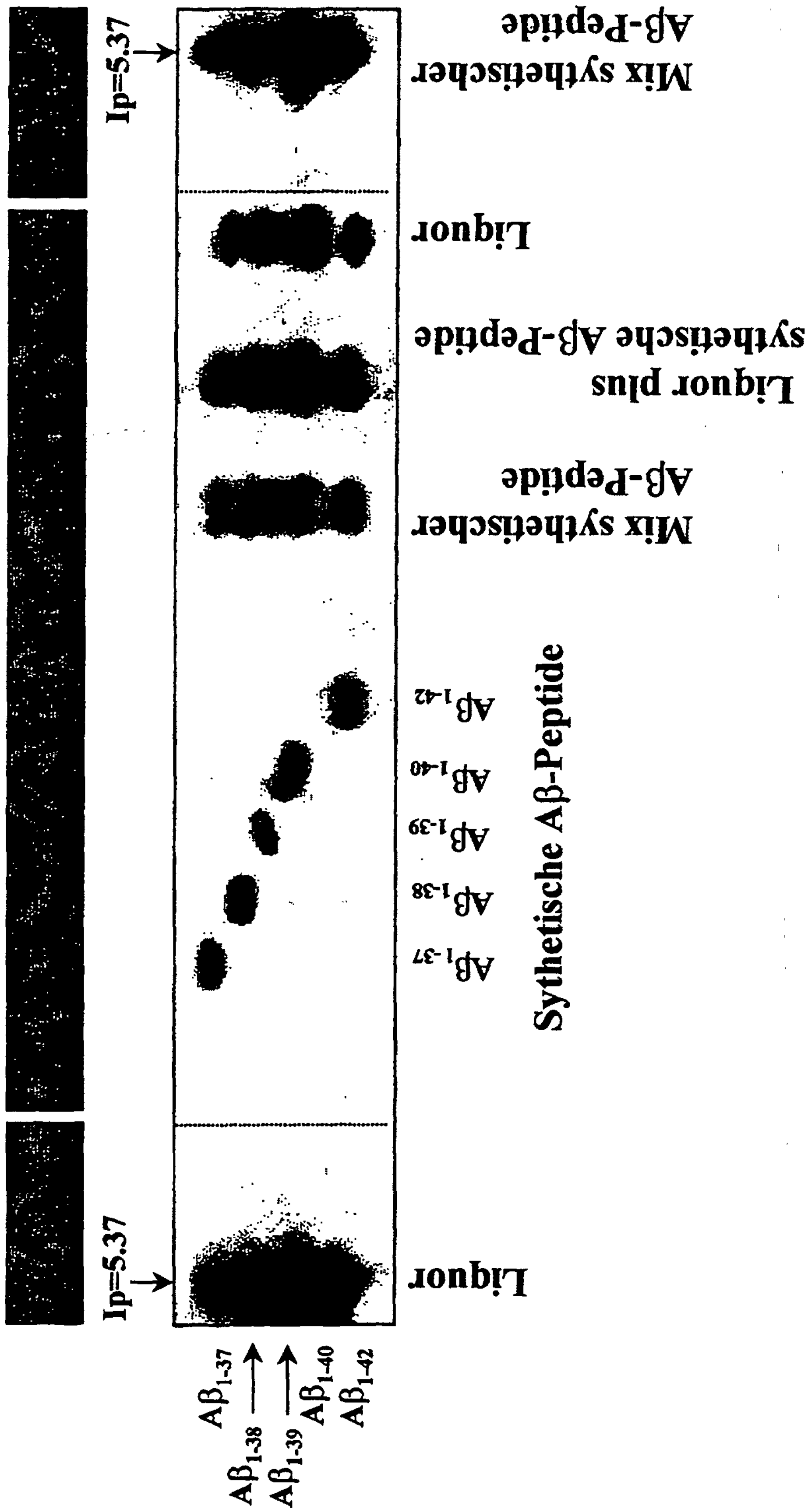
Ip=5.37
Ip=5.37
Liquor
Sythetische Aβ-Peptide
$A\beta_{1-37}$
$A\beta_{1-38}$
$A\beta_{1-39}$
$A\beta_{1-40}$
$A\beta_{1-42}$
Mix sythetischer Aβ-Peptide
Liquor plus sythetische Aβ-Peptide
Liquor
Mix sythetischer Aβ-Peptide
$A\beta_{1-37}$
$A\beta_{1-38}$
$A\beta_{1-39}$
$A\beta_{1-40}$
$A\beta_{1-42}$

Fig. 3

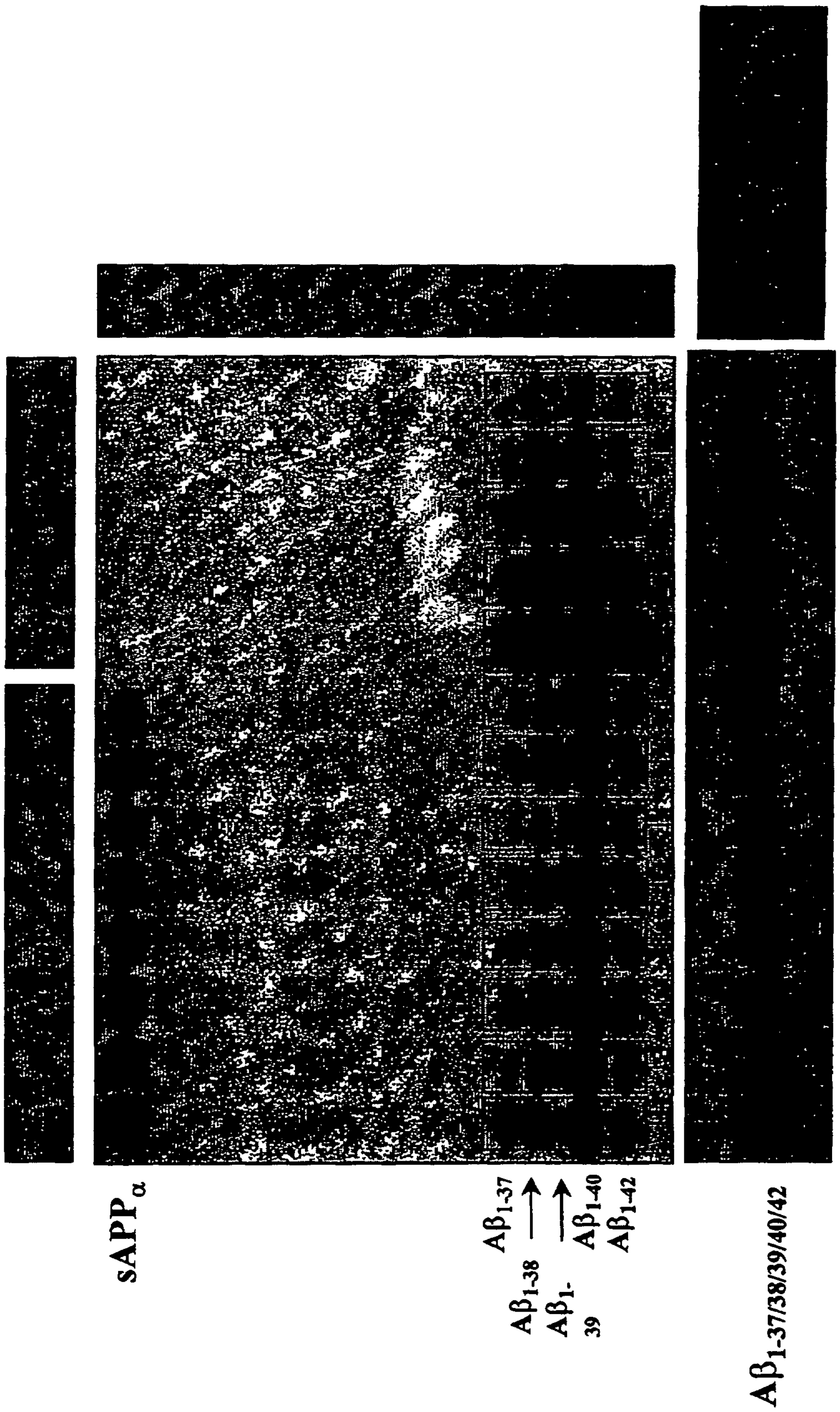
sAPPα
Aβ1-37
Aβ1-38
Aβ1-39
Aβ1-40
Aβ1-42
Aβ1-37/38/39/40/42
1 2 3 4 5 6 7 8 a b c d e

Fig. 4

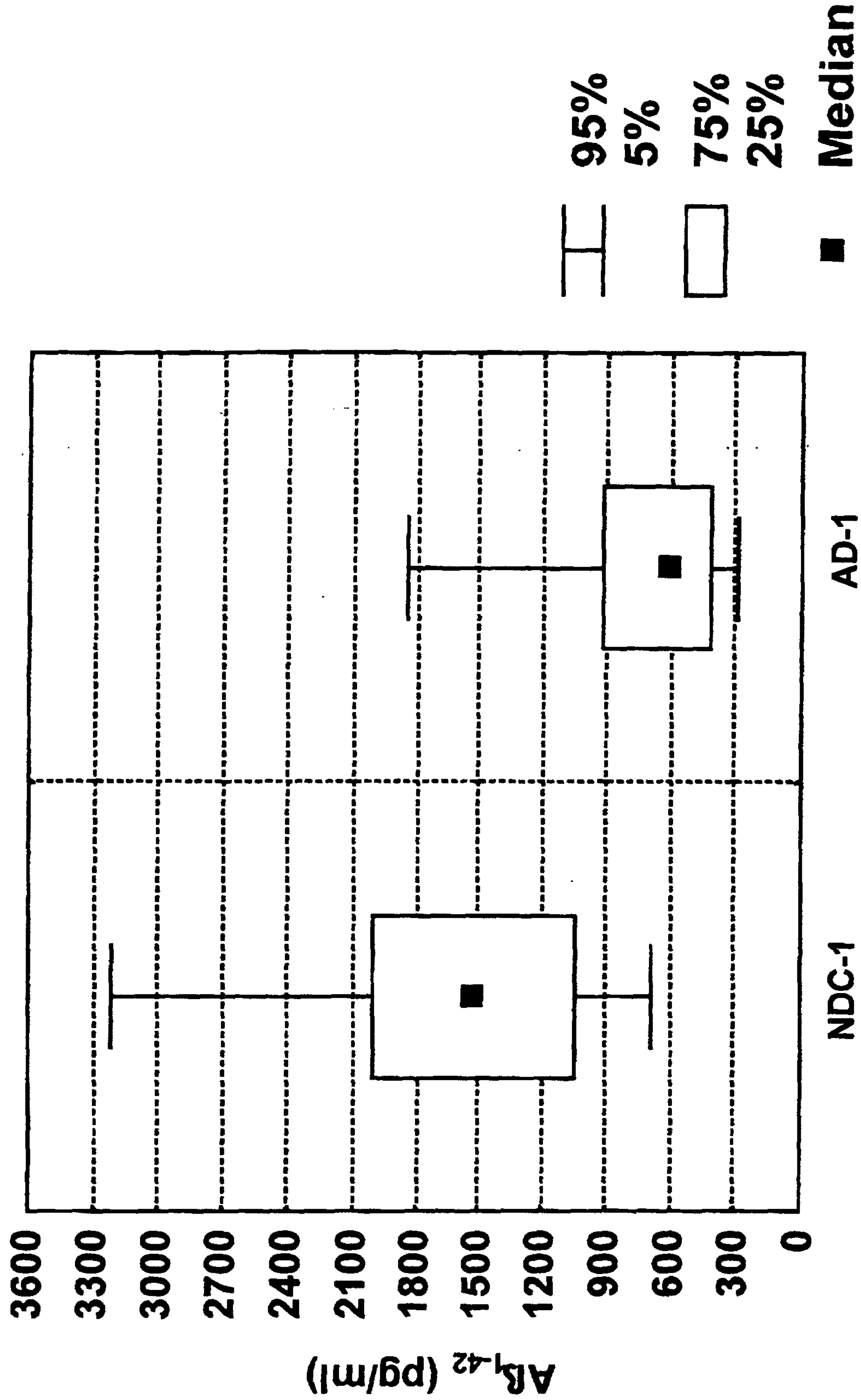
3600
3300
3000
2700
2400
2100
1800
1500
1200
900
600
300
0
Aß1-42 (pg/ml)
NDC-1
AD-1
95%
5%
75%
25%
Median

Fig. 5

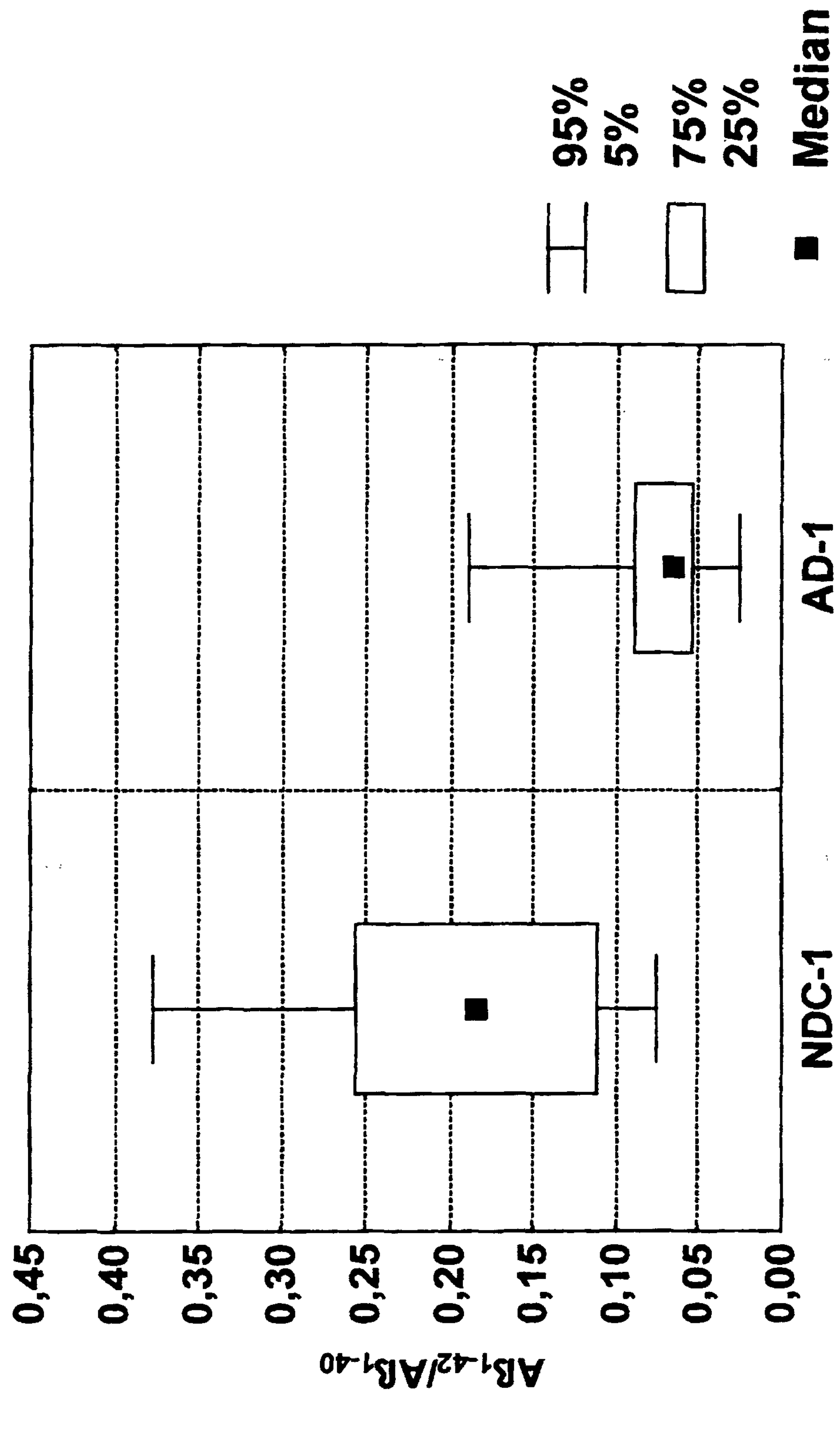

Aß1-42/Aß1-40
0,45
0,40
0,35
0,30
0,25
0,20
0,15
0,10
0,05
0,00
NDC-1
AD-1
95%
5%
75%
25%
Median

Fig. 6

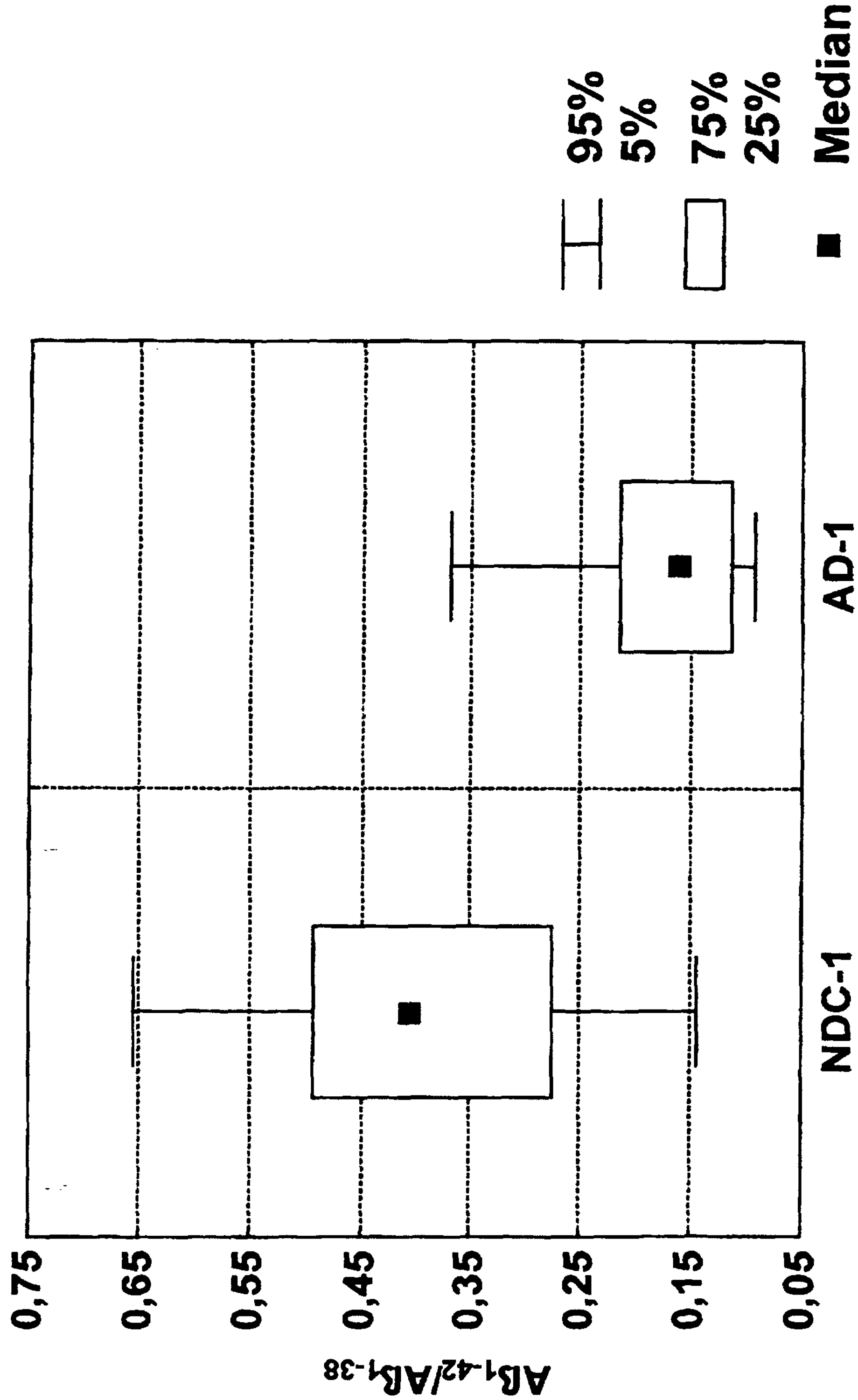
0,75
0,65
0,55
0,45
0,35
0,25
0,15
0,05
Aβ1-42/Aβ1-38
NDC-1
AD-1
95%
5%
75%
25%
Median

Fig. 7

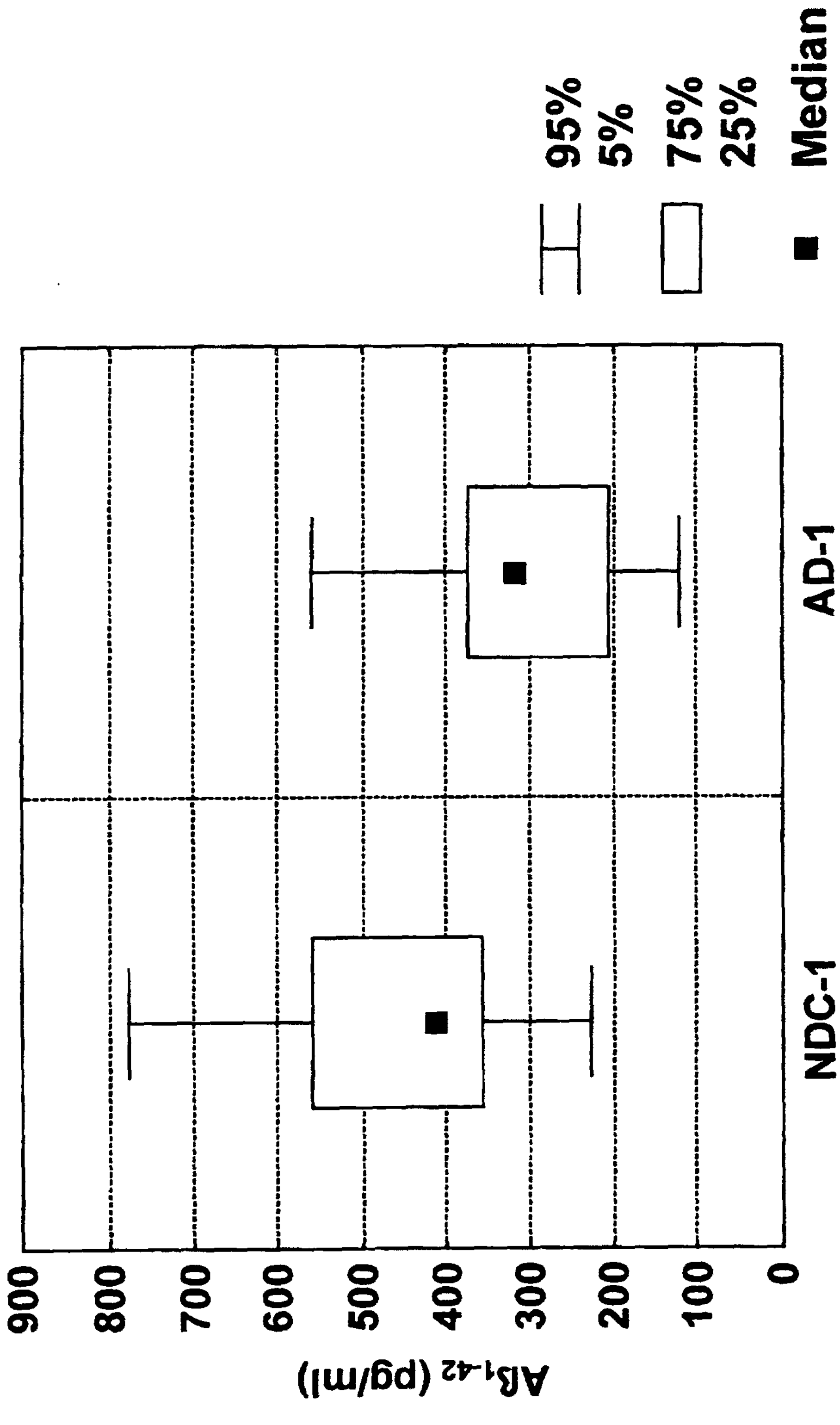
Aβ1-42 (pg/ml)
900
800
700
600
500
400
300
200
100
0
NDC-1
AD-1
95%
5%
75%
25%
Median

Fig. 8

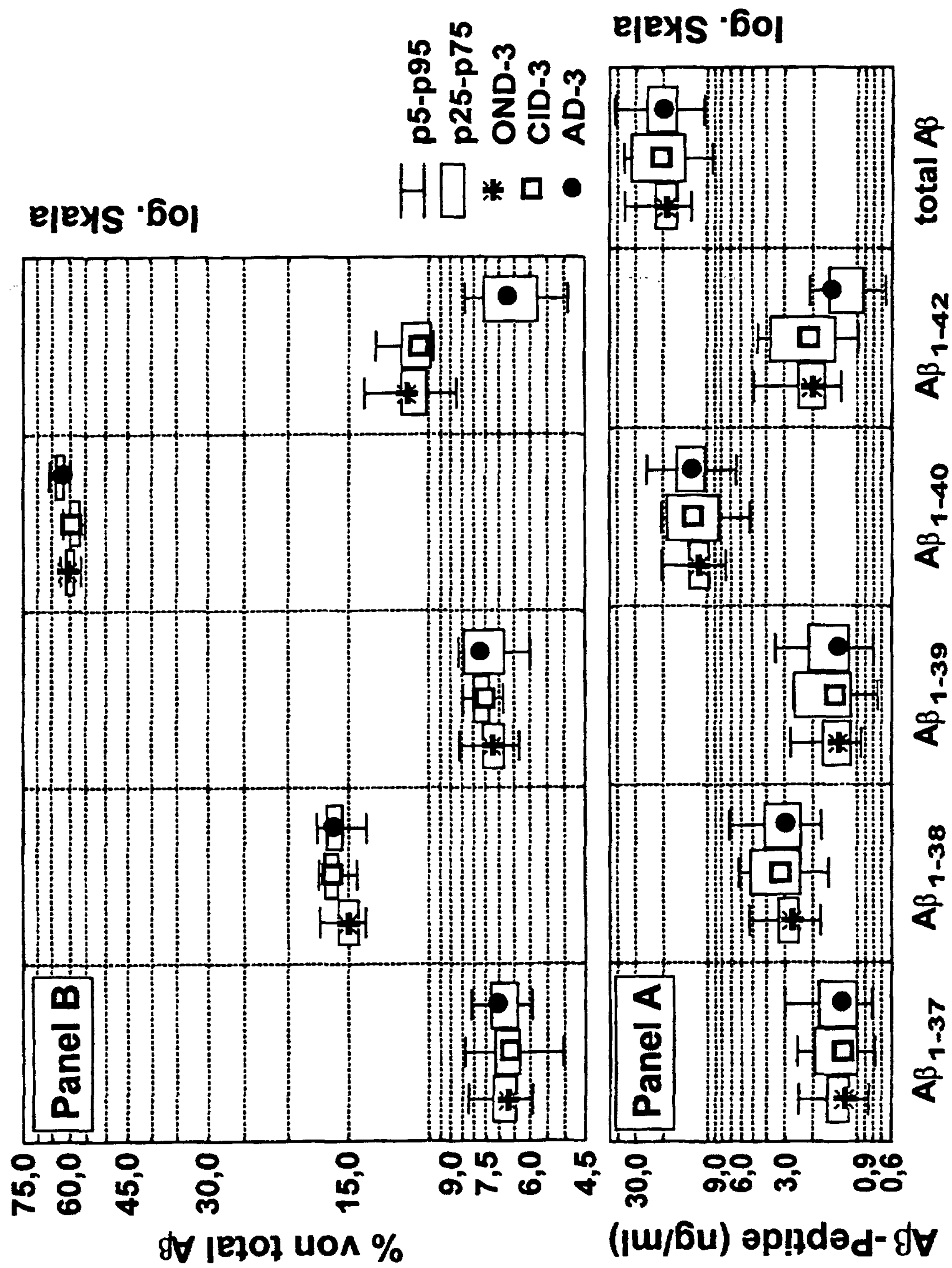
Panel B
Panel A
% von total Aβ
Aβ-Peptide (ng/ml)
log. Skala
log. Skala
75,0
60,0
45,0
30,0
15,0
9,0
7,5
6,0
4,5
30,0
9,0
6,0
3,0
0,9
0,6
$Aβ_{1-37}$
$Aβ_{1-38}$
$Aβ_{1-39}$
$Aβ_{1-40}$
$Aβ_{1-42}$
total Aβ
p5-p95
p25-p75
OND-3
CID-3
AD-3

Fig. 9

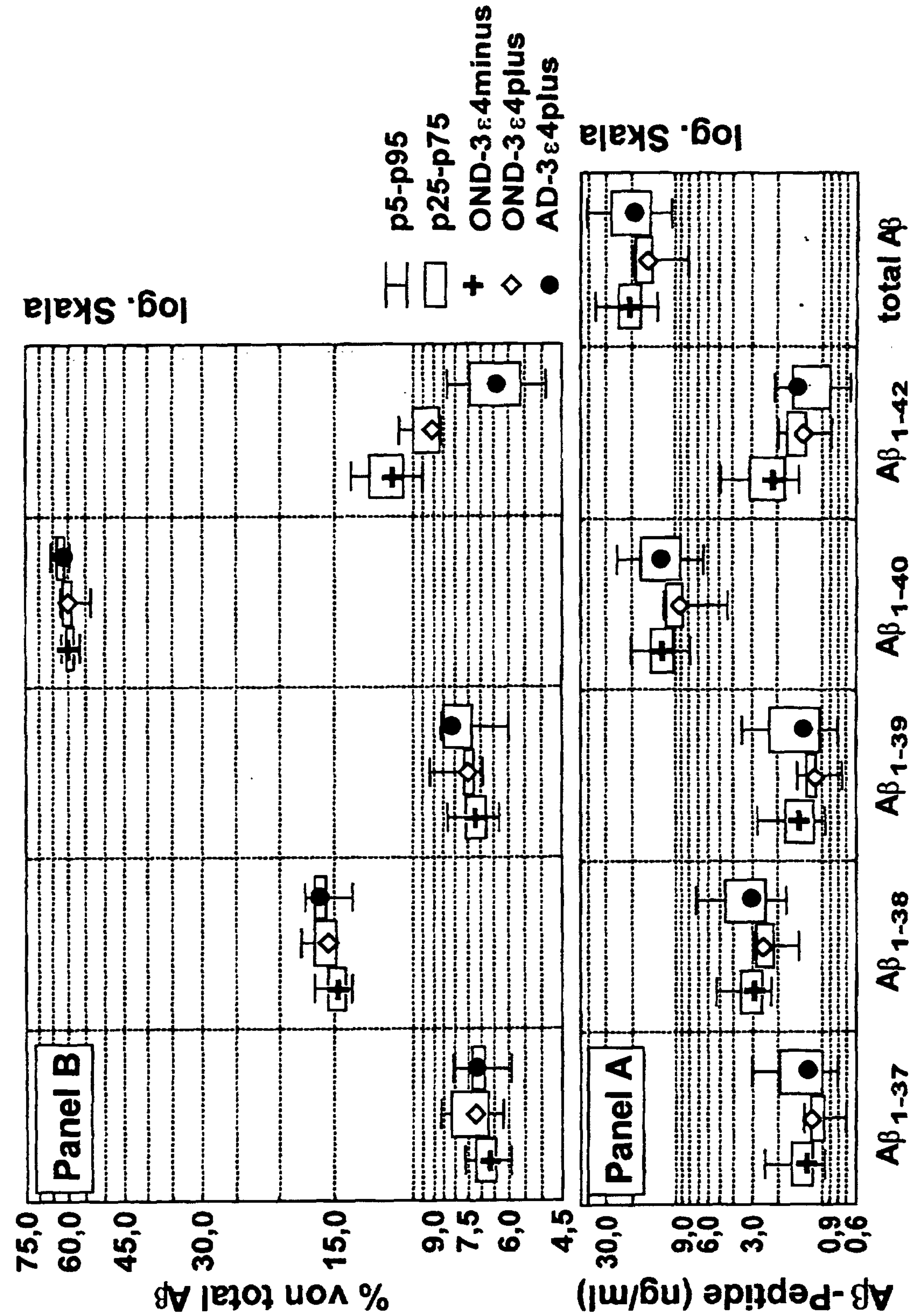
Panel B
log. Skala
% von total Aβ
75,0
60,0
45,0
30,0
15,0
9,0
7,5
6,0
4,5
p5-p95
p25-p75
OND-3ε4minus
OND-3ε4plus
AD-3ε4plus
Panel A
log. Skala
Aβ-Peptide (ng/ml)
30,0
9,0
6,0
3,0
0,9
0,6
Aβ 1-37
Aβ 1-38
Aβ 1-39
Aβ 1-40
Aβ 1-42
total Aβ

Fig. 10

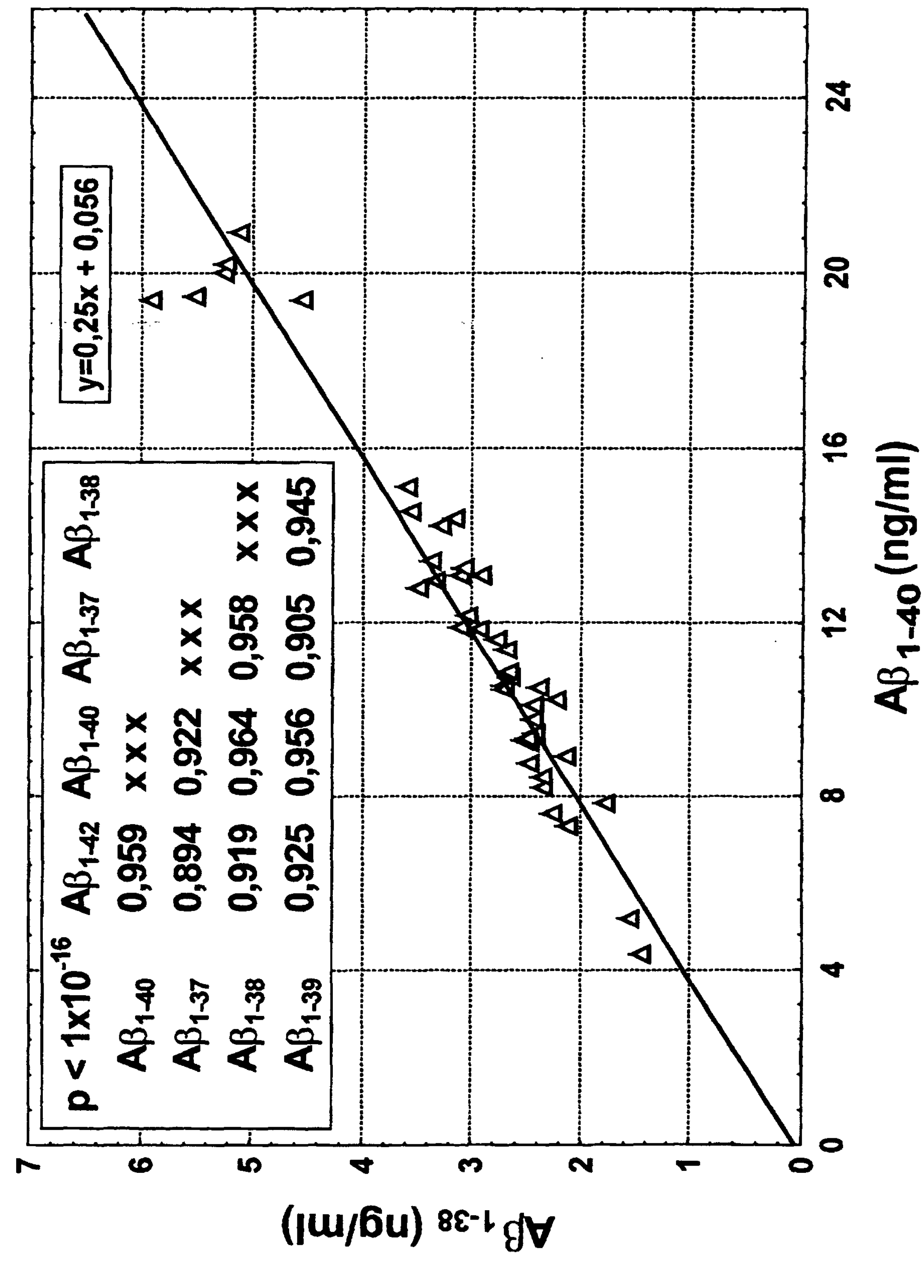

p < 1x10-16 Aβ1-42 Aβ1-40 Aβ1-37 Aβ1-38
Aβ1-40 0,959 x x x
Aβ1-37 0,894 0,922 x x x
Aβ1-38 0,919 0,964 0,958 x x x
Aβ1-39 0,925 0,956 0,905 0,945
y=0,25x + 0,056
Aβ1-38 (ng/ml)
Aβ1-40 (ng/ml)
0
1
2
3
4
5
6
7
0
4
8
12
16
20
24

Fig. 11

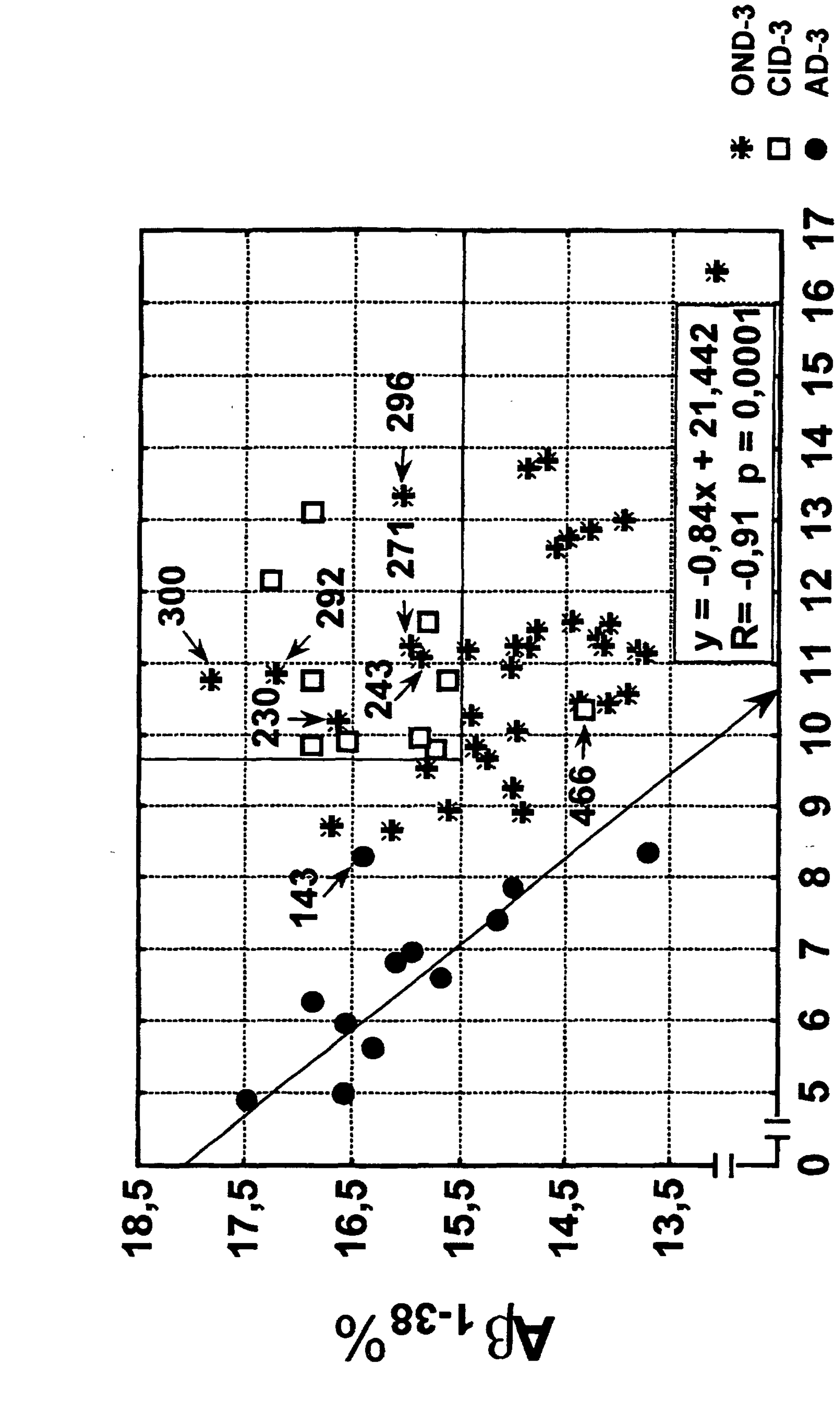
Aβ 1-38 %
18,5
17,5
16,5
15,5
14,5
13,5
0
5
6
7
8
9
10
11
12
13
14
15
16
17
Aβ 1-42 %
300
292
230
243
271
296
143
466
y = -0,84x + 21,442
R= -0,91 p = 0,0001
OND-3
CID-3
AD-3

Fig 12

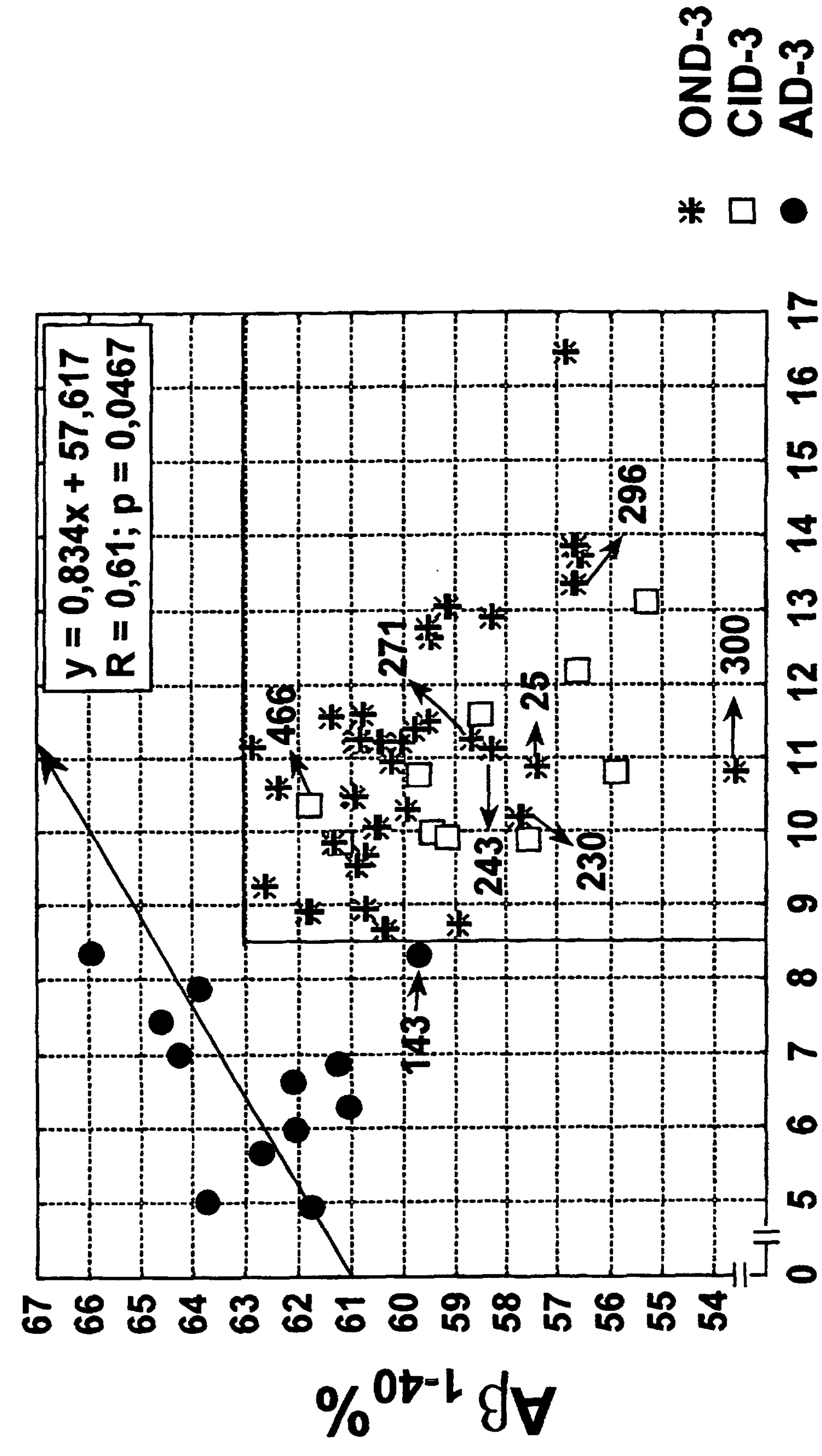
y = 0,834x + 57,617
R = 0,61; p = 0,0467
Aβ 1-40%
Aβ 1-42%
OND-3
CID-3
AD-3
466
271
296
25
300
243
230
143

Fig. 13

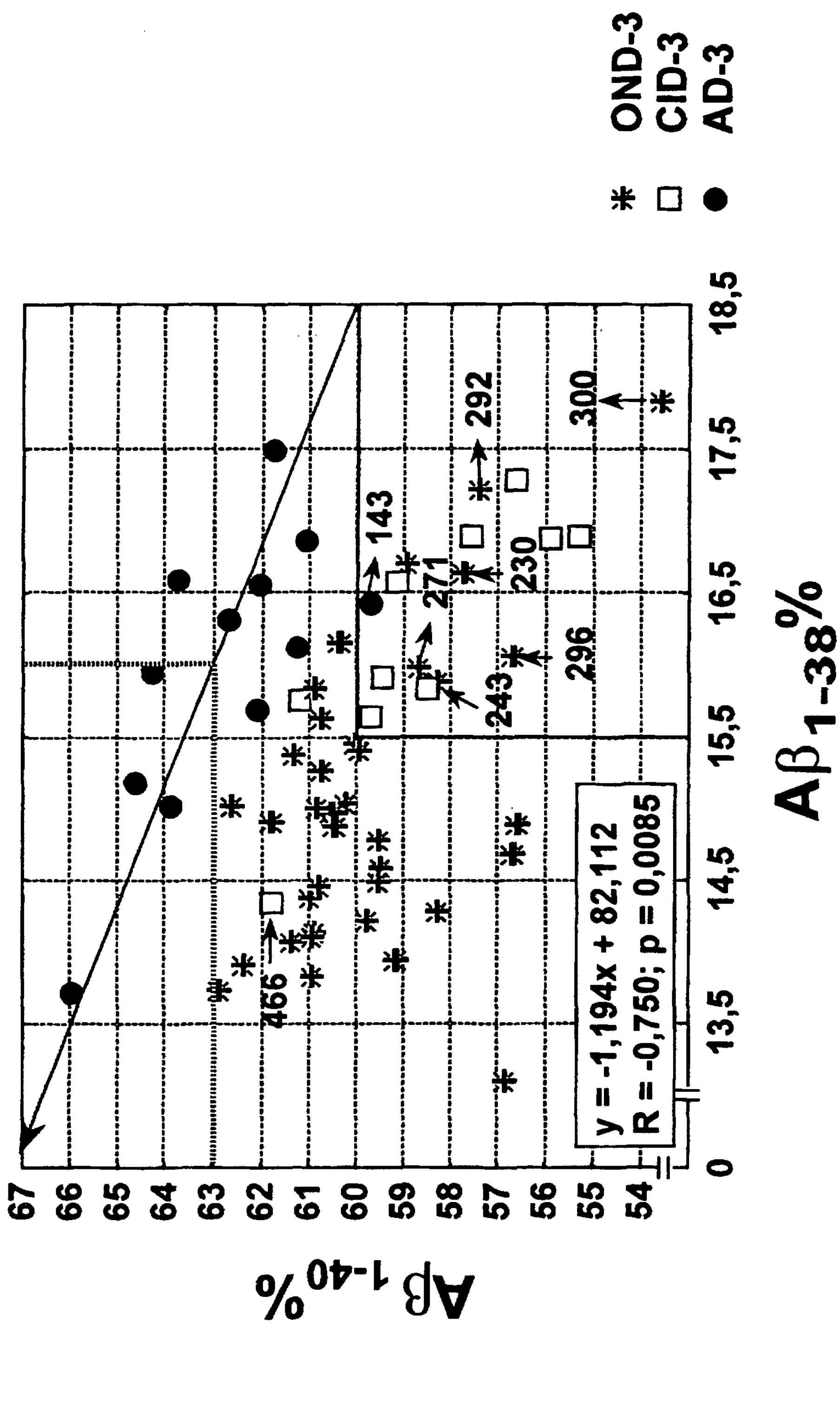
Aβ 1-40%
67
66
65
64
63
62
61
60
59
58
57
56
55
54
0
13,5
14,5
15,5
16,5
17,5
18,5
Aβ 1-38%
y = -1,194x + 82,112
R = -0,750; p = 0,0085
466
243
296
271
230
143
292
300
OND-3
CID-3
AD-3

Fig. 14

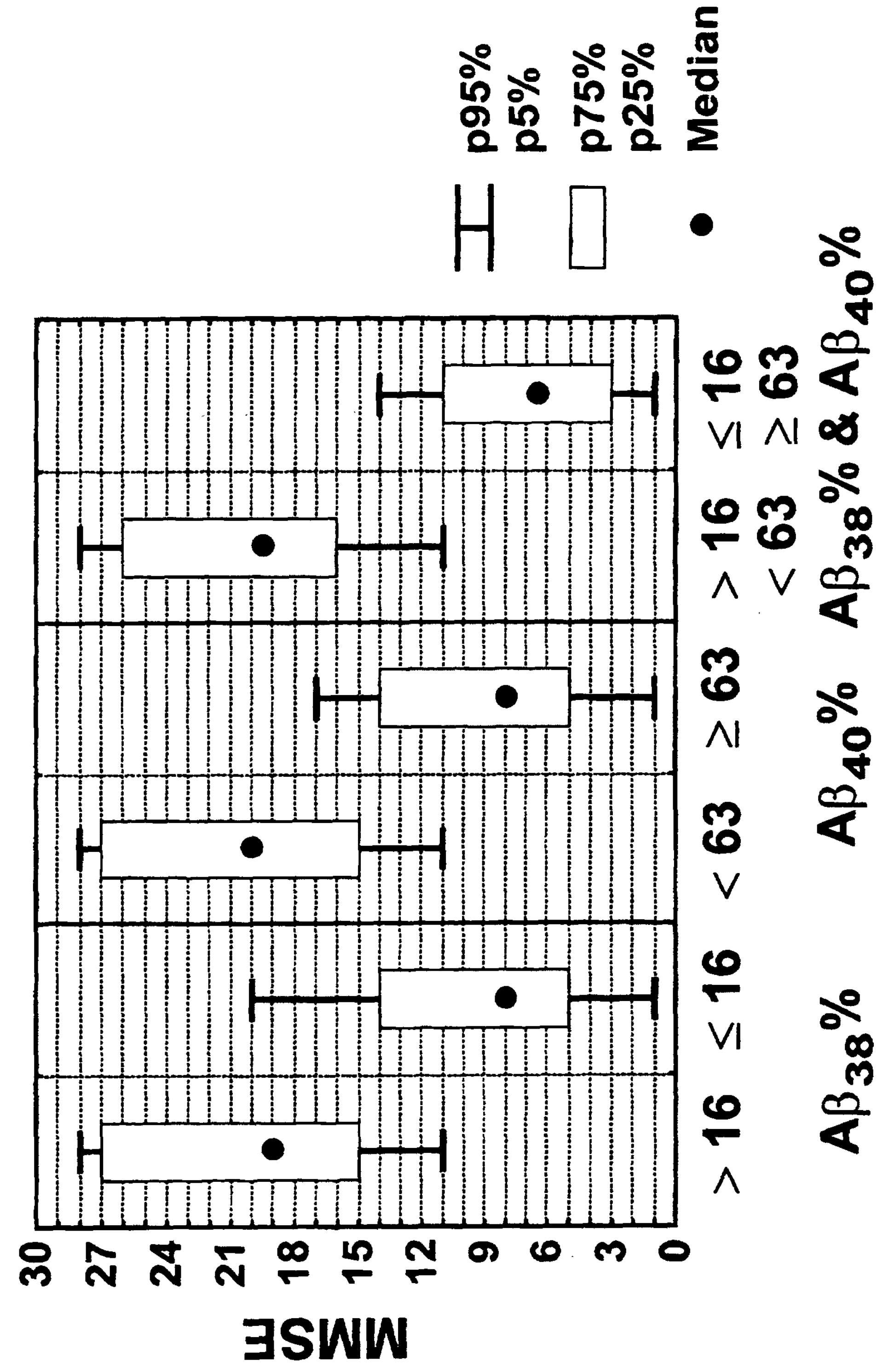
MMSE
30
27
24
21
18
15
12
9
6
3
0
> 16
≤ 16
$A\beta_{38}$%
< 63
≥ 63
$A\beta_{40}$%
> 16
< 63
≤ 16
≥ 63
$A\beta_{38}$% & $A\beta_{40}$%
p95%
p5%
p75%
p25%
Median

Fig. 15

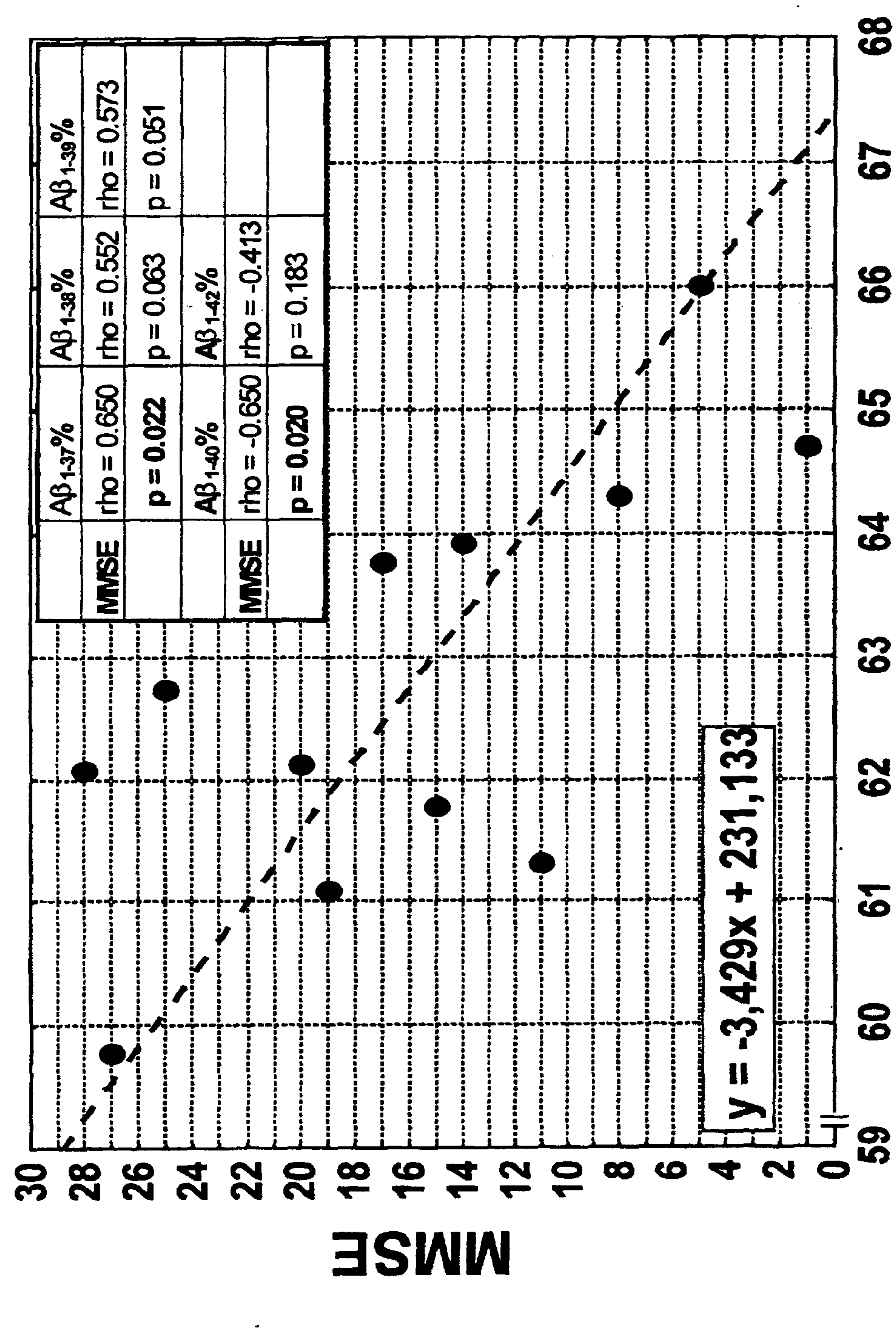
MMSE
Aβ1-40%
y = -3,429x + 231,133
Aβ1-37% | rho = 0.650 | p = 0.022
Aβ1-38% | rho = 0.552 | p = 0.063
Aβ1-39% | rho = 0.573 | p = 0.051
Aβ1-40% | rho = -0.650 | p = 0.020
Aβ1-42% | rho = -0.413 | p = 0.183
MMSE
MMSE
30
28
26
24
22
20
18
16
14
12
10
8
6
4
2
0
59
60
61
62
63
64
65
66
67
68

Fig. 16

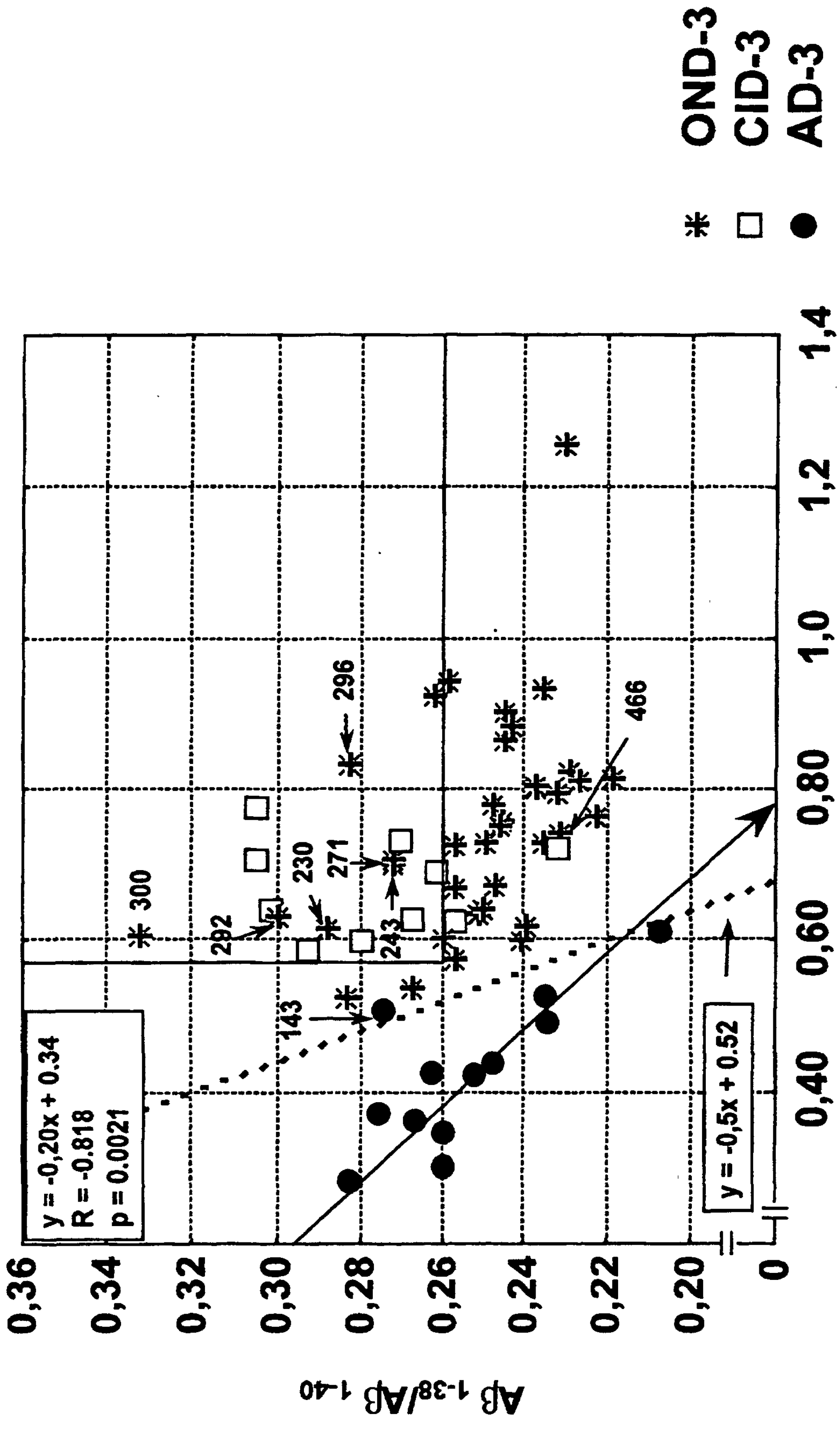

OND-3
CID-3
AD-3
y = -0,20x + 0.34
R = -0.818
p = 0.0021
y = -0,5x + 0.52
300
292
230
271
243
296
143
466
0,36
0,34
0,32
0,30
0,28
0,26
0,24
0,22
0,20
0
0,40
0,60
0,80
1,0
1,2
1,4
Aβ 1-38/Aβ 1-40
Aβ1-42/Aβ1-38

Fig. 17

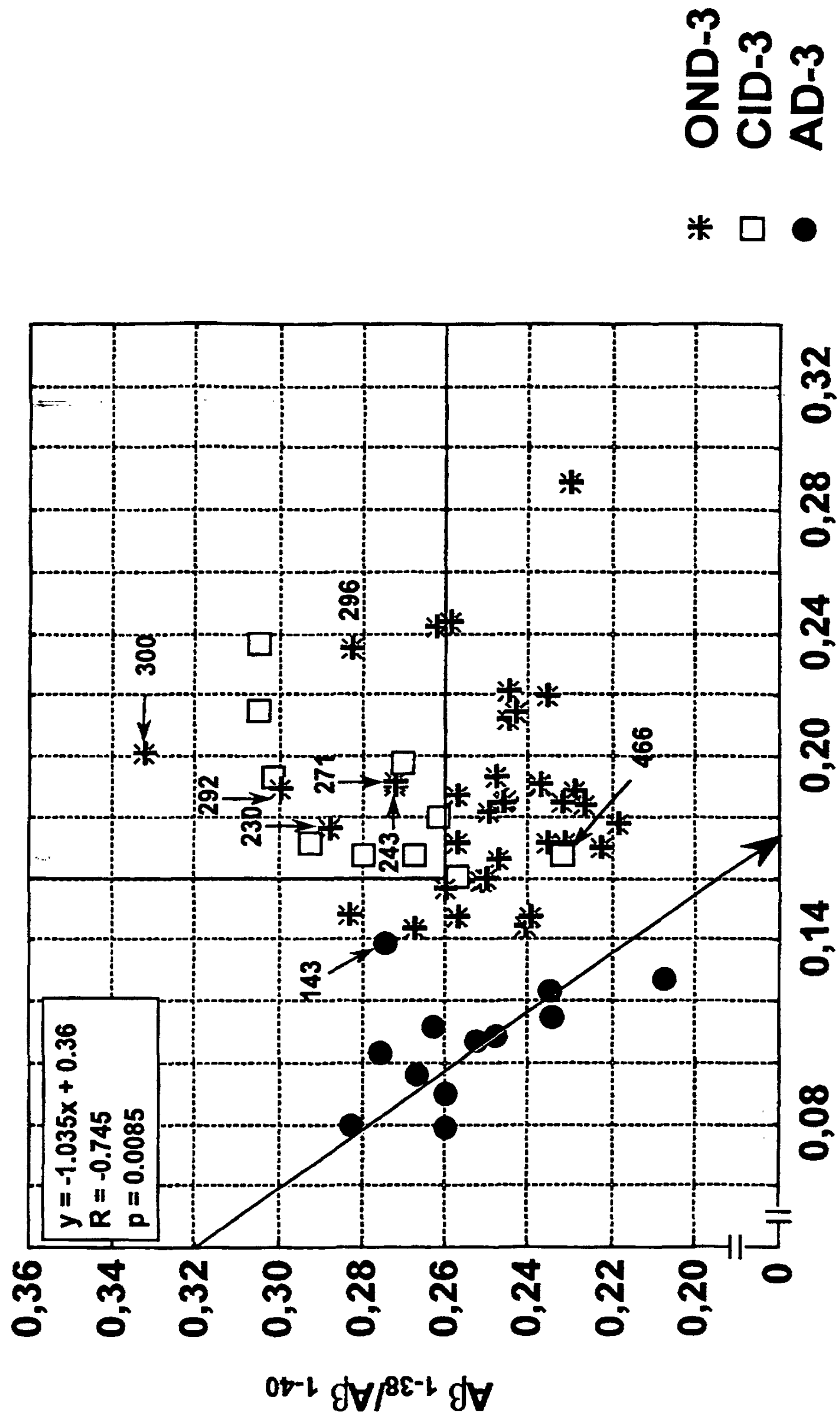
y = -1.035x + 0.36
R = -0.745
p = 0.0085
0,36
0,34
0,32
0,30
0,28
0,26
0,24
0,22
0,20
0
0,08
0,14
0,20
0,24
0,28
0,32
Aβ 1-38/Aβ 1-40
Aβ 1-42/Aβ 1-40
300
296
292
230
271
243
143
466
OND-3
CID-3
AD-3

Fig. 18

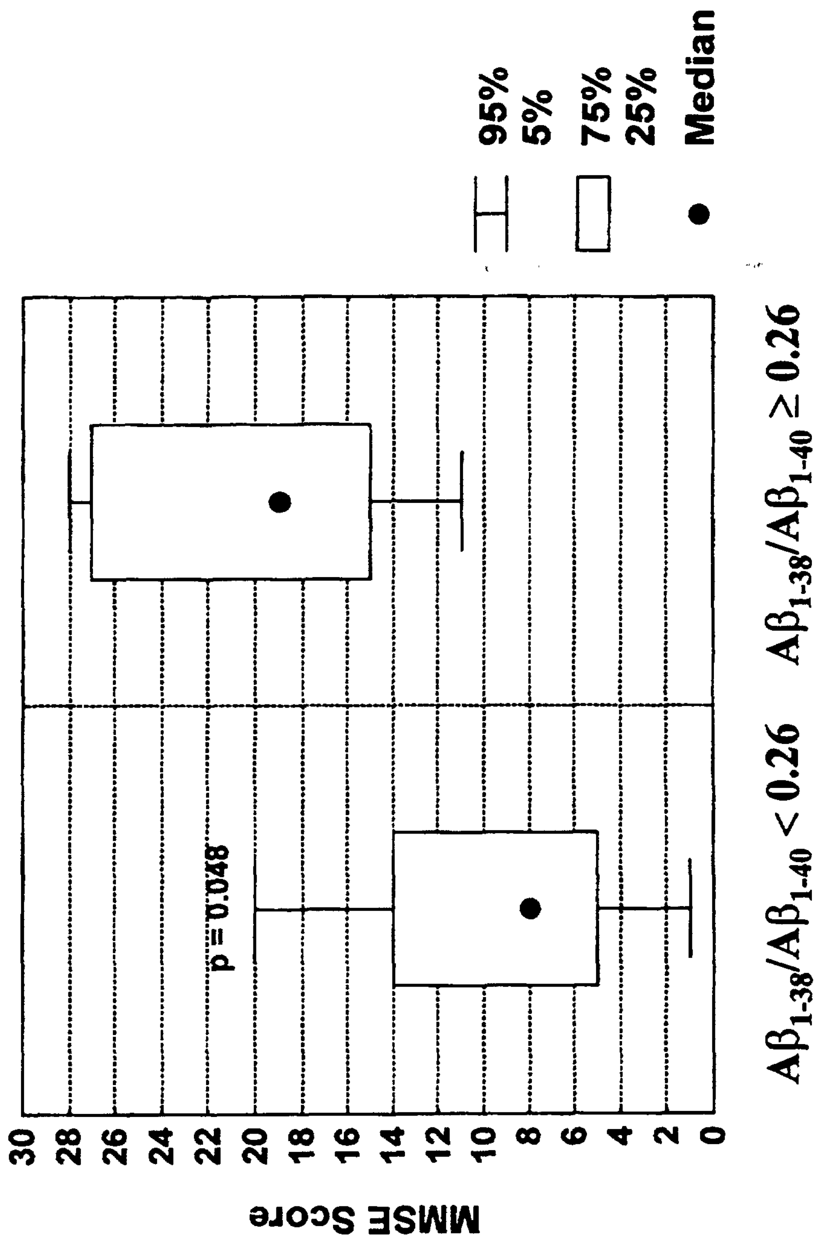
MMSE Score
30
28
26
24
22
20
18
16
14
12
10
8
6
4
2
0
p = 0.048
$A\beta_{1-38}/A\beta_{1-40} < 0.26$
$A\beta_{1-38}/A\beta_{1-40} \geq 0.26$
95%
5%
75%
25%
Median

Fig. 19

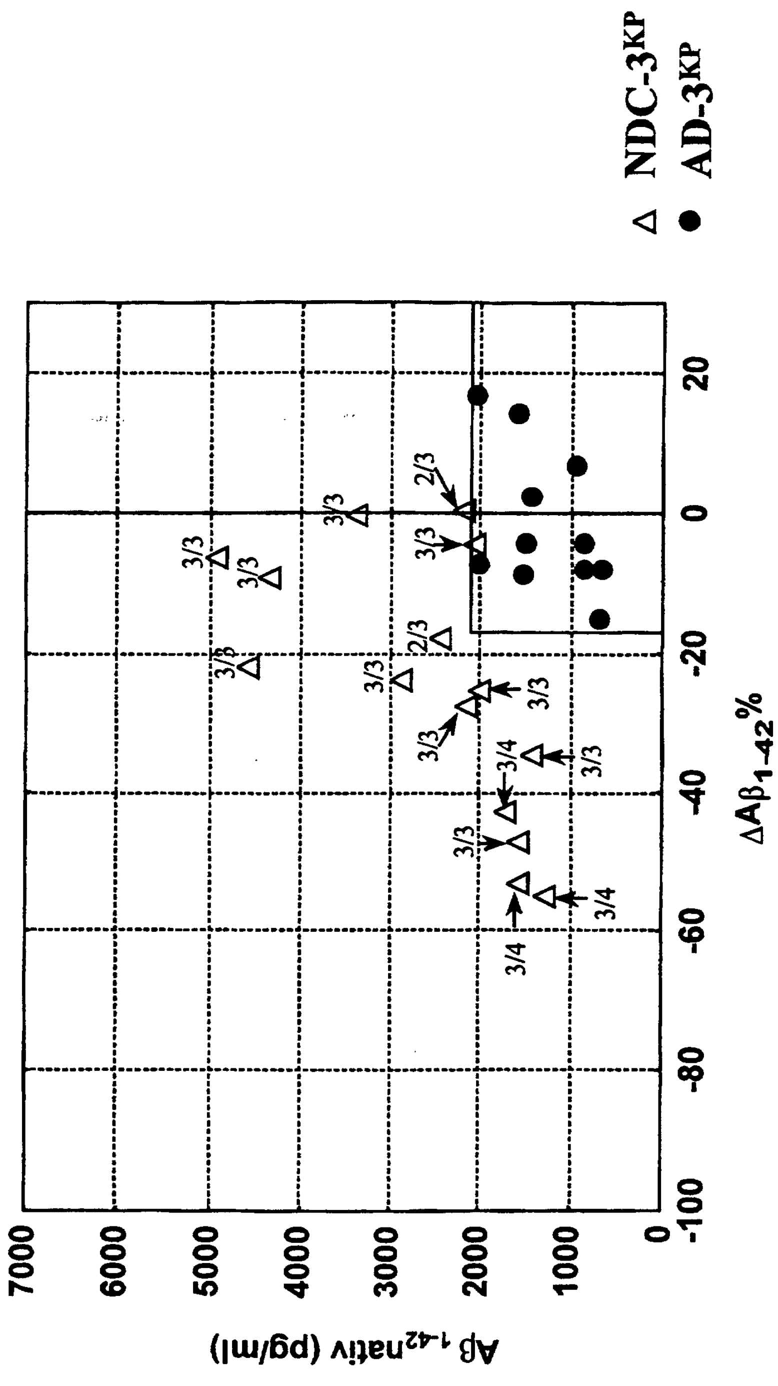
Δ NDC-3KP
● AD-3KP
Aβ 1-42 nativ (pg/ml)
ΔAβ1-42%
7000
6000
5000
4000
3000
2000
1000
0
-100
-80
-60
-40
-20
0
20
3/3
2/3
3/4

Fig. 20

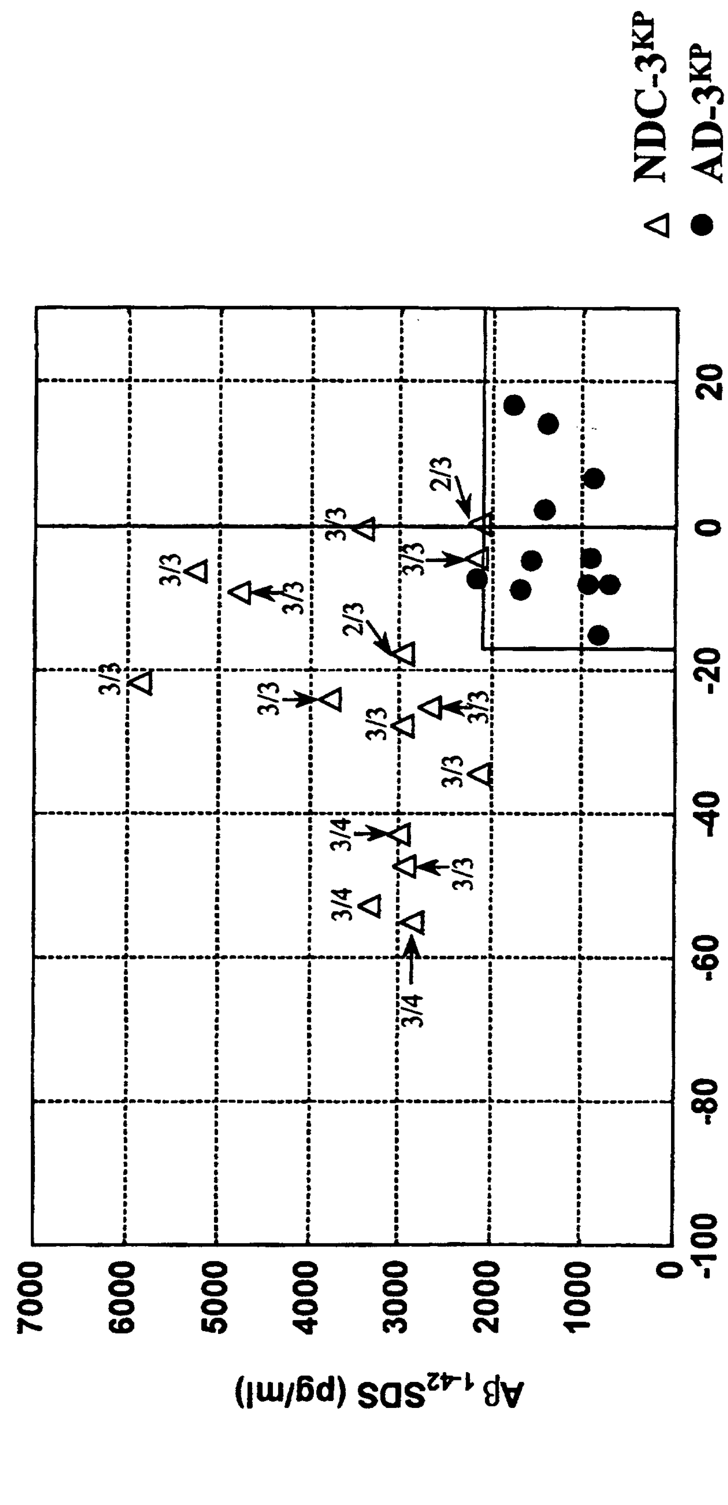
Aβ 1-42 SDS (pg/ml)
7000
6000
5000
4000
3000
2000
1000
0
-100
-80
-60
-40
-20
0
20
ΔAβ1-42%
3/3
3/4
2/3
Δ NDC-3KP
● AD-3KP

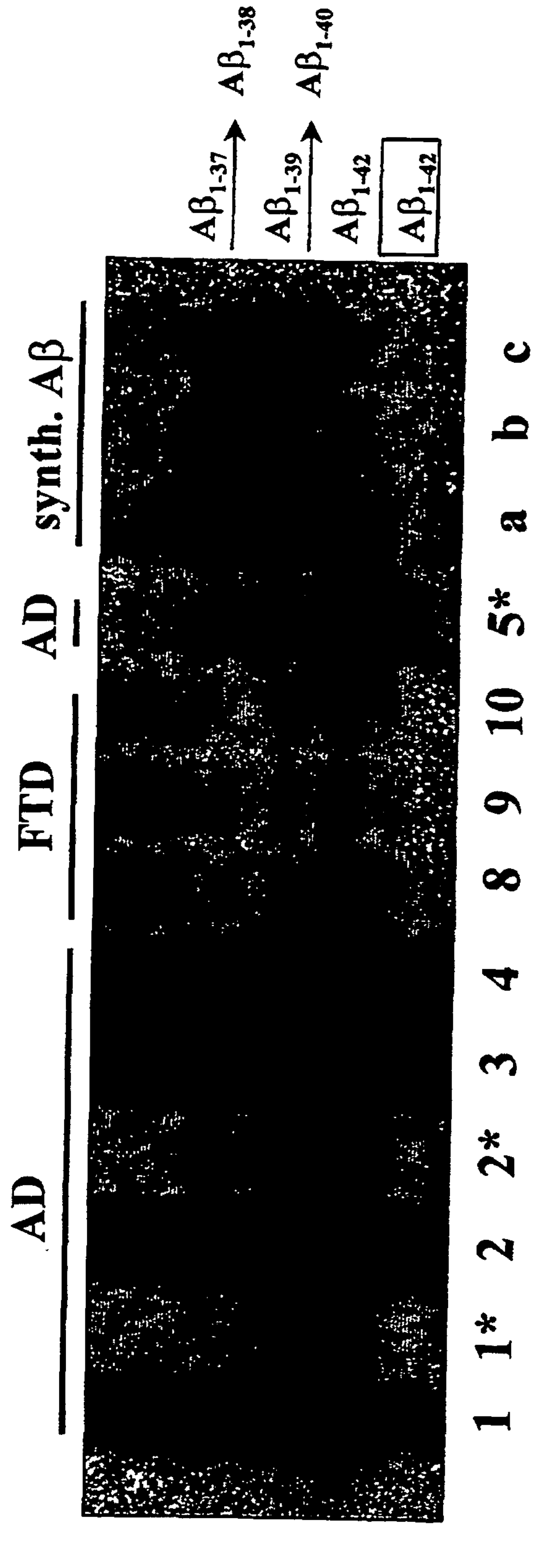
AD
FTD
AD
synth. Aβ
1 1* 2 2* 3 4 8 9 10 5* a b c
$A\beta_{1-37}$
$A\beta_{1-38}$
$A\beta_{1-39}$
$A\beta_{1-40}$
$A\beta_{1-42}$
$A\beta_{1-42}$

Fig. 21a

Fig. 21b

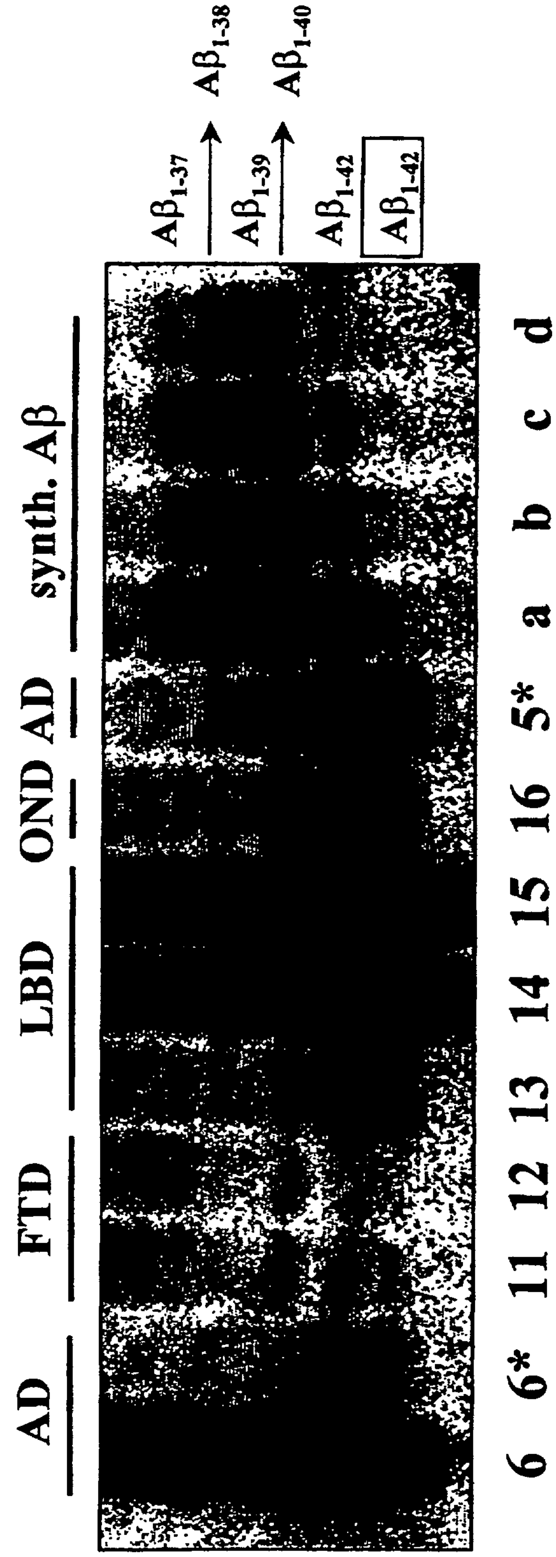
AD
FTD
LBD
OND
AD
synth. Aβ
6 6* 11 12 13 14 15 16 5* a b c d
$A\beta_{1-37}$
$A\beta_{1-38}$
$A\beta_{1-39}$
$A\beta_{1-40}$
$A\beta_{1-42}$
$A\beta_{1-42}$

Fig. 22

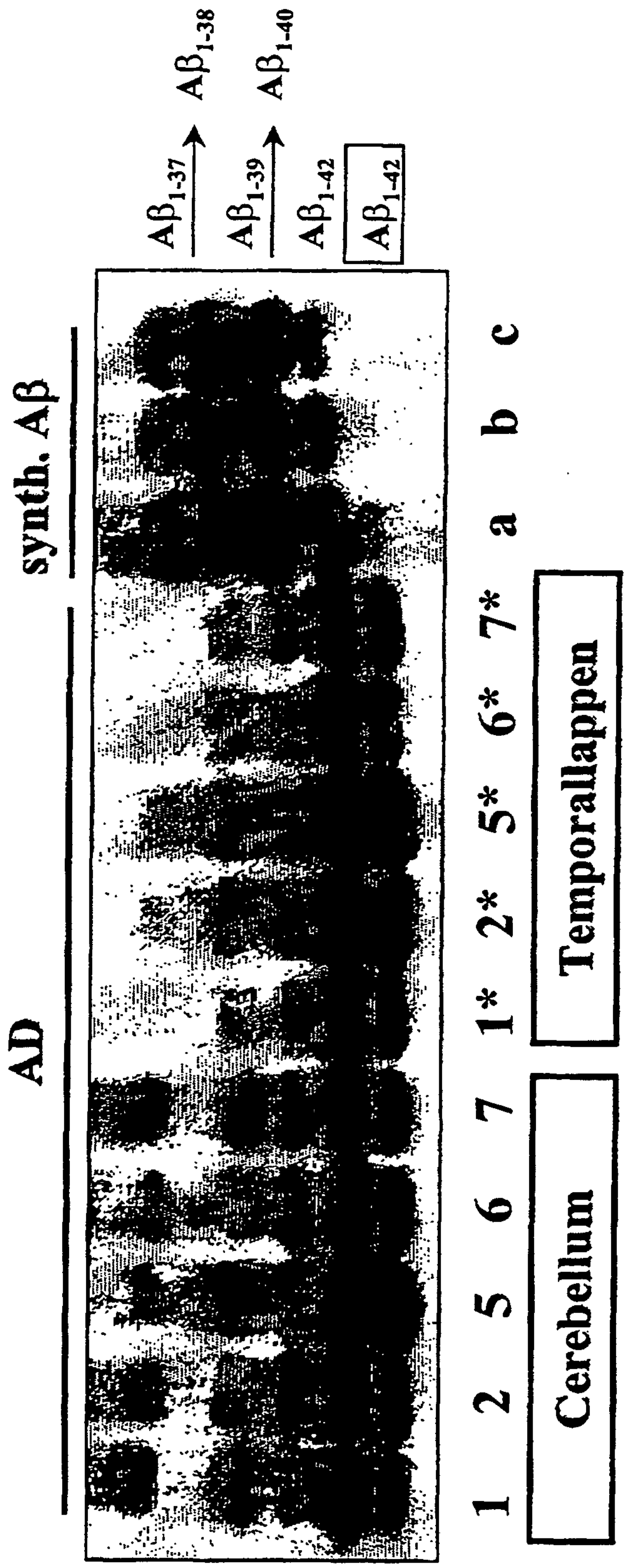
AD
synth. Aβ
Aβ1-37 → Aβ1-38
Aβ1-39 → Aβ1-40
Aβ1-42
Aβ1-42
1 2 5 6 7 1* 2* 5* 6* 7* a b c
Cerebellum
Temporallappen

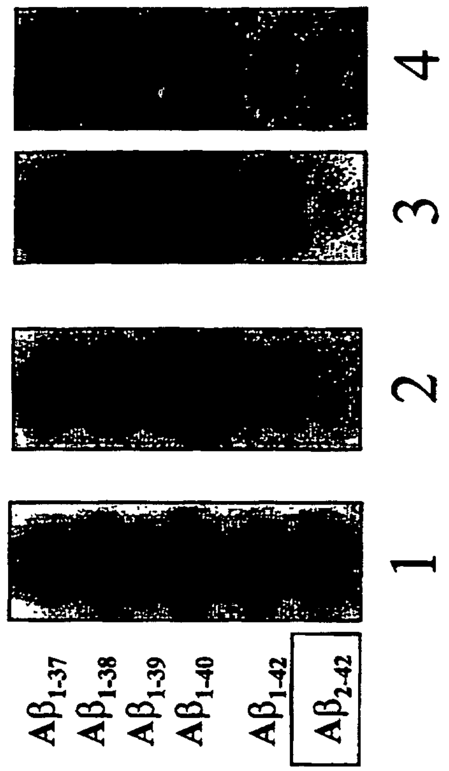


Fig. 23a

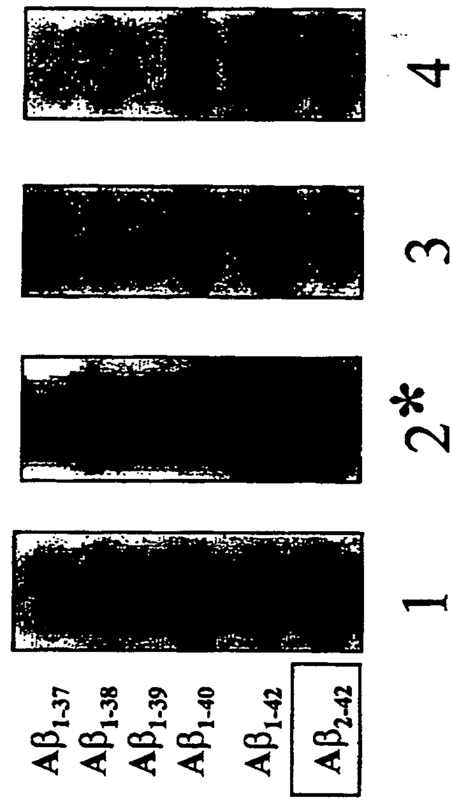


Fig. 23b

Fig 24

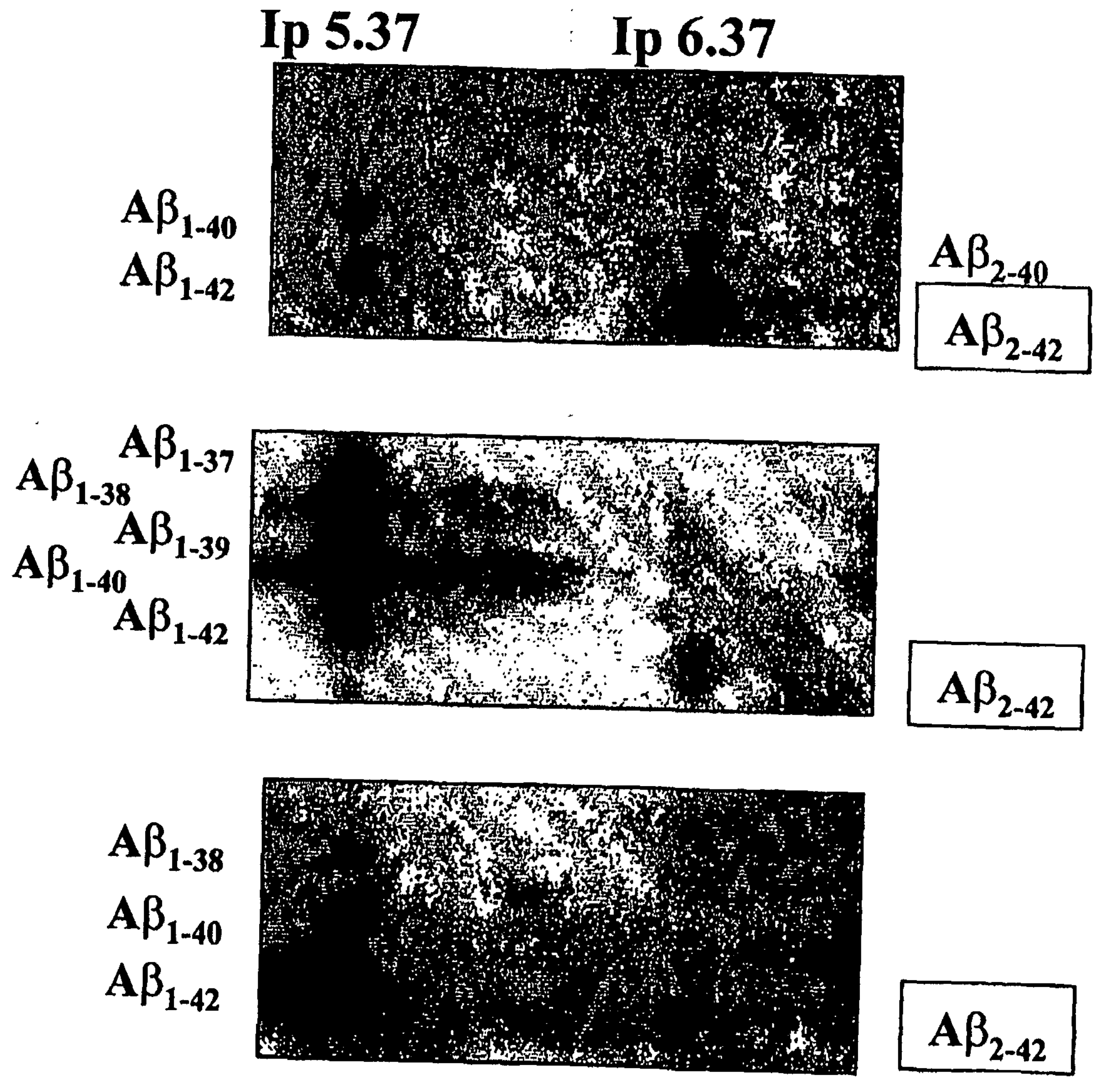
Ip 5.37
Ip 6.37
Aβ1-40
Aβ1-42
Aβ2-40
Aβ2-42
Aβ1-37
Aβ1-38
Aβ1-39
Aβ1-40
Aβ1-42
Aβ2-42
Aβ1-38
Aβ1-40
Aβ1-42
Aβ2-42

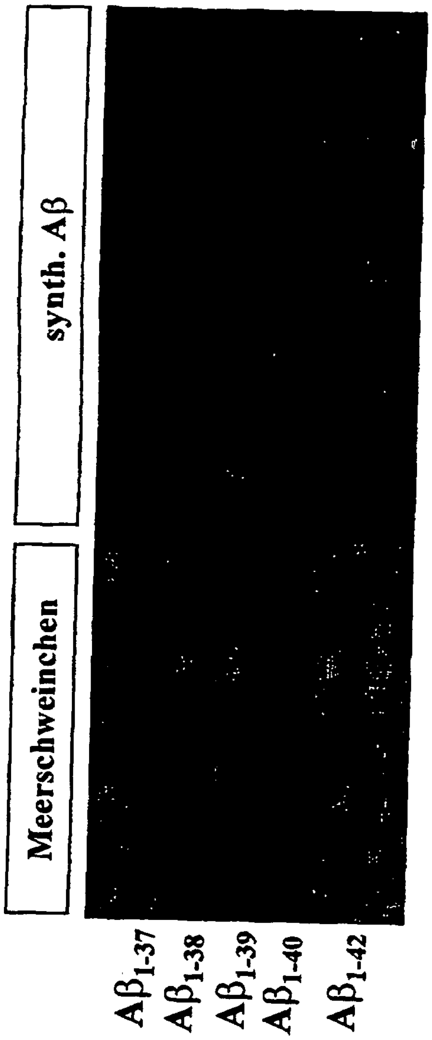


Fig. 25a

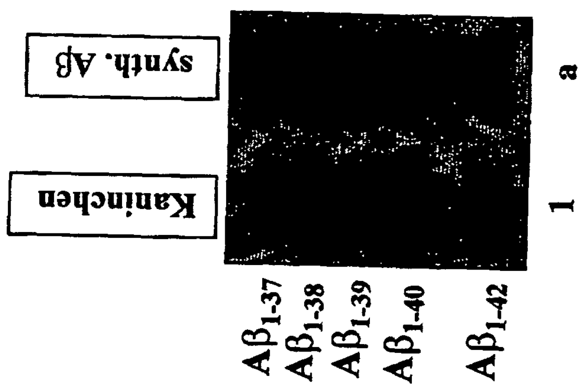


Fig. 25 b

Fig. 26

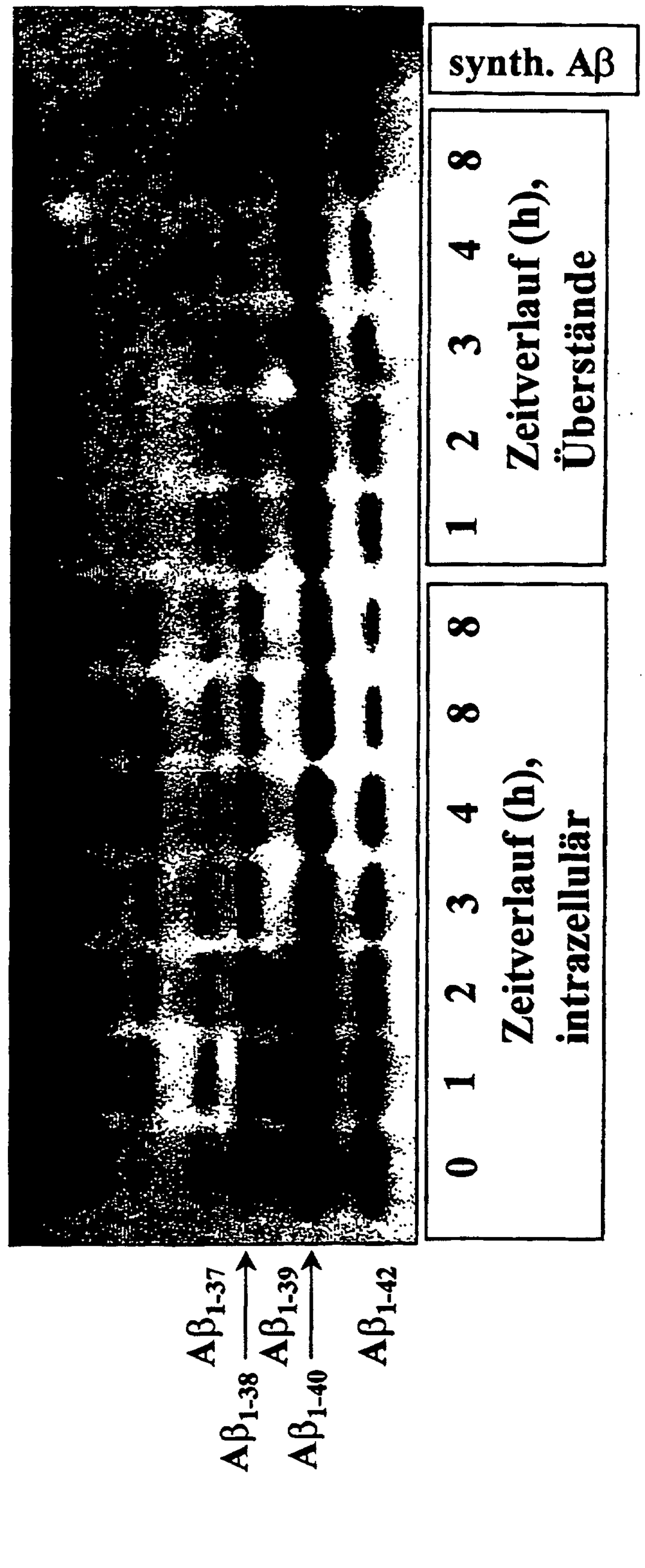
synth. Aβ
1 2 3 4 8
Zeitverlauf (h),
Überstände
0 1 2 3 4 8 8 8
Zeitverlauf (h),
intrazellulär
$A\beta_{1-37}$
$A\beta_{1-38}$
$A\beta_{1-39}$
$A\beta_{1-40}$
$A\beta_{1-42}$

Fig 27

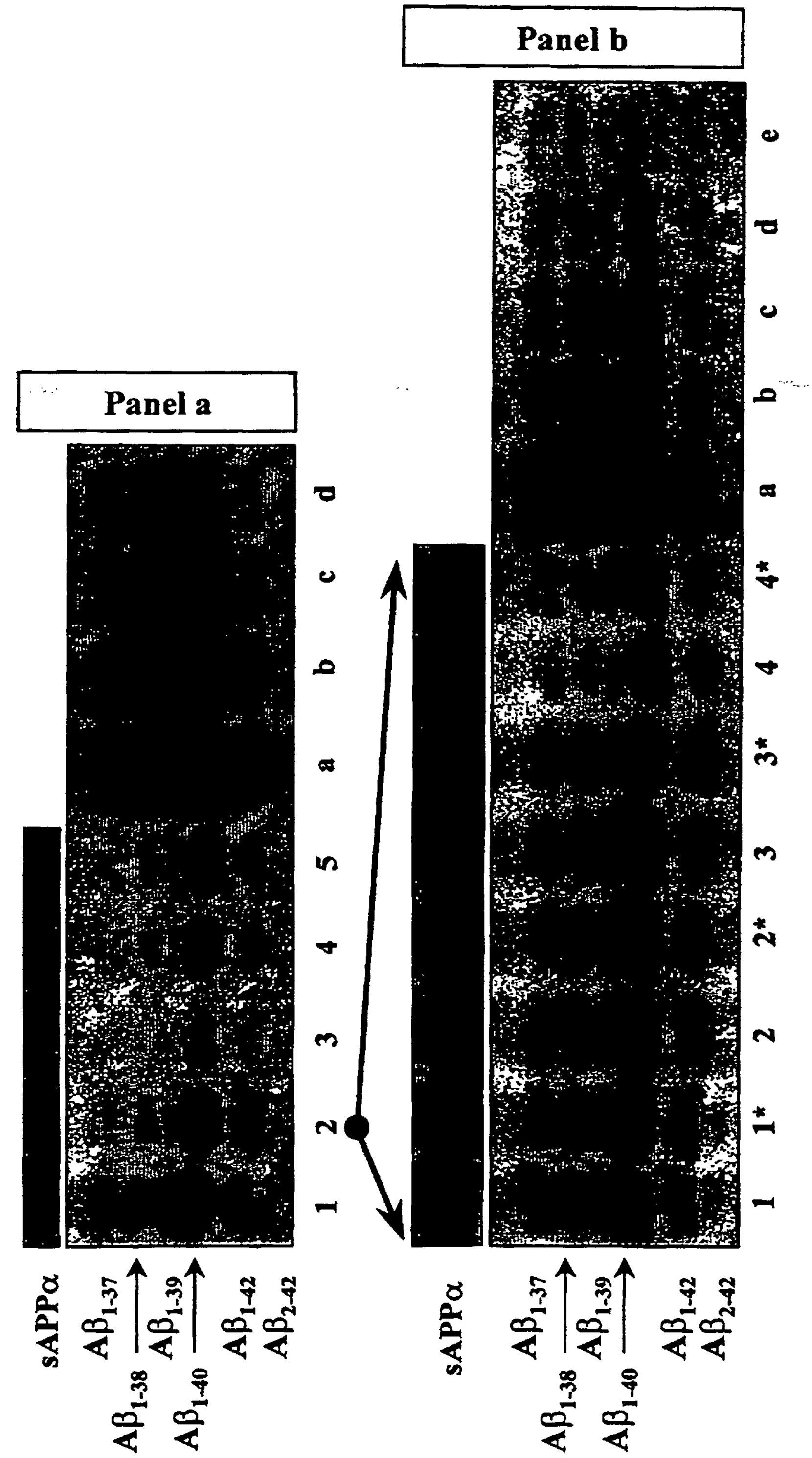
Panel a
sAPPα
Aβ1-37
Aβ1-38
Aβ1-39
Aβ1-40
Aβ1-42
Aβ2-42
1 2 3 4 5 a b c d
Panel b
sAPPα
Aβ1-37
Aβ1-38
Aβ1-39
Aβ1-40
Aβ1-42
Aβ2-42
1 1* 2 2* 3 3* 4 4* a b c d e

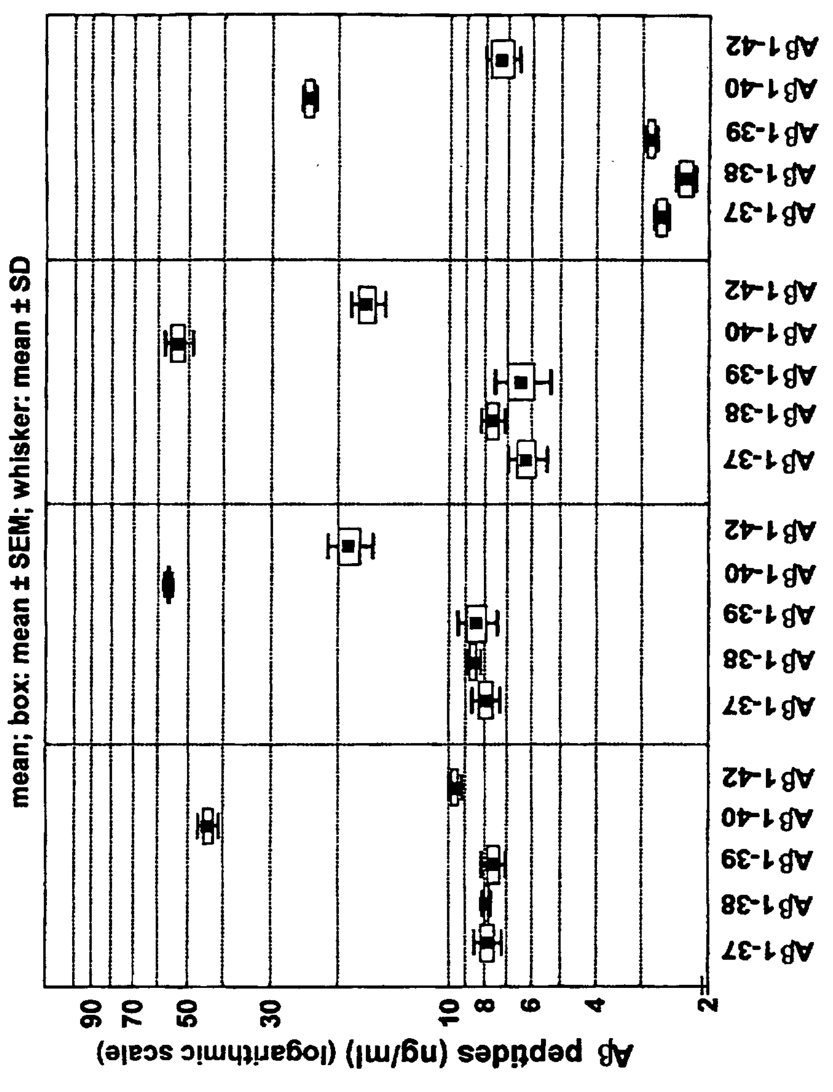
mean; box: mean ± SEM; whisker: mean ± SD
Aβ peptides (ng/ml) (logarithmic scale)
90
70
50
30
10
8
6
4
2
Aβ1-37
Aβ1-38
Aβ1-39
Aβ1-40
Aβ1-42
DMSO
Aβ1-37
Aβ1-38
Aβ1-39
Aβ1-40
Aβ1-42
Calpain-Inh.-1
Aβ1-37
Aβ1-38
Aβ1-39
Aβ1-40
Aβ1-42
Calpain-Inh.-1
Aβ1-37
Aβ1-38
Aβ1-39
Aβ1-40
Aβ1-42
Calpain-Inh.-1

Fig 28a

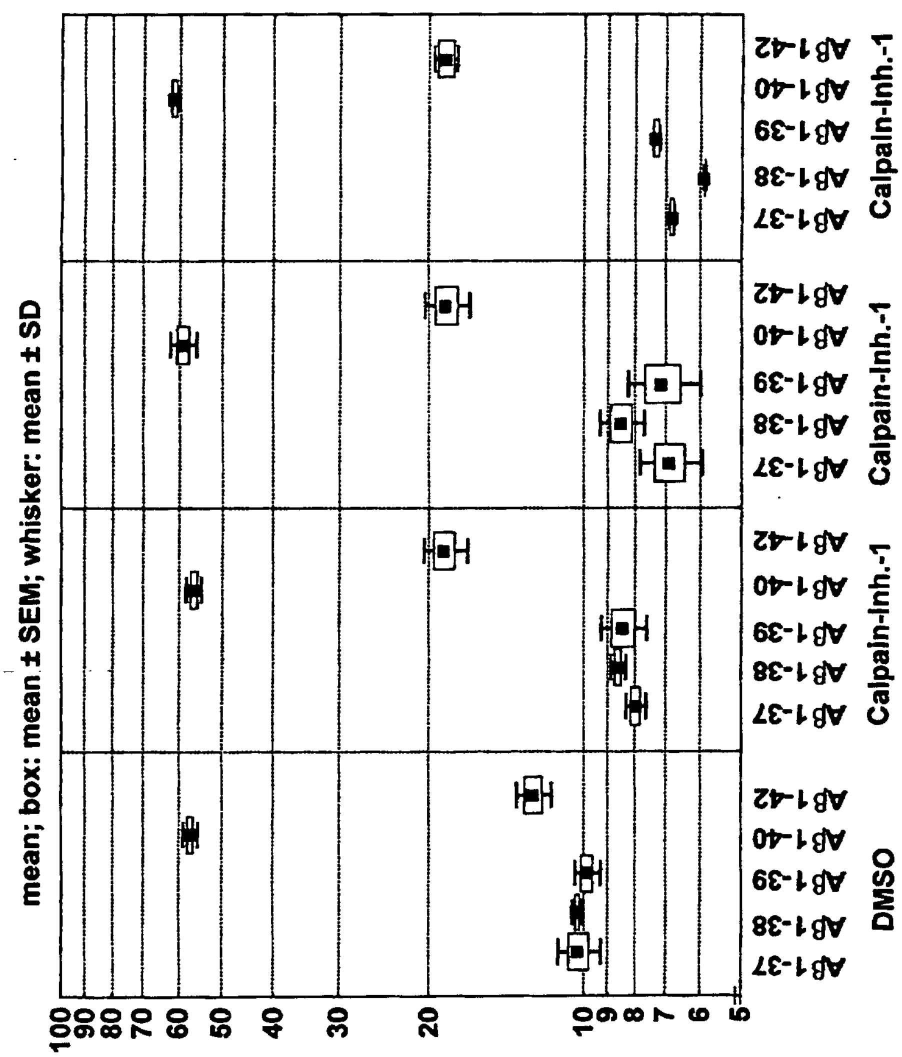
mean; box: mean ± SEM; whisker: mean ± SD
% of total Aβ peptides (logarithmic scale)
100
90
80
70
60
50
40
30
20
10
9
8
7
6
5
Aβ1-37
Aβ1-38
Aβ1-39
Aβ1-40
Aβ1-42
DMSO
Calpain-Inh.-1
Calpain-Inh.-1
Calpain-Inh.-1

Fig 28b